# EUROPEAN PATENT SPECIFICATION

(11) **EP 4 404 818 B1**
(45) Date of publication and mention of the grant of the patent: **11.03.2026**
(21) Application number: 22790367.1
(22) Date of filing: 28.09.2022
(51) Int. Cl.: A61B 1/07, A61B 1/05, A61B 1/00, A61B 1/06

(54) **ILLUMINATION FOR ENDOSCOPE**
ENDOSKOP-BELEUCHTUNG
ÉCLAIRAGE POUR ENDOSCOPE

(30) Priority: 28.09.2021 US 202163249479 P; 25.05.2022 US 202217824857; 25.08.2022 US 202263400961 P
(43) Date of publication of application: 31.07.2024
(73) Proprietor: PSIP2 LLC, Manchester, NH 03104 (US)
(72) Inventor: HERDA, Michael, Savannah, Georgia 31405 (US); MOHAN, Karan, Union City, California 94587 (US); ARNOLD, Stephen, Honeoye Falls, New York 14472 (US); DESAI, Siddharth, Mission Viejo, California 92692 (US)
(74) Representative: Stellbrink & Partner Patentanwälte mbB
(86) International application number: PCT/IB2022/059262
(87) International publication number: WO 2023/053042

(56) References cited:
- EP-B1- 0 904 725
- US-A1- 2013 012 783
- US-A1- 2021 169 316

## Description

### BACKGROUND

Reference is made to U.S. Provisional Application Ser. No. 63/400,961, filed Aug. 25, 2022, titled Endoscope; to U.S. application Ser. No. 17/824,857, filed May 25, 2022, titled Endoscope; and to U.S. Provisional App. Ser. No. 63/249,479, filed Sep. 28, 2021, titled Endoscope.

This application relates to endoscopes, laparoscopes, arthroscopes, colonoscopes, and similar surgical devices or appliances specially adapted or intended to be used for evaluating, examining, measuring, monitoring, studying, or testing living or dead human and animal bodies for medical purposes, or for use in operative surgery upon the body or in preparation for operative surgery, together with devices designed to assist in operative surgery. For example, document US 2021/169316 A1 describes an endoscope that includes a first component, a second component, a light source, an image capturing element, and a light guide. The second component has a proximal end and a distal end with proximal end coupled to the first component. The light source is integrated in the first component and includes a laser and a converter. The laser emits primary light and the converter converts the primary light at least partially into secondary light that has a different wavelength. The image capturing element is arranged at the distal end. The light guide has an optical fiber that extends through the second component. The converter is coupled to the proximal end such that the primary and secondary light is injected into the optical fiber, is conducted from the proximal end to the distal end, and emitted at the distal end.

### SUMMARY

In general, the present invention features an arthroscope as defined in claim 1. The arthroscope has a handle and an insertion shaft. The insertion shaft has near its distal end a solid state camera. The shaft has enclosed therein at least one light conductor designed to conduct illumination light to the distal end. The shaft has an outer diameter of no more than 6mm. The shaft has rigidity and strength for insertion of the camera into joints for arthroscopic surgery. The light conductor(s) in the region of the camera are designed to conduct illumination light from a light fiber to the distal end through a space between the camera and the inner surface of the insertion shaft.

A light conduction fiber, which is not individually claimed, is described. The fiber has a flattened region shaped to lie between an endoscope camera and an inner surface of an outer wall of an endoscope shaft, and shaped to conduct illumination light to a distal end of the endoscope shaft for illumination of a surgical cavity to be viewed by the camera. The shaft is no more than 6mm in diameter. The flattened region is formed by heating a region of a plastic optical fiber, and squeezing the heated region in a polished mold.

Preferred embodiments may feature one or more of the following. One or more light guides may be designed to conduct illumination light from a light fiber to the distal end. The light guide may have a cross-section other than circular. The light guide may have a coupling to accept illumination light from a circular-cross-section optical fiber. The light guide's cross-section in the region of the camera may be narrower than the diameter if the light fiber in the light guide's dimension corresponding to a radius of the insertion shaft. According to the present invention at least one of an inner and outer surface of the one or more light guides is longitudinally fluted. A distal surface of the one or more light guides or flattened region may be designed to diffuse emitted light. A distal surface of the one or more light guides may have surface microdomes designed to diffuse emitted light, or may be otherwise configured to improve uniformity of illumination into a surgical cavity accessed by the arthroscope. One or more light conductors in the region of the camera may be formed as a flattened region of an optical fiber. The flattened region may be shaped to lie between the endoscope camera and an inner surface of an outer wall of an endoscope shaft. The flattened region may be shaped to conduct illumination light to a distal end of the endoscope shaft for illumination of a surgical cavity to be viewed by the camera. The shaft may be no more than 6mm in outer diameter. The flattened region may be formed by heating a region of a plastic optical fiber. The flattened region may be formed by squeezing an optical fiber in a polished mold. Component parts for mounting near the distal end of the endoscope may be shaped using poka-yoke design principles to ensure correct assembly. Component parts of a lens assembly for mounting near the distal end may be shaped using poka-yoke design principles to ensure correct assembly. Component parts near the distal end may be formed to permit focus adjustment of a lens assembly during manufacturing. The endoscope may have a terminal window designed to seal with the shaft to prevent intrusion of bodily fluids, bodily tissues, and/or insufflation fluid. The terminal window may be designed to reduce optical artifacts. The artifacts may reduced may be reflection, light leakage within the endoscope, fouling by bodily fluids and/or bodily tissues, and fogging. The light conductors in the region of the camera may include at least nine optical fibers of essentially continuous diameter from a light source, the light fibers being no more than about 0.5mm diameter, and arrayed to subtend at least 250° of the circumference of the distal end of the endoscope. An arthroscope insertion shaft may have near its distal end a solid state camera. The shaft may have enclosed therein light conductors designed to conduct illumination light to the distal end. The shaft may have rigidity and strength for insertion of the camera into joints for arthroscopic surgery. The flattened region may be dimensioned to conduct illumination light from a light fiber to the distal end through a space between the camera and the inner surface of the insertion shaft.

The above advantages and features are of representative embodiments only, and are presented only to assist in understanding the invention. It should be understood that they are not to be considered limitations on the invention as defined by the claims. Additional features and advantages of embodiments of the invention will become apparent in the following description, from the drawings, and from the claims.

### DESCRIPTION OF THE DRAWINGS

FIGS. 1A to 1H, 2A to 2Q, and 3A to 3G, 4C, 4E to 4H, 4N to 4W, 4Z, 4AA to 4GG, 4II to 4MM, 5B, 7A, 7G to 7J, and 7N to 7Z, 9B, 9C, 9G, and 9J are perspective views of endoscopes and/or endoscope related apparatus.
FIGS. 4A, 4X, 4Y, 4HH, 5C, 5G to 5J, 6D, 7B, 7D, 8A to 8E, 9C, 9E to 9I are plan section views of endoscopes and/or endoscope related apparatus.
FIGS. 4B, 4I, 4M, 6E, 7M, are exploded views of endoscopes and/or endoscope related apparatus.
FIGS. 4D, 4J, 4K, 4L, 5D to 5F, 7C, 7E, 7F, 7K, 7L, 8F, 9A, 9D are perspective section or cutaway views of endoscopes and/or endoscope related apparatus.
FIGS. 5A, 6A to 6C, 9K and 9L are plan views of endoscopes and/or endoscope related apparatus.

### DESCRIPTION

The Description is organized as follows.
I. Overview
II. An endoscope that is partially-reusable, partially disposable/replaceable, and a coupling joint between
III. Extendable, bendable, or articulated camera tip
IV. Additional features of an endoscope
V. Endoscope tip
   V.A. Molding of components of the endoscope tip
   V.B. Fiber optic illumination for a single-use use scope tip
   V.C. A tip design with light guides
   V.D. Diffusion terminal surface
VI. Antifouling and antifogging
   VI.A. Heating
   VI.B. For endoscope's delivery packaging, vial of fluid to protective a coating on the endoscope's lens/window
VII. Lens cap with optical correction for use during insertion of offset-view endoscope
VIII. 360° view tip
IX. Avoiding fasteners, springs, and other small components
   IX.A. A button without springs
   IX.B. Overmolding of case over circuit board
   IX.C. Avoiding internal fasteners
   IX.D. Rotational resistance via O-rings
   IX.E. Ultrasonic welding of the two halves of the outer handle shell
   IX.F. Thermoplastic elastomer coating handle
X. Liquid flow
   X.A. Liquid-tight seal
   X.B. Inducing spiral flow
XI. Molding and Joining
   XI.A. Diagonal slots to connect dissimilar materials
   XI.B. Joining component parts of obturator
   XI.C. Twist-locking the parts together
XII. Embodiments

### I. Overview

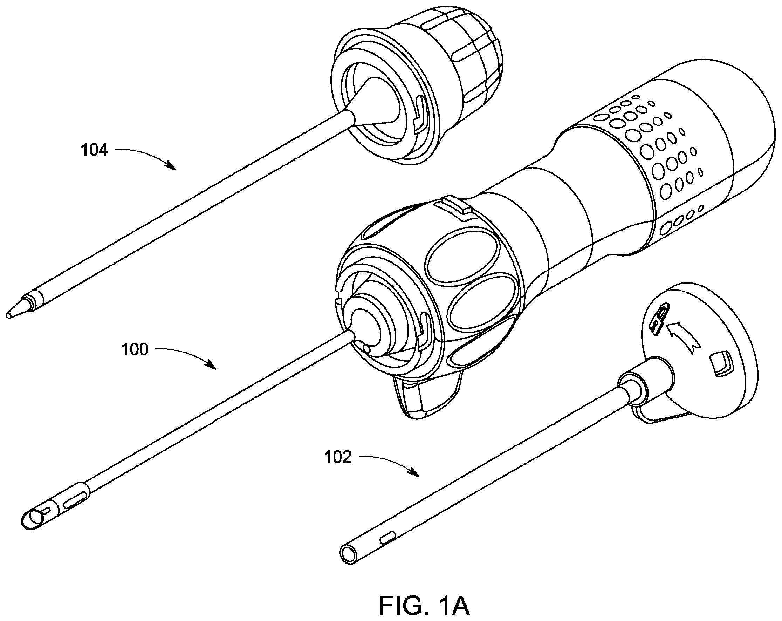

Referring to FIG. 1A, endoscope 100 (such as an arthroscope, laparoscope, or other), trocar 102, and obturator 104 may be used for joint surgery, joint access, or other minimally-invasive surgery. A minimally-invasive surgery generally begins with penetration to the surgical site using a pointed instrument such as trocar 102 or obturator 104. Trocar 102 is a hollow tube used to pierce from the skin to the surgical site; trocar 102 will remain in place during the surgery to maintain an access port. During piercing, trocar 102 may have obturator 104 inserted through the lumen. Obturator 104 and/or trocar 102 may have sharp points for piercing into the tissue of a joint, abdominal cavity, or other surgical site to create an access portal. Obturator 104 may be inserted into trocar 102, used as a unit to pierce skin and other tissues, and once the space of interest is accessed, obturator 104 may be pulled out, leaving the "trocar cannula." The scope may be placed through the cannula for visualization of the surgical site. The endoscope may be a "chip on a tip" scope, having an illumination source and camera 410. After an access portal is cut via obturator 104 and trocar 102, the endoscope may be inserted through trocar 102 to the surgical site, to provide the visual access to a surgeon who performs the surgery with other instruments. Other trocars may placed for access by other instruments, such as laparoscopic scalpels, shavers, staplers, suture machines, and the like.

In some cases, endoscope 100 may be inserted down the inner lumen of obturator 104, and the point of obturator 104 may be transparent. The endoscope-within-obturator configuration may provide visual guidance to guide obturator 104 or trocar 102 to the correct surgical site. In other cases, trocar 102 may have a sharp point, and the endoscope may be inserted down the inner lumen of trocar 102, to guide trocar 102.

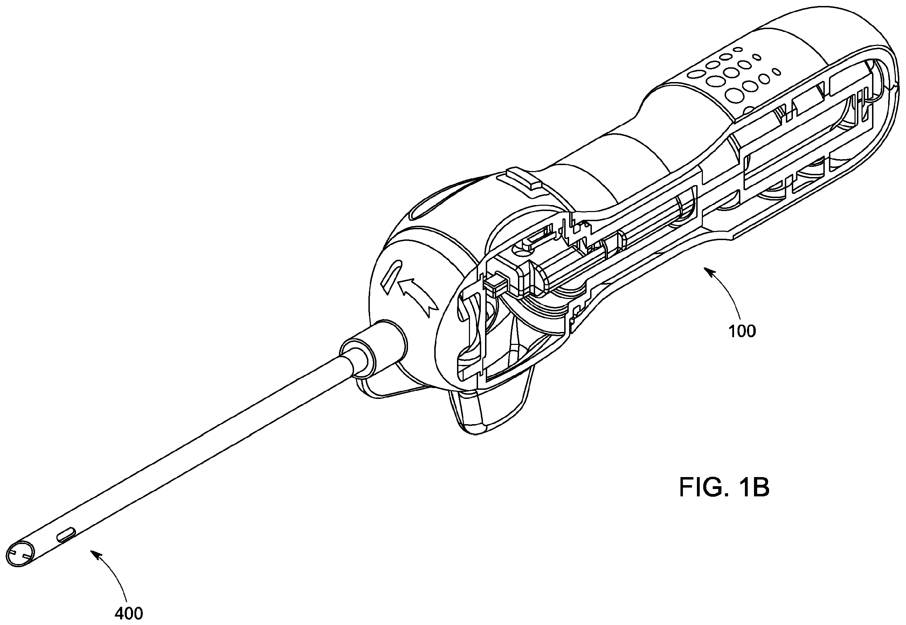

Referring to FIG. 1B, endoscope 100 may have a number of features that ensure reliability, reduce manufacturing costs to the point that the entire endoscope, or portions thereof, may be disposable. Resterilization of endoscopes is costly, and always imperfect, which increases risk of cross-infection. Also, as endoscope 100 is reused, the optics become scratched and clouded, reducing the precision of the view available to the surgeon. Sections II to XI will discuss multiple features of the endoscope that provide such cost reduction and disposability.

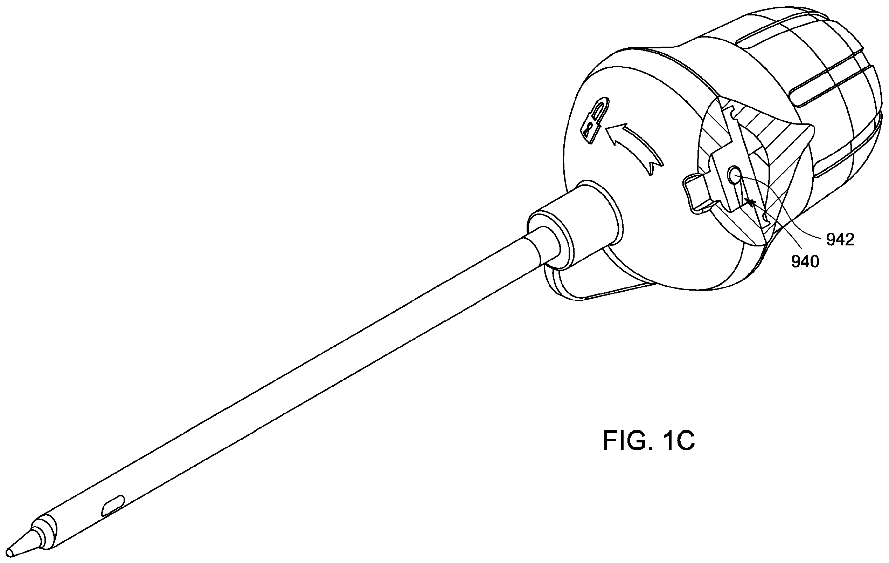

Referring to FIG. 1C, obturator 104 may be locked into the outer sheath or trocar 102 via a twist lock 940, 942.

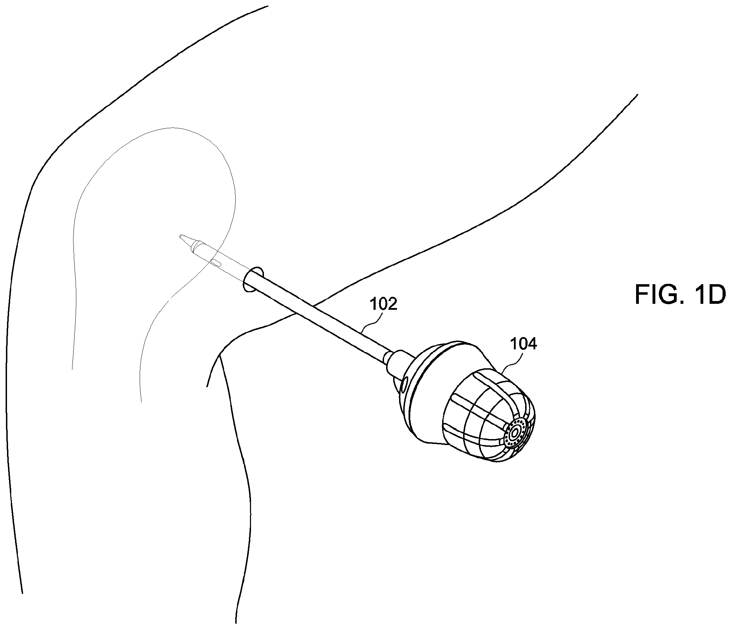

Referring to FIG. 1D, obturator 104 and trocar 102 may be used to pierce into the surgical site, for example, a region of a knee behind the patella.

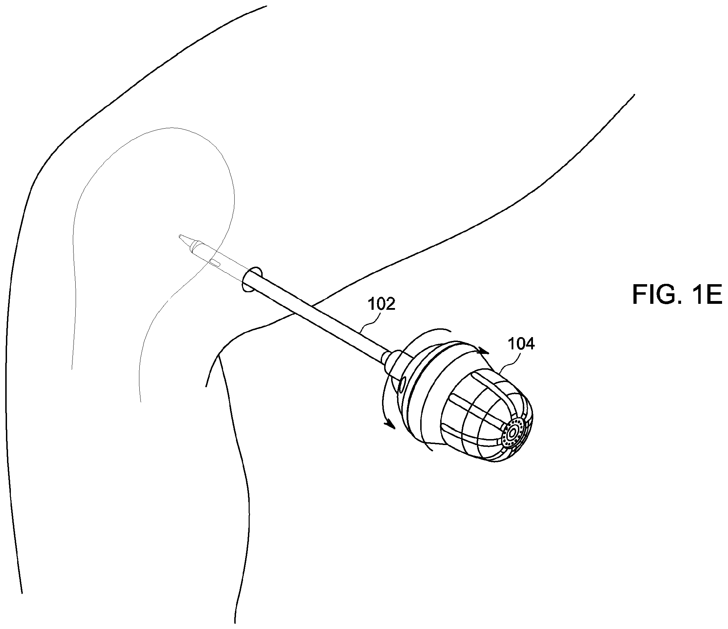

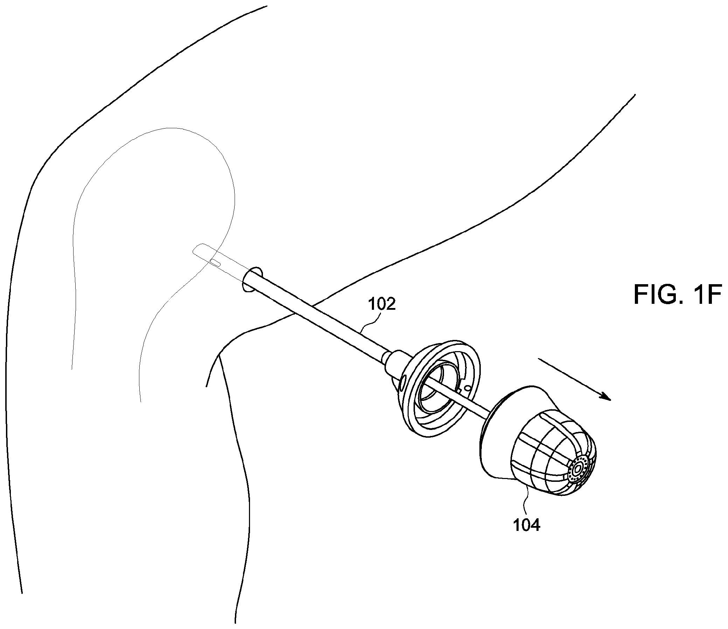

Referring to FIGS. 1E and 1F, obturator 104 may be unlocked from outer sheath/trocar 102. Obturator 104 and endoscope may be withdrawn, leaving hollow trocar 102 as a port to the site.

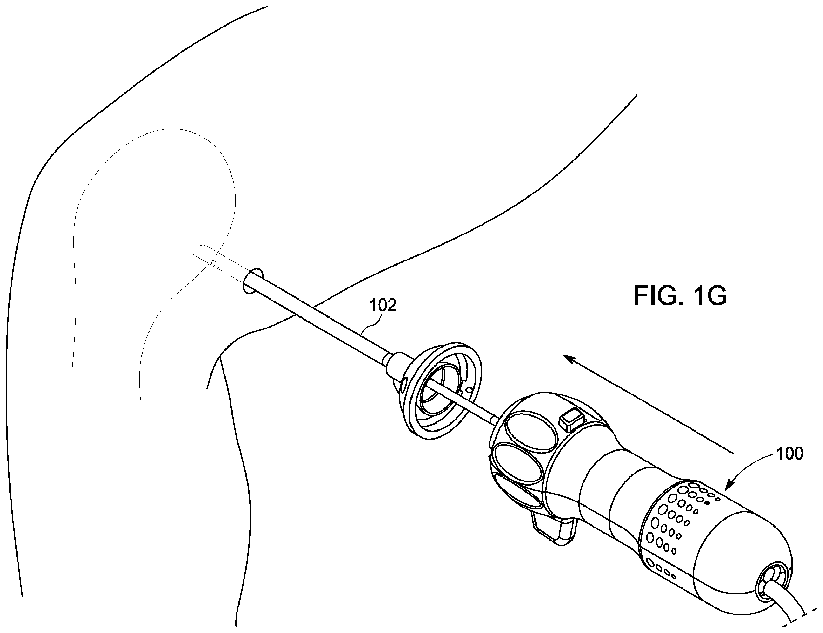

Referring to FIG. 1G, instruments, such as endoscope 100, etc. may be inserted through the outer sheath/trocar 102 to perform the surgery.

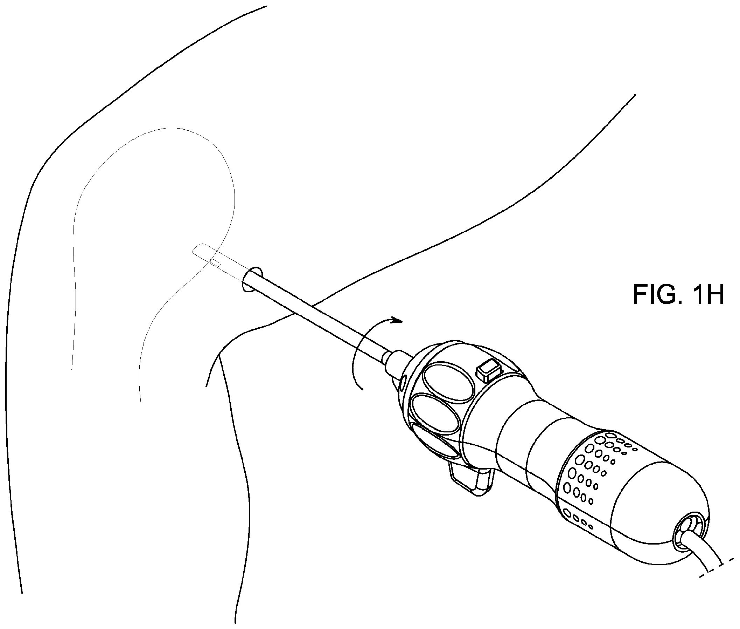

Referring to FIG. 1H, trocar/sheath 102 may be locked to the endoscope via the same twist lock motion as was used to hold the outer sheath/trocar 102 to obturator 104.

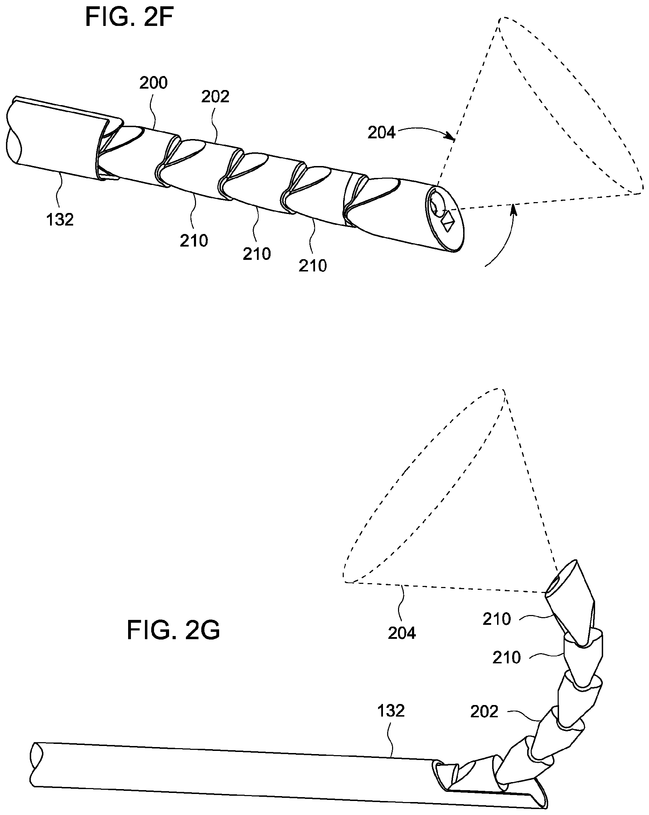

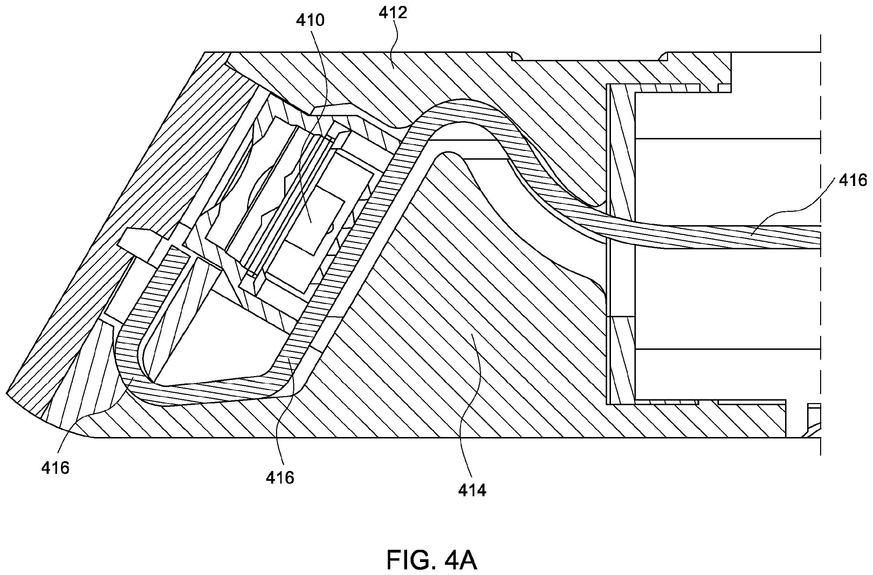

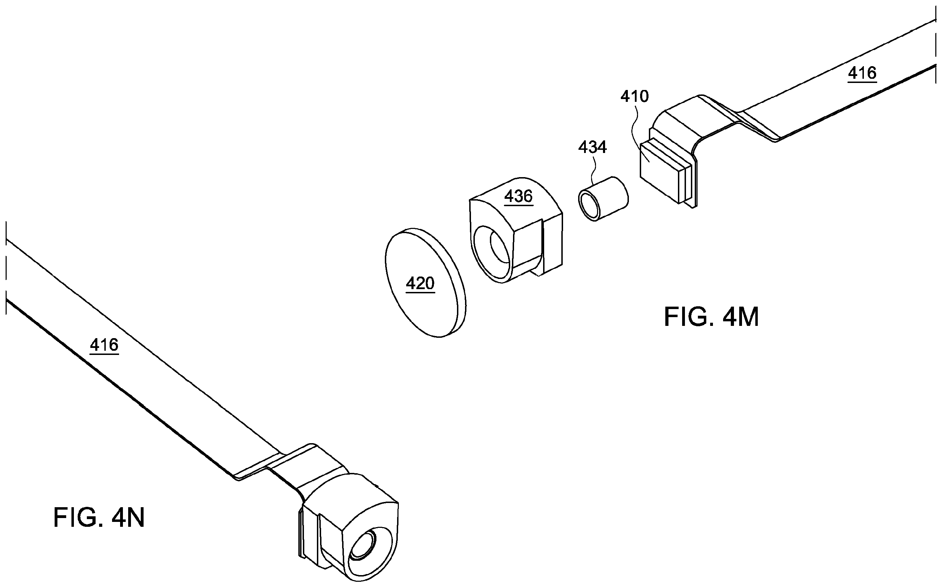

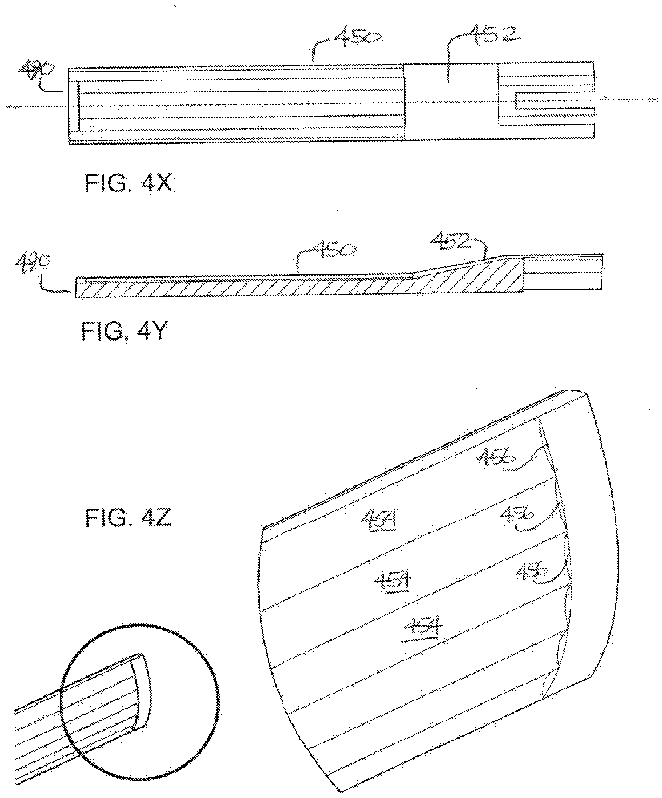

The various endoscope tip designs (FIGS. 2F-2M, 4A-4K, 4M-4W, 4X-4OO), may have the following properties. The overall tip may be small enough to meet the dimensions of the endoscope, typically the dimensions in the table of paragraph [0028] above. In some cases, the tip may be slightly larger or smaller in diameter than shaft The tip may hold camera 410, illumination, fluid injection or evacuation ports, procedural tools, etc. mechanically stable, within that diameter. The tip may seal against elevated pressures that are typically used to distract tissues out of the view of the scope, to prevent intrusion of bodily tissues and fluids and insufflation fluid. The tip may deliver or allow delivery of illumination light, either via an LED mounted in the tip, or using fiber optics 430 to convey light from the handle or a controller. Opaque parts of the tip assembly may exclude stray light, both from the illumination fibers/LEDs/light guides, and reflected light from the surgical cavity. The tip may be manufacturable at desired quantities and cost. The tip may have a configuration that is atraumatic to surrounding tissue, for instance, without sharp points or edges. In some cases, the tip may have a piercing point for an initial entry. The tip may be designed to resist fogging or fouling. The tip may permit cleaning, preferably while *in situ* the surgical site.

### II. An endoscope that is partially-reusable, partially disposable/replaceable, and a coupling joint between

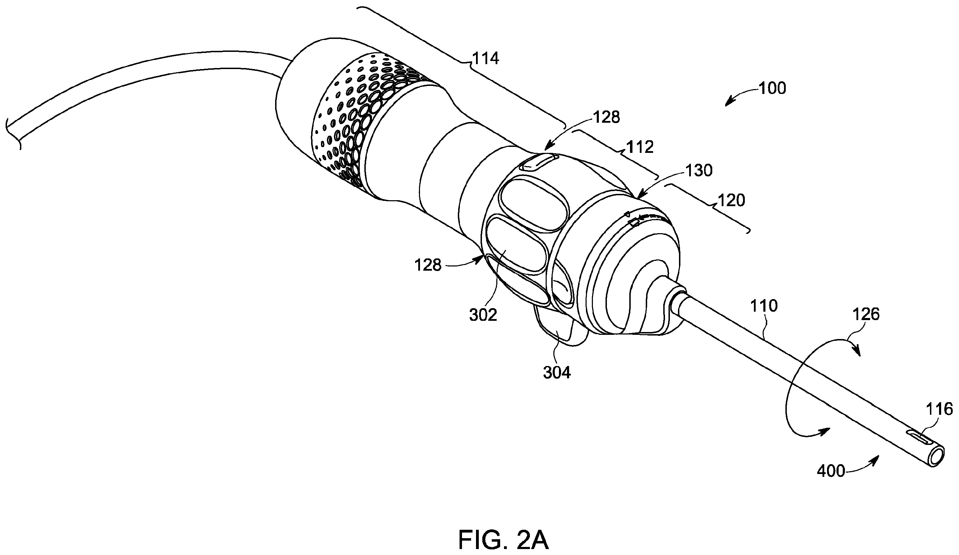

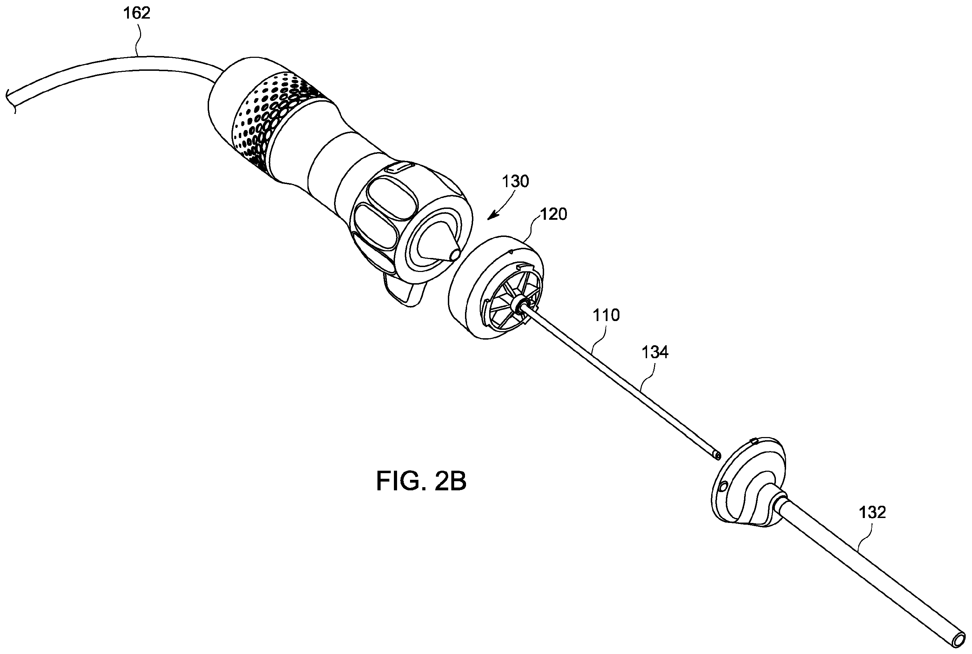

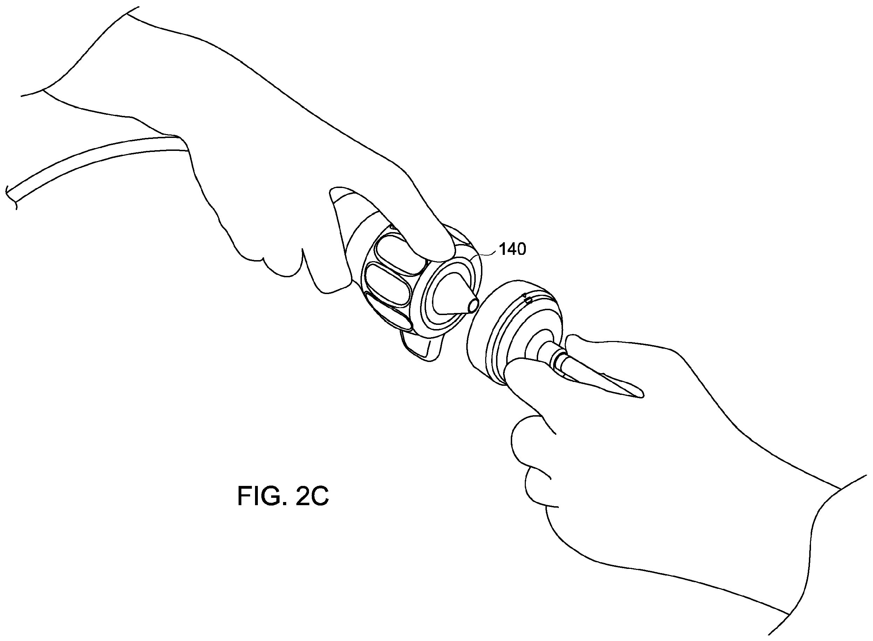

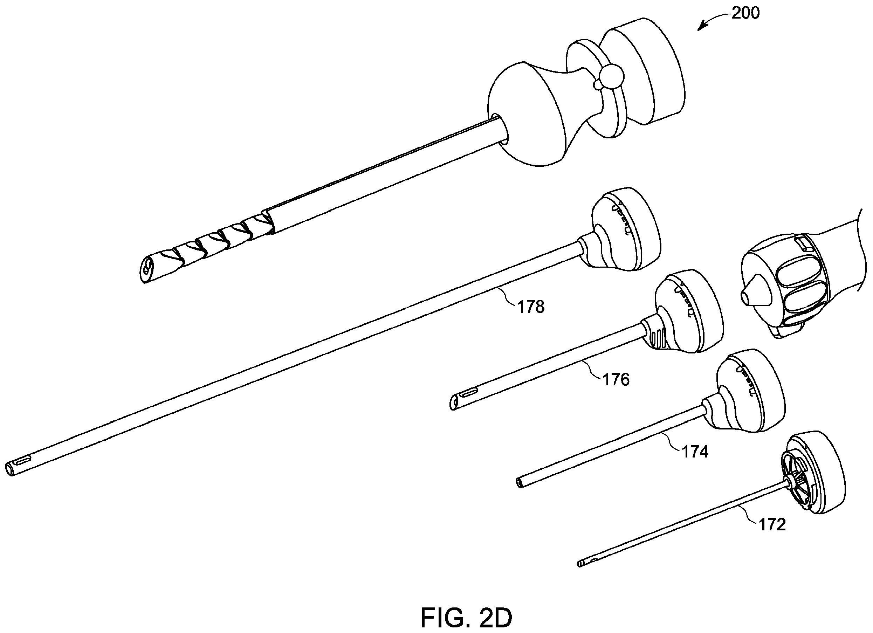

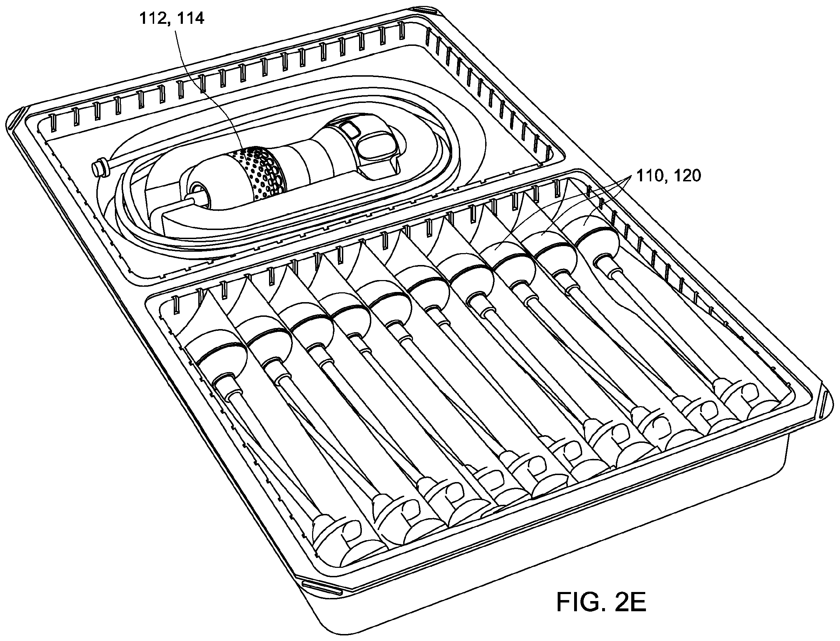

Referring to FIGS. 2A, 2B, 2C, 2D, and 2E, a surgical endoscope 100 may be structured to permit detachment of a shaft 110 portion from the endoscope's handle 112, 114. A camera or image sensor 410 at tip 116 of the shaft, any panning mechanism, illumination, power and signal connectors, and fluid flow channels may be in the disposable shaft 110. Handle 112, 114 may be designed to be reusable (which implies that handle 112, 114 may be sterilizeable, for example in an autoclave or other sterilization device, or protectable by a disposable sterility sleeve). Joint 130 between the detachable shaft and the reusable parts of handle 112, 114 may be generally distal in the handle (but not necessarily at the distal-most end). The replaceable shaft portion 110 may be disposable, along with a disposable portion 120 of the handle that is disposable with shaft 110.

Referring to FIGS. 2A and 2B, the handle of the endoscope 100 may include three principal components:
- The disposable cap 120. This distal-most portion of the handle may serve as a mounting base for shaft 110, and may disconnect from the remainder 112, 114 of the handle. This disposable cap portion 120 (along with shaft 110 and componentry inside) may be disposable.
- Rotation collar 112 may have surface features 302, 304 to allow a surgeon to rotate the rotation collar 120 about the central axis of the handle, that is, about the roll axis 126 of shaft 110. During surgery, insertion shaft 110, disposable cap 120 and rotation collar 112 may be locked to rotate with each other, so that rotating the rotation collar effects rotation 126 of the disposable cap 120 and shaft 110.
- Proximal stationary handle 114 has a shell surrounding componentry within the handle. The outer diameter and outer surface of handle 114 may be designed to provide an easy and low-slip grip for a surgeon's hand. Joint 128 between the proximal handle and rotation collar may allow these two components to rotate relative to each other. In some cases, a circuit board and similar componentry inside proximal handle 114 may rotate with disposable cap 120 and rotation collar 112, inside proximal handle 114.

Disposable cap 120 and rotation collar 112 may be separable from each other at joint 130, so that disposable cap 120 and shaft 110 may be disposable, while handle 114 and rotation collar 112 (and componentry inside them) are reusable.

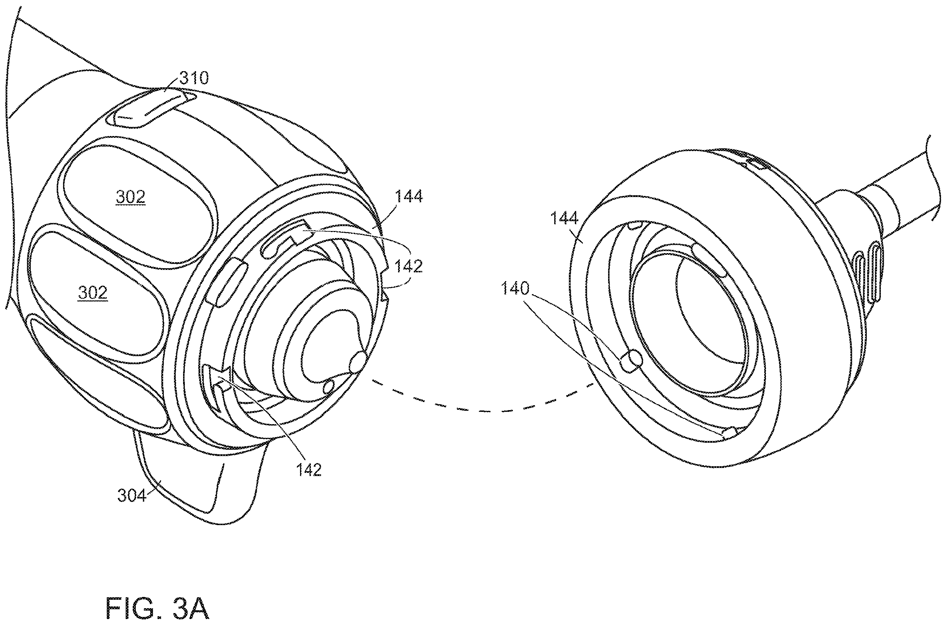

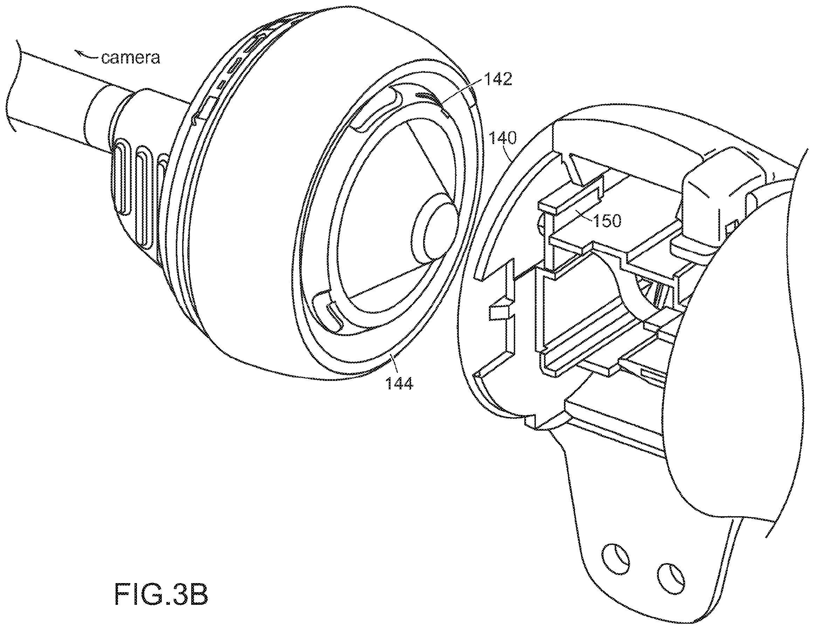

Referring to FIGS. 2A, 2B, 2C, 3A, and 3B, between the disposable cap 120 and rotation collar 112, three basic connections may be made:
- A rotation-locking coupling 140, 142 to hold the disposable portion 120 to the reusable handle 112, 114. Coupling 140, 142 may have sufficient strength to transmit insertion and withdrawal forces, roll, pitch, and yaw torques, lateral forces, and similar forces from the proximal reusable handle 112, 114 to the distal disposable portion 120 and shaft 100, thereby to allow a physician to aim the illumination and/or camera as needed. Joint 130 between disposable cap 120 and rotation collar 112 may lie generally toward the distal end of the handle. The disposable cap and rotation collar 112 may engage through flat force-transmittal surfaces 144 at the center of joint 130 and around the circumferences, so that these forces are supported around the circumference of separable joint 130. One or more release buttons 146 may be pressed or squeezed to cause one or more locking snaps 148 to disengage. The mechanical connection may include a rotatable locking ring or other release/fixation mechanisms.
- An electrical connection to supply power to the illumination source and camera 410, and to carry optical signals back from camera 410 to the processing board in handle 112, 114 and display system outside the endoscope. The disconnectable electrical connections for power and signal may be effected by a USB-C connector 150, 152, mini HDMI connector, or similar connector that can maintain signal integrity for high speed signals. If illumination is conveyed by optical fiber 430, joint 130 may include an optical connector.
- A disconnectable connection to any panning mechanism for camera 410 may be effected by a physical connector, such as a linkage.

In some cases, the camera/image sensor 410, LED, and electronic connections (and any mechanical connections for panning the camera/image sensor 410) may be removable from insertion shaft 110. Shaft 110 and cap 120 may be smooth and simple enough in shape to allow easy sterilization. Similarly, once the electronics are removed from interior of shaft 110, they may be sterilizeable as well. it may be cost-effective, especially in lower-labor-cost markets, to disassemble, sterilize, and reassemble the shaft and its interior components for reuse.

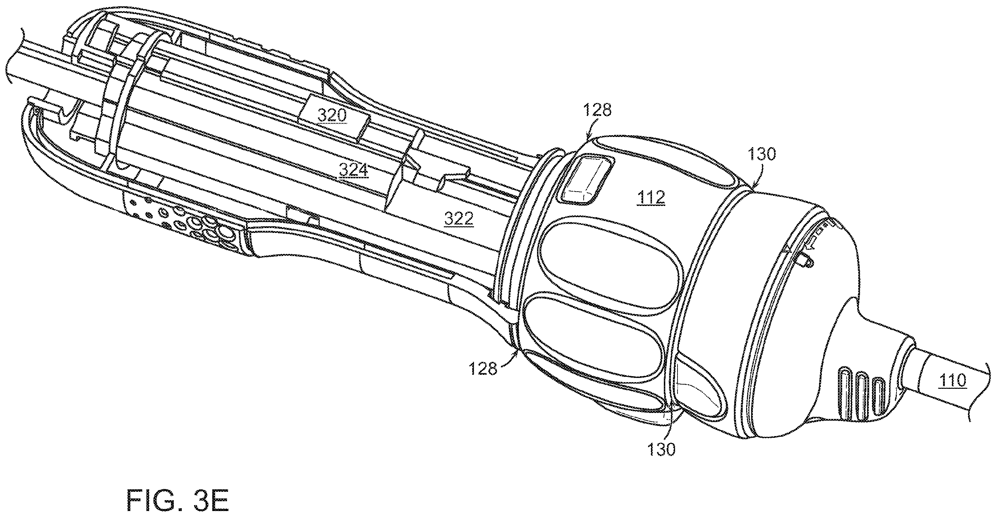

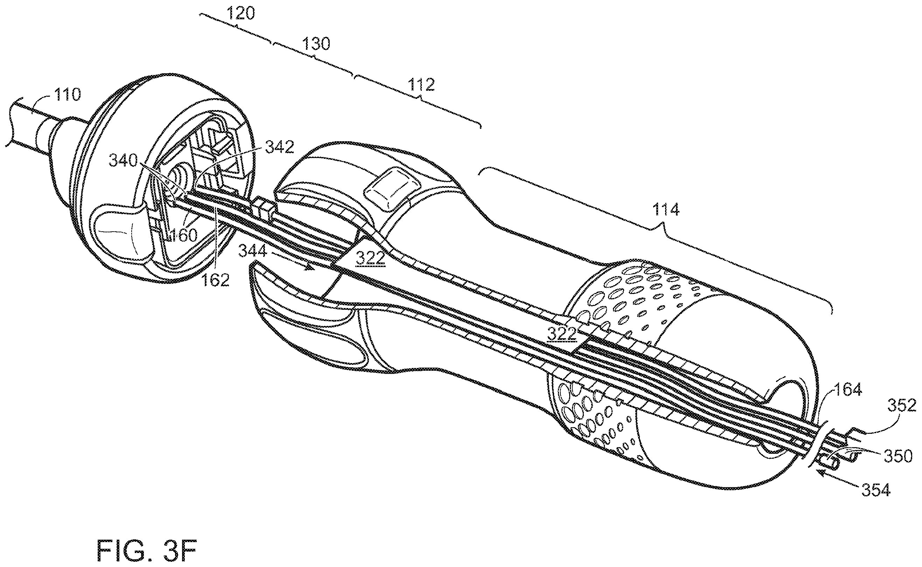

One or more fluid hoses 160 for irrigation liquid or inflation gas (or two hoses, one for fluid and one for gas) may enter through disposable cap 120, so that the entire set of fluid tubing for the irrigation/inflation channel may be disposable with the disposable shaft portion. In other cases (*e.g.,* FIGS. 3E and 3F), a fluid hose 162 may enter the proximal end of the scope, and disconnectable fluid connections within joint 130 for fluid inflow and outflow may be effected by gaskets, O rings, and the like. Alternatively, connectors for the hoses may be outboard of the endoscope itself, either near the endoscope (for applications where it may be desirable to allow "quick change" replacement of the insertion shaft 110 in the course of a single procedure), or far from the endoscope, typically at the receptacle for waste fluid, to ease disposal of all hoses that are potentially contaminated by contact with the patient.

Disposable shaft 110, 120 may be designed to facilitate disposability of components that come into contact with bodily fluids. Because sterilization is often imperfect, patient safety may be improved by disposing of components that have come into contact with patient bodily fluids. To improve sterilizability, it may desirable to reduce componentry in the disposable component 110, 120 so that cost of the disposable component may be reduced, and to reduce surface features and crevices that may be difficult to sterilize. Thus, lens 460, image sensor, LED, panning mechanism, and shaft 110 may be disposable. In addition, because shaft 110 is used for fluid inflow and outflow, and is disposable, sealing against bodily fluids may be unnecessary.

Referring to FIG. Referring to FIG. 2D, the replaceable/disposable shaft 110 and its mounting componentry 120 may be specialized to different types of surgery. For example, a purely diagnostic scope 172 may have an outer diameter of 1 to 3mm. A replaceable disposable cap / shaft unit 110, 120, 178 for laparoscopic thoracic surgery may have a shaft of 400 mm length and diameter of 10 mm. Replaceable components 176 for arthroscopic surgery of knees and hips may be 155 mm in length, and 5.5 mm or 4 mm in diameter. For small joints, a replaceable shaft 174 of 2.9 mm diameter or less may be preferred. A replaceable shaft/scope unit with an bendable end 200 may be dimensioned for laparoscopic surgery. Typical dimensions for various surgical specialties may be as follows (measured in millimeters):

| | | **Cannula diameter** | | **Scope diameter** | |
|---|---|---|---|---|---|
| **Scope Type** | **Discipline** | **Min** | **Max** | **Min** | **Max** |
| Arthroscope (small joint) | Arthroscopy | 2.8 | 4.0 | 1.9 | 2.9 |
| Arthroscope (large joint) | Arthroscopy | 4.7 | 6.0 | 2.9 | 5.3 |
| Cytoscope | Cytoscopy | | | 2.9 | 5.3 |
| Encephaloscope | ENT | | | 2.0 | 4.0 |
| Hysteroscope | Gynecology | 3.7 | 7.0 | 2.0 | 5.0 |
| Laparoscope | Laparoscopy | | | 2.0 | 10.0 |
| Sinuscope | ENT | | | 2.0 | 4.0 |
| Thoracoscope | Pulmonary | | | | 10 |

Various replaceable components 110 may have different instruments at tip 116. For example, various replaceable shafts may have cameras 410 oriented at 0° (directly on-axis), 30°, 45°, 70°, and 90°.

Referring to FIG. 2B, disposable shaft portion 110, 120 may in turn be separable into an outer cannula 132 for protection and strength, and an inner shaft portion 134 carrying various illumination, optical, and fluid-carrying componentry. The scope may be sold as a handle unit 112, 114 with a set of ten or twelve or twenty replaceable shaft/cap unit 110, 120.

U.S. App. Ser. No. 16/434,766, filed Jun. 7, 2019, and its formal drawings filed Aug. 13, 2019, are incorporated by reference.

### III. Extendable, bendable, or articulated camera tip

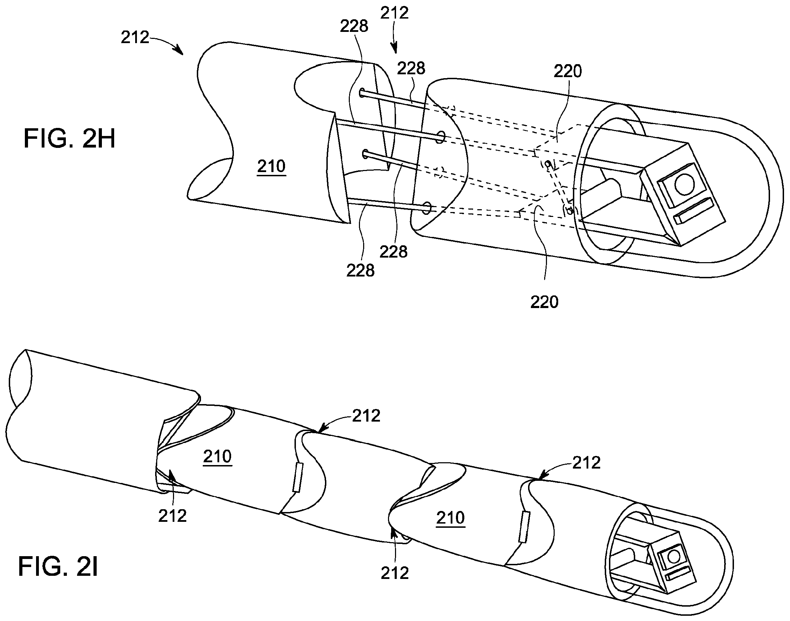

Referring to FIGS. 2F and 2G, the camera tip 202 may be slideable within cannula shaft 132, to be extendable and retractable. When extended, the distal portion of camera stalk 202 may be bendable, for example, as rigid segments 210 joined at articulation joints. In FIGS. 2F and 2G, cone 204 shows the field of view of camera 410. The extendable/bendable portion of shaft 202 may be formed of a series of elements that are each essentially rigid in the longitudinal dimension, but articulated at each joint to permit bending or flexure. The articulation may all be in the same dimension, as shown in FIGS. 2F and 2G. Alternatively, as shown in FIGS. 2H and 2I, articulation pivots 212 may be at alternating 90° angles, so that the bending may be in two dimensions, which in combination, may yield 360° of bending angles. Alternatively, the bendable portion 202 may be formed of elastic material, with an internal stiffener that is relatively stiff and incompressible against longitudinal compression dimension, and flexible in lateral and/or inclination bending. Bendable portion 202 may include two or four cable channels spaced around an outer surface, so that tension cables may cause bending in a desired direction.

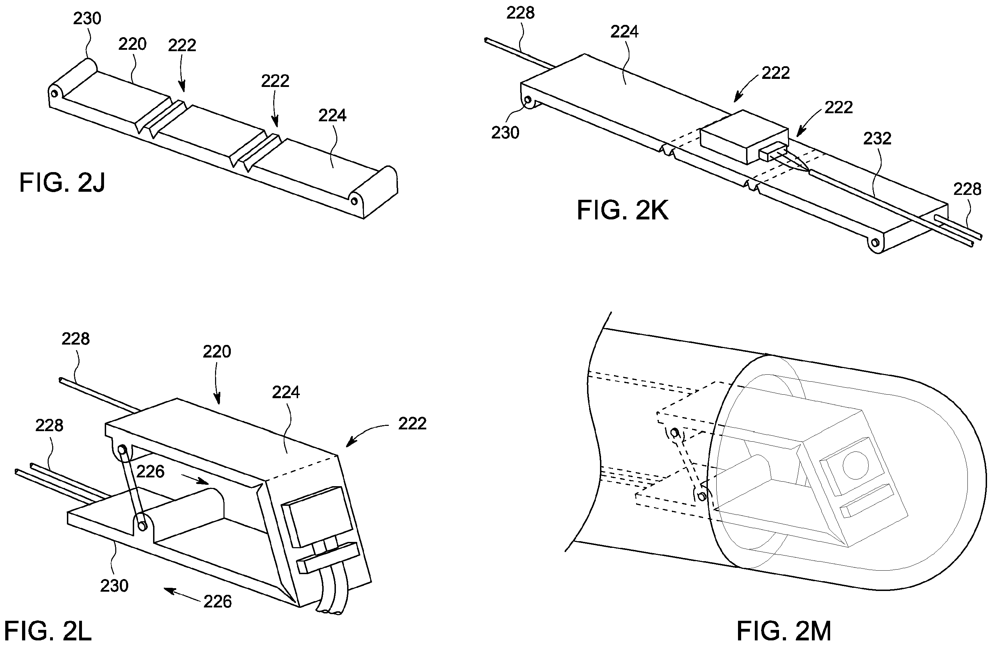

Referring to FIGS. 2H, 2I, 2J, 2K, 2L, and 2M, in some cases camera 410 may be pannable within endoscope tip 400. For example, camera 410 and its illumination LED may be mounted on one side of a substrate 220 formed as three rigid segments with two hinges 222, so that the two outer segments 224 may be moved relative to each other 226, and the center segment rotates in place, in the manner of a flexing parallelogram. The two outer segments 224 may be mounted in slide channels, and connected by cables 228 to controls at the handle. Referring to FIG. 2J, substrate 220 may be molded onto a flat flexible backing. The backing may contain folds 222 to create hinge points that allow the backing to fold into its parallelogram configuration (FIGS. 2K and 2L). Pivot points 230 may be molded into substrate 220. Referring to FIG. 2K, a flex circuit 232 may be laminated onto the substrate, and control tension cables 228 attached to the two ends. A camera, illumination LED, pressure sensor, temperature sensor, or other sensors may be affixed to substrate 220. Referring to FIG. 2L, substrate 220 may be folded into three sides of a parallelogram, and a fourth side may be formed by a linkage connected to hinge points. Further details are described in U.S. Pat. No. 9,375,139, incorporated herein by reference.

Longitudinal movement 226 of one face of the substrate relative to the other changes the angle of the center segment, and thus the angle of the image CCD or other camera, and any other sensor. This may provide an adjustable view angle over a range that may be as large as 90°. The endoscope can also accommodate for a 180° or retrograde view where the endoscope has a flat top construction and a rotatable or living hinge rectangular endoscope architecture.

Passages and apertures for ingress and egress of irrigation, inflation, or other fluids may be provided in the tip. An aperture for irrigation fluid may be aimed to clear fouling from a window or lens over camera 410.

At least one of surfaces 224 may contain a metal strip bonded onto or into segment 224. The metal strip may be a spring steel or nickel-titanium alloy with a preformed radius of curvature. The metal alloy may alternatively be a malleable metal such as aluminum or may be a nickel-titanium (nitinol) alloy with a shape memory feature. The metal strip allows the elongated core to reliably bend in one plane of curvature. Where the memory substrate is spring-steel or nitinol, it may bend to a shape if malleable, or may be made steerable with a nitinol shape-memory component.

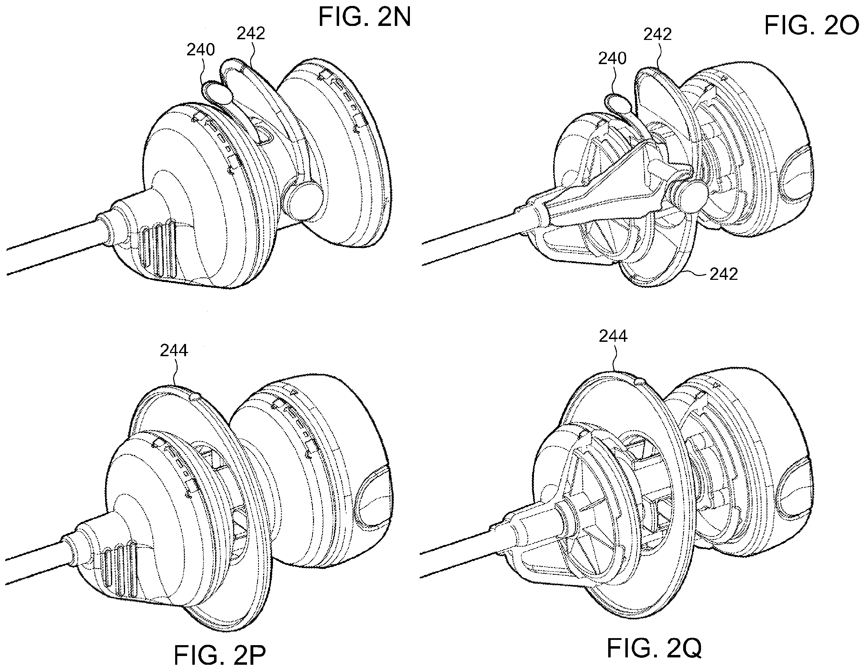

Referring to FIGS. 2N and 2O, lever 240 may be moved to advance/project or retract/withdraw camera 410 within insertion shaft (FIGS. 2F and 2G). Another switch/lever 242 (for example a paddle-shaped switch) may control cables or levers that flex the articulable tip by exerting tension on cables 228 that extend to the tip, to cause rotational articulation at joints 212 along the extendable portion 202 of shaft 110, thereby to control articulation of the camera tip to move in positive-y and negative-y directions. Another lever may be used to control camera panning (FIGS. 2H, 2I, 2J, 2K, 2L, and 2M).

Referring to FIGS. 2P and 2Q, a four-point control 244 may control four control cables 228 or rods or other load bearing components to the articulated or bendable portion of the extendable/retractable and/or articulated camera shaft 202, so that the camera tip may be articulated in positive-x, negative-x, positive-y, and negative-y directions.

Referring again to FIG. 2D and 2E, the extendable/retractable and/or articulated camera shaft 110, 200 may be used with a reusable handle, disposable tip configuration. The extendable/retractable and/or articulated camera shaft may be used with a reusable or single-use unibody scope configuration.

The articulated camera tip 200 may be especially useful in abdominal thoracic laparoscopy. Typically, during abdominal surgery, the abdominal cavity is inflated with carbon dioxide, to give the surgeon a large open field of view. This gives an extendable/retractable and/or articulated tip space to move. The extendable/retractable and/or articulated tip may be useful to provide a view behind an organ, such as the stomach or liver. If the surgeon only has a fixed view endoscope/laparoscope, the only way to obtain a view behind an organ would be to open another port from the opposite side of the body.

### IV. Additional features of an endoscope

Referring to FIGS. 2A and 2B, disposable shaft portion 110, 120 may in turn be separable into an outer cannula 132 for protection and strength, and an inner shaft portion 134 carrying various illumination, optical, and fluid-carrying componentry. Illumination may be provided by an LED at or near the distal tip, or via fiber optics from an illumination source in the handle, or illumination at an external controller.

Referring again to FIG. 2A, the endoscope may have a handle 112, 114, 120, and a shaft 110 for insertion into a body. At or near distal tip 116 of the shaft 110 may be a camera, electronic image sensor, or other optical component. The camera's orientation may be fixed in the scope, or may be pannable. Camera 410 may be at tip 116, looking out from the shaft, or may be recessed a short distance behind the structural tip of the shaft. Also at or near the tip may be an illumination source, such as an LED. Tip 116 may have a rigid pointed tocar tip, or may have a spoon-shaped portion that reaches past the image sensor, or may be flexible (in the manner of the tip of a colonoscope), in each case extending a little beyond imaging camera to provide physical protection to the camera/image sensor 410 during insertion or to protect camera/image sensor 410 from a surgical cutting device.

Illumination may be in visible light, infrared, and/or ultraviolet. In some cases, an illumination LED (light emitting diode) or other illumination source may be placed in reusable handle 112, 114 or in a docking station/controller, and the disposable shaft may have fiber optics 430 to transmit light to the tip, and joint 130 may have an optical coupler. In other cases, the illumination LED may be placed in tip 116 to illuminate the surgical cavity directly; in such cases, joint 130 may have a power connector. In some cases, the LED may be recessed from the tip, or placed somewhere in the shaft, or may be in an external controller, and optical fiber 430 may carry illumination light to the tip. Optical fiber 430 may be configured, for example, with a split, so that light will be arrayed in a desired pattern around the image sensor to better distribute the light into the surgical cavity around the image sensor.

The shaft 110 itself may be rigid, made of a nonbioreactive metal such as stainless steel or coated aluminum. In some cases, a surgical cavity around endoscope tip 400 may be insufflated by gas (typically carbon dioxide), or irrigated by saline solution. In either case, fluid inflow and outflow may be effected by channels through the shaft.

Shaft 110 may also carry power wires to the illumination LED and camera 410, and carry signal wires that carry an optical signal back from camera 410 to electronics in the reusable portion 112, 114 of the handle. Electrical power to camera 410 may be supplied over conductors in a flexible cable or on a printed circuit board (flexible or rigid), and insulated with a conformal and insulating coating such as parylene. This same flexible circuit board 416 may have signal conductors for the video signal from camera 410. The video signal may be transmitted from camera 410 to the handle using any video signal protocol, for example, MIPI (Mobile Industry Processor Interface) or HDMI. Parylene may also improve biocompatibility.

Shaft 110 may also carry cables or other mechanical elements to control panning of camera 410.

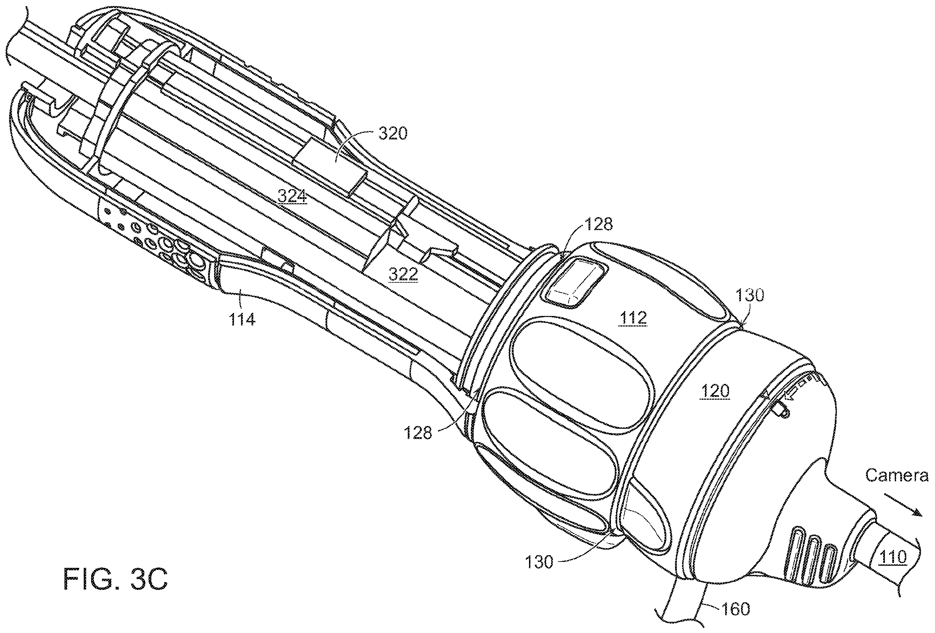

Referring to FIG. 3A, 3C, and 3E, rotation collar may have various features that make rotation easy. For example, depressions 302 may provide a good grip for fingers for light roll torque. Fin 304 may provide greater leverage for greater roll torque, and may also provide a fixed rotational point of reference.

A button 310 may perform various functions, such as turning illumination LED on or off, taking pictures, starting and stopping video, and the like. A single button may perform all these functions based on the nature of the press. For example, press-and-hold for 3 seconds may turn the illumination LED on and off. A quick press may capture a single-frame still picture. A double-click may start and stop video recording.

If camera 410 at the tip 116 of shaft 110 is pannable or has other controllable features, there may be a control (for example, a lever, or a touch-slide panel, etc.) near button 310 to control that adjustment of camera 410.

One or more ultraviolet LEDs or other illumination source may be placed inside handle 112,114, inside shaft 110, or near tip 116 to assist with insuring sterility of the internal components of the device or of the water as it passes thru the device

Referring to FIGS. 3C, 3E, and 3F, irrigation/insufflation hose(s) 160, 162 may enter at various points through the handle. For example (FIG. 3C), irrigation/insufflation hose(s) 160, 162 may enter laterally, somewhere near the distal end of the handle, for example, through fin 304. Or, as shown in 3E and 3F, irrigation/insufflation fluid/gas hose(s) 160, 162 may enter through the proximal end of handle 114. This hose may then be disconnectable via a fluid disconnect joint 320 within joint 130. In cases where hose(s) 160 for insufflation fluid/gas enters through disposable cap 120 (FIG. 3C), various joints and strain relief features 340 may be used to hold hose(s) 160 in place.

Referring to FIG. 3B and FIG. 3F, electrical connectors 150, 152 such as USB-C or mini-HDMI connectors may be used to connect camera 410 to a circuit board interior to handle 114.

Referring to FIG. 3B, rotation-locking coupling 140, 142 may lock disposable cap 120 in rotational relationship to rotation collar 112. Various rigid and resilient features 144, 148 may lock them together for other forces and torques, and release buttons 146 may permit them to disengage to allow replacement of disposable cap 120. The coupling between cap portion 120 and rotation-locking coupling 140, 142 may place much of the stress at the periphery of the joint, so that joint 130 may carry and transmit forces (especially torques) well.

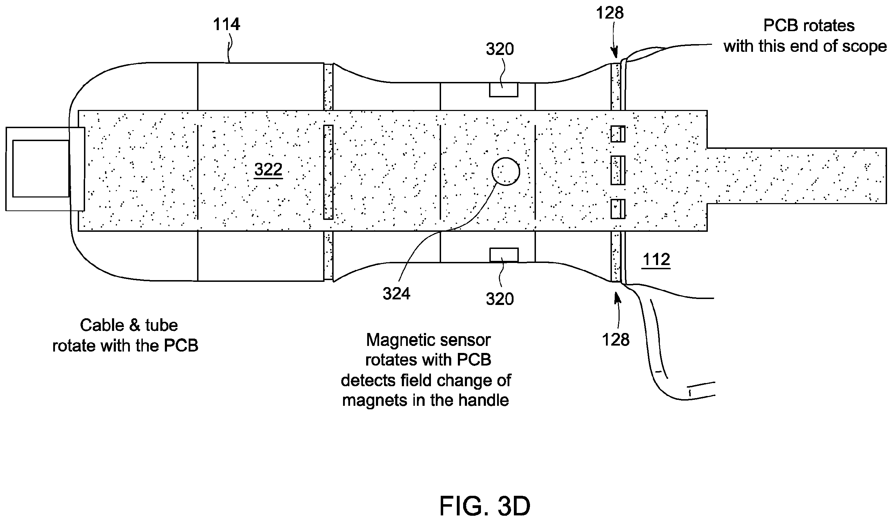

Referring to FIG. 2A, 3C, and 3D, rotation between the handle's stationary portion 114 and rotation collar 112 may be provided via a rotational bearing at joint 128.

Referring to FIGS. 3C, 3D, and 3E, proximal handle 114 may contain a number of components, typically components that have only incidental patient contact (and therefore present less risk of cross-infection), are higher in cost (and therefore desirably reusable), and either sterilizeable or may be covered by a sterility sleeve. For example, proximal handle 114 may hold power transformers, signal amplifiers, controls for the illumination LED and camera, a mechanical control for panning camera 410, rotation sensors for righting of an image from camera 410, and the like. The handle may also include connections to external sources and destinations of power, signal, fluid, and the like.

Proximal handle 114 may include rotational sensors so that an angular orientation of camera 410 may be ascertained. For example, the inner surface of proximal handle 114 may mount one or more magnets 320, and printed circuit board 322 (which rotates with rotation collar 112 and disposable cap 120) may have Hall effect sensors 324 that detect the magnets. This may be used to compute a rotational orientation, which may in turn be used to "right" the image from camera 410 on a video display screen.

The distal tip of the shaft, camera 410 mounted therein, and the mounting of componentry within shaft 110 may be designed to be robust. Occasionally, during surgery, the tip of the endoscope may come into contact with a shaver, ablation probe, or cauterization probe, and it may be desirable to have the tip be robust to such contacts. To reduce risk that componentry may be dislodged and left in the patient, the disposable shaft and its componentry may be designed to avoid joints that are at high risk of mechanical failure. A disposable optical system may prevent the image degradation that occurs when nondisposable optics are reused in multiple surgical procedures.

Endoscopes as a genus include arthroscopes, laparoscopes, colonoscopes, and other specialized scopes for various body cavities. For an arthroscope for joint surgery, the shaft may be as small as 4.5mm, 5mm, 5.5mm, or 6mm, and highly rigid. For other endoscopes, such as a colonoscope, the diameter may be larger, and the shaft may be flexible.

The endoscope may be delivered as a handle and multiple tips, each tip individually sealed for sterility.

Referring to FIG. 3F, hoses 160, 162 for irrigation/insufflation fluid/gas in, irrigation/insufflation fluid/gas out, and electrical connection cord 164 may be permanently affixed 340, 342 to disposable cap 120. This arrangement may allow that hose 162 that carries water out of the surgical cavity, and which is therefore contaminated, may be disposable, and no fluid will come into contact with the reusable part 114 of the handle. Hoses and cord 160, 162 may be routed through channel 354 running the length of reusable handle 112, 114. Channel 344 may be of inner diameter large enough to permit easy passage of hoses and cord 160, 162, 164, and connectors 350, 352, and have a continuous smooth wall that permits easy sterilization, to permit ready replacement of the replaceable components. Channel 354 may be off the central axis, to allow printed circuit board 322 to lie on the central axis. Connectors 350, 352 at the end of hoses and cords 160, 162 may be small enough to pass through channel 354. Thus, replacement of shaft 110, cap 120, hoses and cords 160, 162 may be effected by threading connectors 350, 352 and hoses and cord 160, 162 through channel 344. Electrical cord 164 may have a connector 354 at or near joint 130, and hose(s) 160 for irrigation/insufflation fluid/gas flowing into the surgical cavity may likewise have a connector at joint 130 to allow this hose(s) to be reusable, or may be permanently affixed 340 to reduce possibility of leaking. Having hoses and cable 160, 162 roughly on-axis reduces undesirable cable flop as the scope is in use, and reduces undesirable torque on cap 120. Forming shaft 120, cap 120, and hoses 160, 162 as an integral unit for replacement reduces possibility of leaking, and improves sterility of the replacement operation.

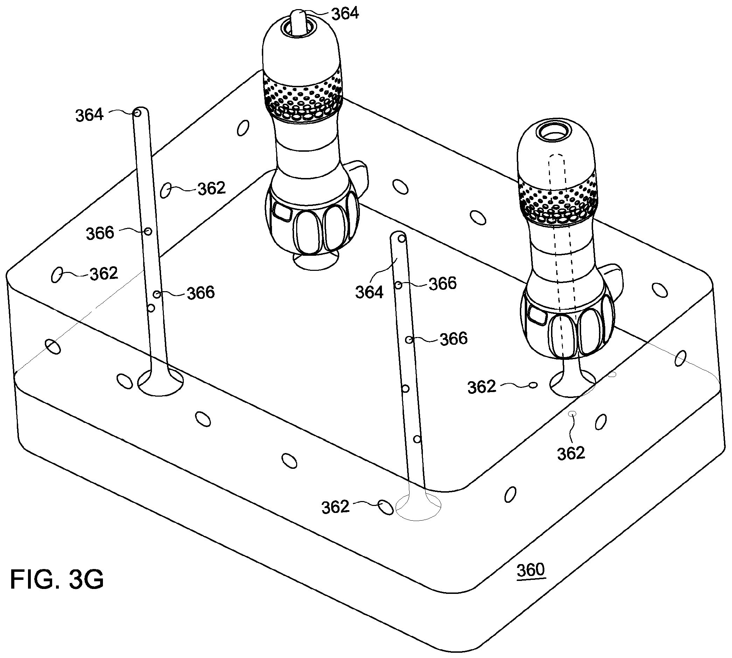

Referring to FIG. 3G, reusable handles 112, 114 may be sterilized in a sterilizer 360. Preferably, hose(s) 160, 162 and all other portions of endoscope 100 that come into contact with the patient, or with fluids that have come into contact with the patient, are disposable, and the design for reusable portions 112, 114 reduces contamination by avoiding contact with the patient's bodily fluids. Sterilizer 360 may be arranged to accept one or more reusable handles 112, 114, and irradiate them with ultraviolet light from ultraviolet LEDs 362. Rods 364 that pass through handle channel 344 may have ultraviolet LEDs 366 arranged along their lengths, to sterilize internal channels 344.

### V. Endoscope tip

Components of endoscope tip 400 may be designed to permit a camera or vision sensor 410, and an illumination emission source, and an objective lens or window to be mounted within a confined space, such as an endoscope or an arthroscope for joint surgery, having a diameter of 6mm or less, 5.5mm or less, 5 mm or less, 4.5mm or less, or 4mm diameter or less. In some cases, fluid management may be managed in the same space. In some cases, the shaft may have the strength and rigidity commonly found in arthroscopes. In some cases, the illumination emission may be by one or more LEDs located at or near the endoscope tip. In other cases, the illumination emission may be via fiber optical fibers 430 and/or light guides 450 that conduct illumination light around the camera or vision sensor 410, within the diameter of shaft 110.

### V.A. Molding of components of the endoscope tip

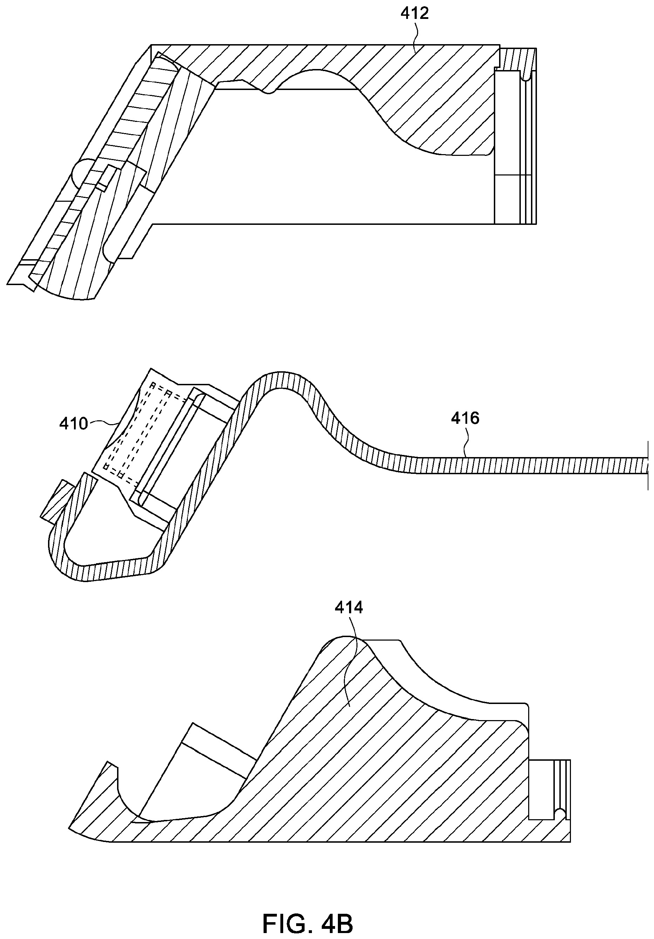

Referring to FIGS. 4A and 4B, endoscope tip 400 may be formed of top brace 412, bottom brace 414, and flexible circuit board 416. Camera 410 may be mounted on one side of flexible circuit board 416, and an illumination LED on the other side. Clear window 420 may protect camera 410 from the outside environment, such as the tissues and bodily fluids of an endoscopic procedure, and pressurized insufflation fluids. The entire assembly may be locked together via overmolding.

Referring to FIG. 4B, to assemble the components, one end of flexible circuit board 416, the end with the LED mounted thereon, may be slotted into a slot or channel in top brace part 412, which holds the LED into place. Then board 416 may be folded around a bend in top brace 412, so that camera 410 comes into place through its hole in top brace 412. The folding and rotation brings the LED close to the sensor, which permits the assembly to fit within the 5mm diameter of tip 400. Then bottom brace 414 may be brought into place, which holds top brace 412, bottom brace 414, circuit board 416, LED, and camera 410 in their positions of mutual alignment. A locking notch and clip, or ultrasonic welding may hold this assembly together for a time. Then an overmolding step may lock everything together.

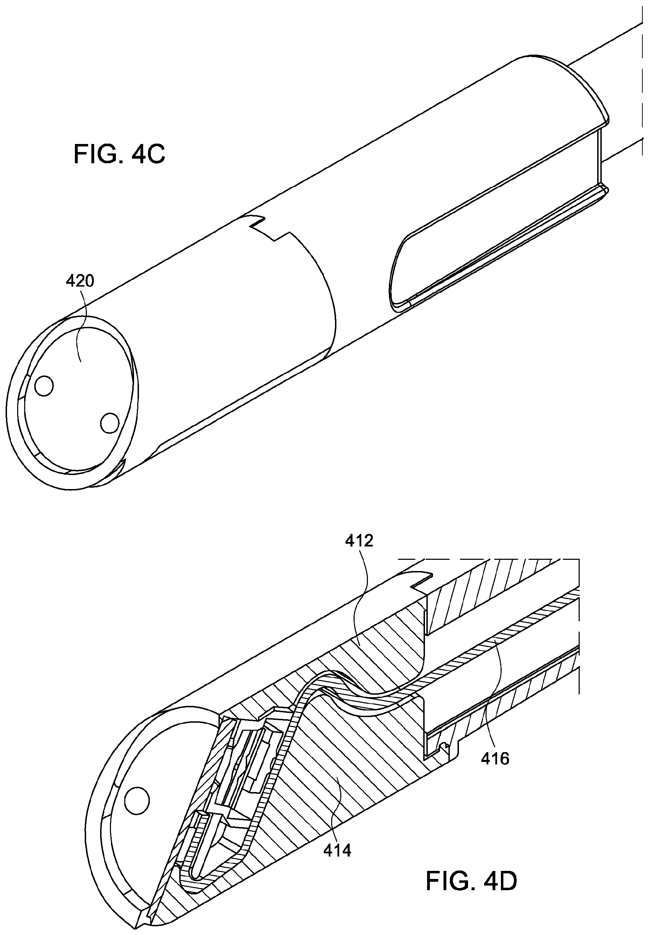

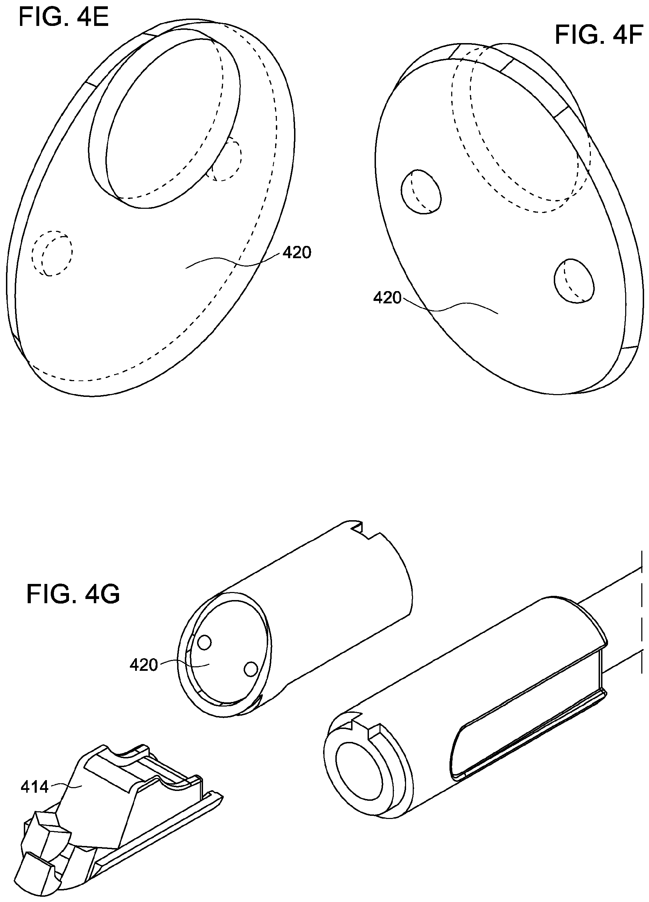

Referring to FIGS. 4C, 4D, 4E, 4F. and 4G, transparent window 420 may cover camera 410 to protect it. Window 420 may be two thicknesses, a thicker region over camera 410, and a thinner region for the portions used for mounting and for the LED to shine through. A peripheral ridge of endoscope tip 400 may extend beyond window 420 by a small amount. Top brace 412 may include a wall that surrounds the LED. These shapes, alone or in combination, may offer one or more of the following advantages. First, these shapes may reduce stray light from the LED being internally reflected into camera 410. Second, the thickness of the window 420 on the lens side may reduce vignetting artifacts, when the edge of the field of view of a camera image is occluded or lens 460 gathers less light toward its edge. Likewise, the shape of the lens may be used to reduce distortions such as fisheye distortions. Third, the ridge may tend to keep tissues away from lens 460, reducing obscuring and improving view.

Window 420 of FIGS. 4E and 4F may be placed over the surface of the assembly of circuit board 416 with LED and camera 410, top brace 412, bottom brace 414, and window 420 (FIGS. 4B, 4C). Then the assembly with window 420 may be locked together via overmolding of a covering sheath (FIGS. 4C, 4D, 4G). This overmolding may provide watertightness to the entire tip assembly. The overmolded assembly may then be fitted onto the tip of the endoscope's insertion shift.

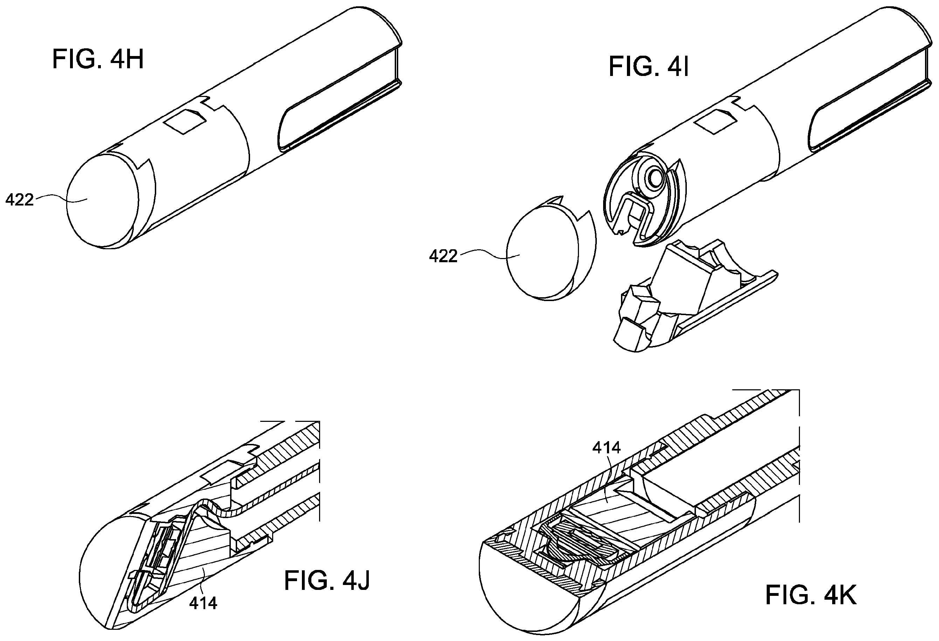

Referring to FIGS. 4H, 4I, 4J, and 4K, in other cases, in an alternative, clear window 422 may be overmolded onto top brace 412, before circuit board 416, LED, camera 410, and bottom brace 414 are assembled. As window 422 is overmolded, a flat platen may be placed to project through camera 410 hole to provide a mold surface, to provide an optically smooth back surface of window 422. The mold may be flat (planar), or may have a desired curvature to form a convex or concave lens in overmolded window 422. As shown in FIG. 4K, the circumferential edges of top brace 412 may be shaped to provide a secure lock that engages overmolded window 422. Then circuit board 416 with LED may be inserted into the slot, and folded around top brace 412, and then bottom brace 414 may be snapped into place and ultrasonically welded.

Referring again to FIG. 4A, at its top right and bottom right corners, and referring to FIG. 4B, at the top right corner of top brace 412 and the lower right corner of bottom brace 414, energy directors molded into top brace 412 and bottom brace 414 may improve ultrasonic welding. Ultrasonic welding may improve adhesion and watertightness among top brace 412, bottom brace 414, and flow director components. The energy directors may be molded as triangular-profile ridges on the inside surface of the top-brace-bottom-brace assembly. The inner diameter of the energy directors may be slightly smaller than the outer diameter of the flow director, so that ultrasonic welding causes the energy directors to melt and to form a seal.

Taken together, these features may provide an endoscope tip 400 of very small diameter, such as 4mm or less, 5mm or less, 4.5mm or less, or 4mm or less, or a tip 400 slightly larger than an endoscope shaft, with all components fitting inside that tip diameter. Mounting the LED and camera 410 on opposite sides of flexible circuit board 416 may assist in making the entire assembly more easily manufacturable. That manufacturing may involve inserting the end of a flexible circuit board 416 into a slot, and wrapping board 416 around a molded part or wrapping board 416 into a channel between molded parts to place various components in their preferred operating orientations. This positioning of board 416, including bending and wrapping, may build some additional slack into the positioning of board 416, which may create some strain relief and improve reliability. Components may be ultrasonically welded together. Overmolding may be used to structurally hold components together and to provide a watertight seal. Clear window 420, 422 may be overmolded onto a structural component, or the structural components may be overmolded over a clear window.

### V.B. Fiber optic illumination for a single-use use scope tip

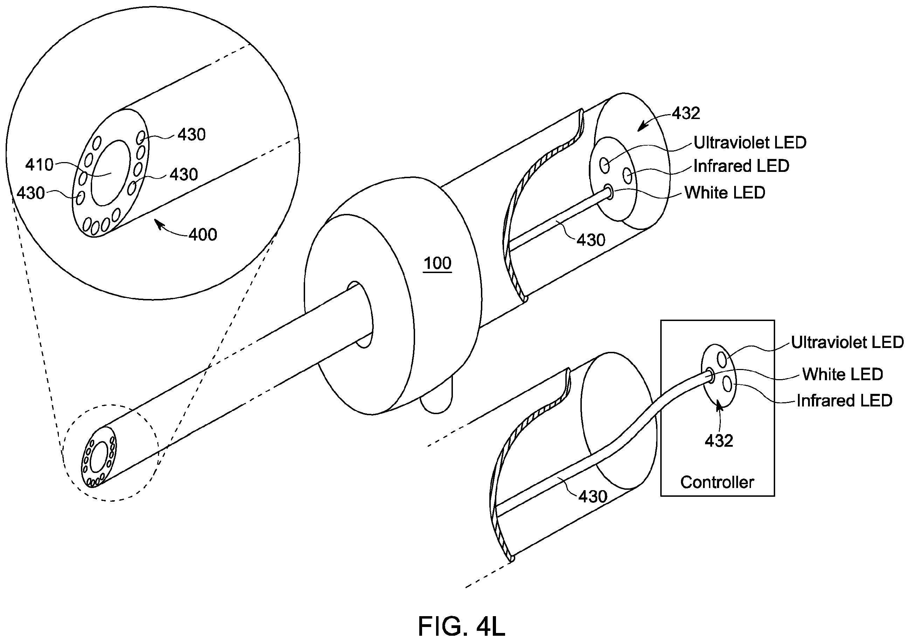

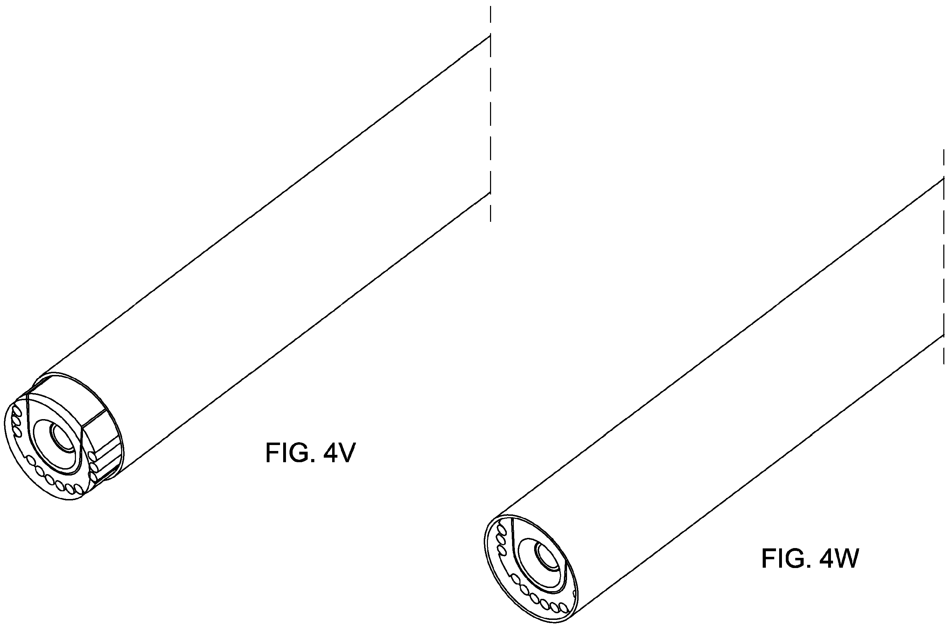

Referring to FIGS. 4L and 4W, a single use endoscope 100 or single-use tip for a reusable handle may have camera 410 on the tip. Single use endoscope 100 may use fiber optical fibers 430 to deliver illumination light. Plastic optical fibers 430 may offer an attractive combination of attributes for disposable or single-use endoscopy applications, including cost, flexibility to bend around curves and for motion during a surgical procedure, numerical aperture (the cone of angles over which the fiber radiates light, and the cone within which it accepts), low heat radiation at the surgical site, and manufacturing resilience. Fiber optic illumination may deliver illumination adequate for applications such as laparoscopy, where the objective surface may be 200 or 300 mm from camera 410, while avoiding problems of heat dissipation that may arise by placing an LED at the tip. Fiber optic illumination may reduce complexity of chip on tip circuitry in the confined space of endoscope tip 400. Fiber optic illumination may permit use of multiple illumination sources of varying wavelength to be coupled at the collection end of the fiber, to change the illumination at endoscope tip 400.

Referring to FIG. 4L, one or more illumination sources 432 may be located either in the reusable endoscope handle or base station/IPU/Master Controller. Illumination source 432 may be one or more of single color LED, a white light source, a tricolor white LED, infrared or ultraviolet light, etc. Illumination source 432 may be an LED, combination of LEDs, flash lamp, incandescent lamp, or other illuminator. Fiber 430 may be coupled to illumination source 432 at the collector end by butt adjacency or other collection mechanisms. In some cases, where multiple illumination sources 432 are provided, they may be on a rotating carousel, sliding multiplexer, or other switch that brings successive ones of the multiple illumination sources into butt adjacent coupling with the coupling end of the light fibers 430. Light source devices 432 that are the same size or slightly larger than the collector end of optical fiber 430 offer the most efficient butt adjacency coupling.

Plastic light fibers 430 are available as fluoridated polymer optical fibers tradenamed Raytela^{™} from Toray Industries, Inc. of Japan. Plastic light fibers 430 may reduce cost relative to glass fibers 430, which may be an especially important consideration in a single-use or disposable endoscope design. Plastic optical fibers 430 may be formed of two different plastic resins that have two different indices of refraction, the higher index resin used as a core, and the lower index resin used as a cladding layer. The boundary between the layers may provide total internal reflection to conduct light down the fiber 430. The diameter of fibers 430 may be chosen to optimize several simultaneous characteristics. The amount of light that can be carried per fiber is roughly proportional to cross-section area. The cost of optical fiber is primarily proportional to length, with a smaller cost growth with diameter. Likewise, manufacturing cost generally grows with the number of fibers, and grows with the number of fibers that break or are damaged during manufacture, so fewer larger-diameter fibers tends to be lower cost. On the other hand, mounting camera 410 and any working channel apparatus is generally more difficult, and the optical fibers are easier to fit into a small space if they are smaller diameter, which tends to favor a larger number of smaller-diameter fibers 430. To optimize among these tradeoffs, in some cases, at least nine fibers, at least twelve fibers, or at least 15 fibers may be used. The fibers may be about 0.4mm, 0.5mm, 0.6mm, 0.75mm, or about 1mm in diameter. They may be placed around the periphery of the working tip 400 of scope 100.

Referring to FIGS. 4L and 4W, in some cases, fibers 430 may be relatively uniformly spaced around the 360° periphery of tip 400. Greater uniformity of the placement of the illumination fibers 430, and centering on camera lens 460, may reduce variability of illumination across the imaging field, shadows, and other undesirable artifacts. In other cases, fibers 430 or the distal face of light guides 450 may be distributed over some arc less than 360°, such as at least about 180°, at least about 240°, at least about 250°, at least about 260°, at least about 270°, or at least about 300°. In some cases, the endoscope may be used very close to the anatomical structures on which surgery is performed, so distributing the illumination emission around the periphery may reduce glare and hot spots. In some cases, larger fibers 430 may be used for part of the periphery, and smaller fibers 430 may be used for a part of the end of the endoscope that is crowded with other mechanical components. The closer the fibers 430 can approximate a uniform 360° distribution, the more uniform the lighting, and thus the better the image. Using fibers 430 with a larger numerical aperture or other dispersion at the end may likewise improve dispersion, and thereby improve uniformity of illumination and image quality. Non-circular fibers 430 may be used to allow greater surface area of the illumination end of the fibers, and thereby provide better illumination.

Referring to FIGS. 4M and 4N, camera 410 may be mounted on a flex circuit board 416. Lens 434 may be held n place by an inner tip part 436, and these parts may be assembled into a lens subassembly 460.

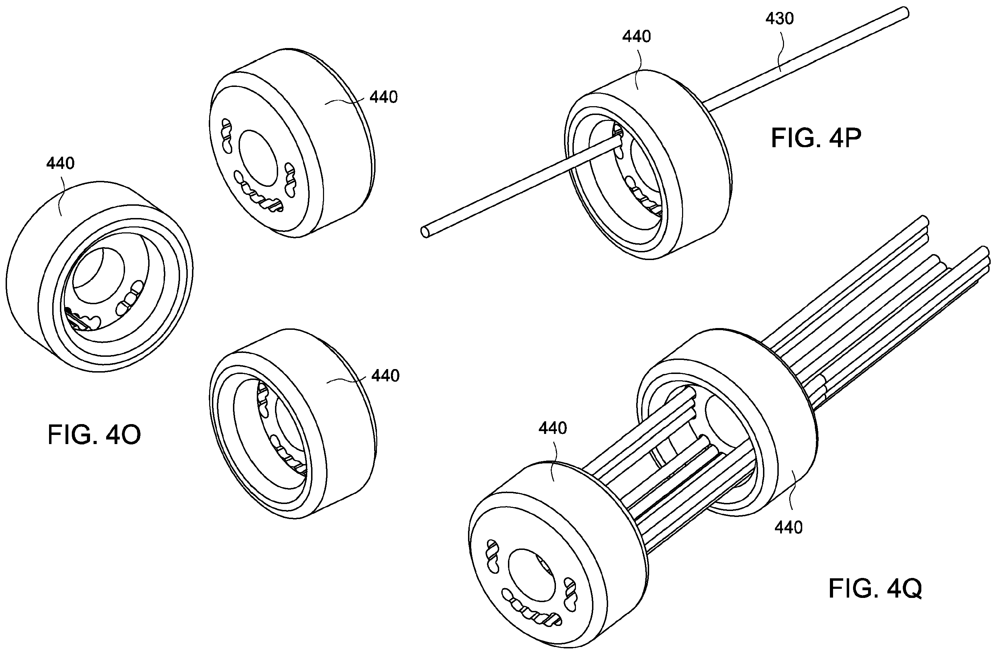

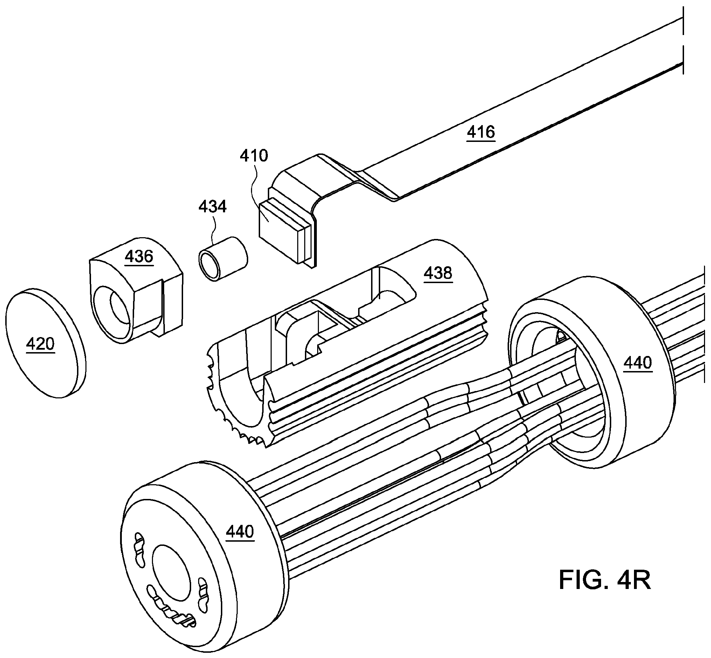

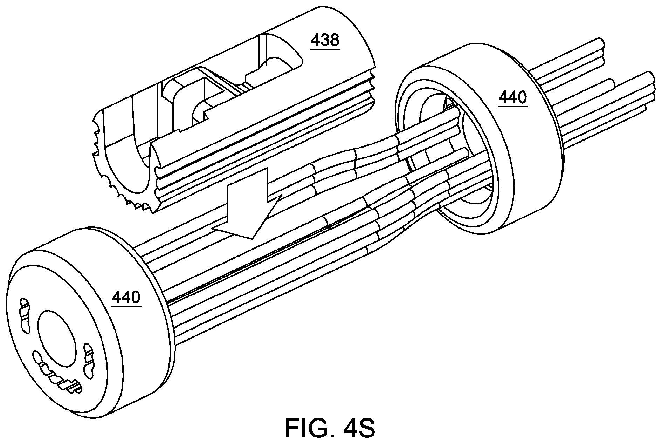

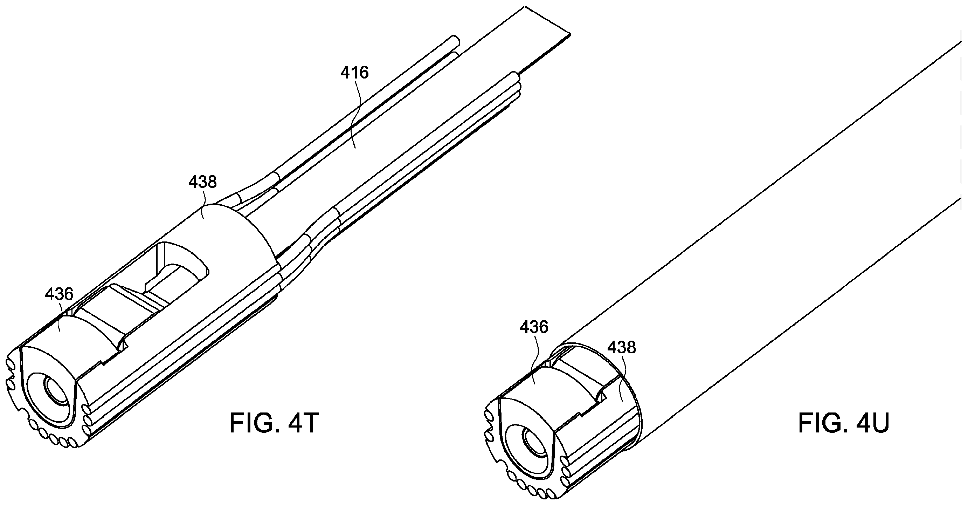

Referring to FIG. 4O, two jigs 440 may have a cap form, with inner diameter matching the outer diameter of tip chassis 438, and with throughholes conforming to the position of channels on the outer surface of tip chassis 438, the channels providing circumferential positions for the optical fibers 430. The holes in the two jigs are typically in pair-wise opposition to each other. Referring to FIG. 4P and 4Q, optical fibers 430 may be routed through the holes in the jig parts, from matching hole to matching hole. Referring to FIGS. 4R, 4S, and 4T, tip chassis 438 may have grooves along its periphery to capture fibers 430, and a set of inner channels and features to hold the camera/inner tip part assembly. Referring to FIG. 4S and 4T, tip chassis 438 may be placed into the nest of fibers held in place by jigs 440. Referring to FIGS. 4R and 4T, the camera/lens/inner tip part assembly may be placed into the tip chassis 438, and flex board 416 laid into a channel formed by the optical fibers. Then rear jig 440 may be slid off the end of the flex board 416 and fibers 430.

Referring to FIG. 4U and 4V, fibers 430 may be cut to match the surface of tip chassis 438. The ends of the fibers may be polished, or the ends may be treated to diffuse light, as discussed below in section V.D. The ends of the fibers may be covered with window 420, typically glass or sapphire. The outer/distal surface of window 420 may be coated to accept a coating, such as that described in U.S. App. Ser. No. 16/069,220 and U.S. Provisional App. Ser. No 63/193,387, incorporated by reference. Window 420 may have a coating to reduce lighting artifacts, such as reflection. Window 420, 422 may be held in place by an adhesive. This adhesive may fill any air gap between the end of optical fibers 430 and window 420. Removing the air gap may improve light transmission from fibers 430 through the window 420. In some cases, a lens or window 420 may be overmolded over the assembly shown in FIGS. 4T and 4U. Referring to FIGS. 4T, 4U, and 4V, the assembly of the fibers 430, tip chassis 438, inner tip part 436, camera 410, and flex board 416, and window 420 may be threaded through the stainless steel tube of the scope's insertion shaft, and slid into place for either friction fit, or to be held in place by an adhesive, such as epoxy or an optical grade UV-curing adhesive (available form Norland Products Inc. of Cranbury NJ, from the Henkel Adhesives division of Loctite, from Dymax Corp. of Torrington CT, and others), or to be held in place by a detent or deformation of the tube. Adhesive may also serve to seal the tip against intrusion by fluids. Referring to FIG. 4W, the surface of camera 410 may be recessed into the stainless steel tube. The surface of window 420 may be flush with the end of the tube, or may be recessed slightly, to provide protection against scratching or dislodgement of the window. The recess may be covered by a clear lens, either entirely flat, or with features or lenses analogous to those shown in FIG. 4E and 4F.

### V.C. A tip design with light guides

Light fibers 430 may be extruded in shapes that improve light delivery, such as rectangular, or a U-shaped light guide 450 that would extend the length of the endoscope from the illumination source to the U-shaped emission surface at the distal end of the endoscope. In some cases, individual fibers 430 may be replaced, for at least some portion of their length, by a shaped light guide 450, such as a circular or U-shaped ring of clear light guide around the periphery of the tip chassis 438, 480. Light guide 450 may be a two-component structure, with two different indices of refraction for internal reflection analogous to optical fiber. In other cases, light guide 450 may be formed of a clear light transmission medium coated by a reflective coating, such as aluminum, gold, or silver. In some cases, the shaped light guide 450 may extend only a short distance, for example, the length of the inner tip part, and traditional circular fibers may be used to bring light from the illumination source to the proximal end of light guide 450 at the tip chassis 438, 480.

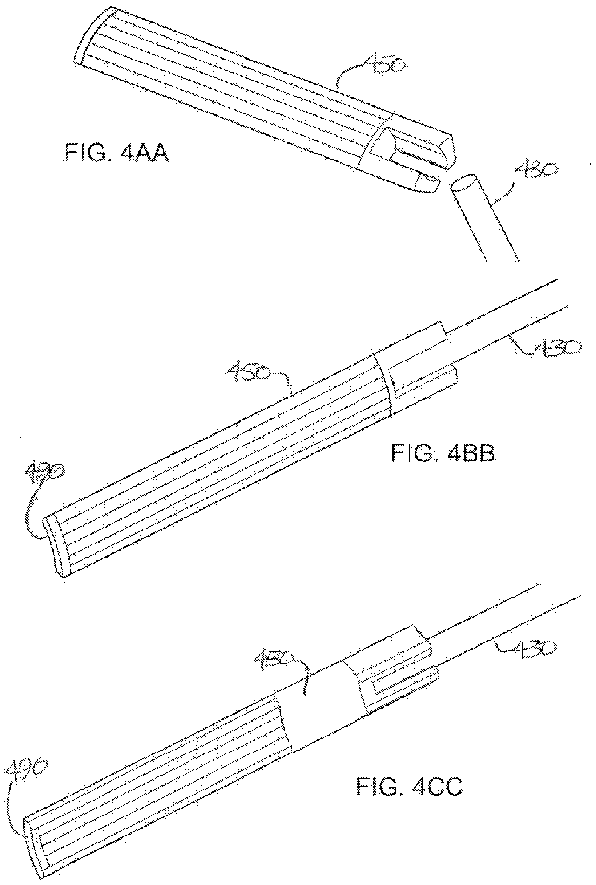

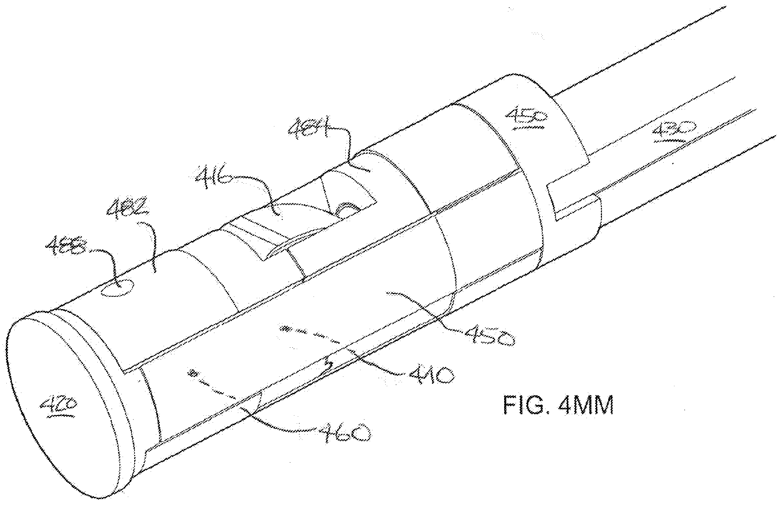

Referring to FIGS. 4X to 4EE and 4MM, light guides 450 may lie along the periphery of the tip components to conduct light from optical fibers 430 to the distal tip of the endoscope around the molded and image capture components of the tip. Light guides 450 may be made of clear optical plastic or glass. Outer diameter of shaft 110 may be designed to be small to reduce trauma and tissue injury, while camera 410 and other components can only be as small as permitted by technology. Thus, it may be desirable to reduce diameter of the light conveyance from the rear of the tip structure to the face of the tip, while maintaining a cross-sectional area sufficiently large to convey enough light. It may be desirable to arrange the illumination emission around a large fraction of the periphery of the scope tip to provide uniform lighting. Referring to FIG. 4X, 4Y, 4Z, 4AA, 4BB, and 4CC, three or four light guides 450 with an arcuate cross section may be arranged around the circumference of the tip. Each may accept one or more light fibers 430 that conveyed light from LEDs in the handle or control box.

FIG. 4Y shows light guide 450 in cross-section, showing the narrowing from a larger-diameter fiber 430 (perhaps 1mm) at the point of connection to light fiber 430 down to a thickness of about ½mm or less to squeeze the light flow between the chassis components 438, 480 in the center, and outer metal shaft 110. The angle of narrowing section 452 may be less than the internal reflection angle of the material of light guide 450. In narrowing area 452, the surfaces may be highly polished, to effect high internal reflection. The axis of fiber 430 may enter at the center of the thickness of light guide 450. Under this arrangement, the cross section of 1mm diameter fiber 430 and the cross-section of light guide 450 may be about equal, with narrowing region 452 designed to capture all of the light from fiber 430 and direct it into light guide 450, through a channel narrowed to about 0.5mm between camera chassis 438, 480 and the wall of the shaft 110.

Referring to FIG. 4Z, the surface of light guide 450 may be concave fluted or scalloped 454. The entry 456 into the fluted region at the proximal end of the flutes 454 may be tapered at an angle less than the angle of total internal reflection of the material Fluting 454 may provide two advantages.

First, fluting 454 may encourage the light to organize to flow down light guide 450 as the light emerges from narrowing region 452 of light guide 450. A light conduit in the region to conduct light past the constriction of the camera at the tip of the endoscope may balance two somewhat-contradictory conditions: it is desirable that the maximum light flow through light guide 450, and simultaneously, illumination light should be dispersed across the entire field of view of camera 410. Dispersion of fibers alone is limited by the numerical aperture (the angle of dispersion or collection at the two ends of the fiber), which is the angle of internal reflection, which in turn is governed by the difference in index of refraction between the core layer of the fiber, the cladding layer, and the ambient material around the fiber (typically air). Dispersion greater than the numerical aperture of the fiber may allow illumination light to reduce darkness at the edges of the field-of-view of the lens (roughly 70°) of camera 410. Fluting/scalloping 454 may create an appropriate level of dispersion within light guide 450, so that when the light emerges from light guide 450, the dispersion at the tip of the scope may nearly match the field-of-view of lens 460.

Second, organizing light guide 450 so that contact between the inner surface of shaft 110 and the outer surface of the plastic light guide, and between the inner surface of light guide 450 and outer surface of chassis 438, 480 occur on line contacts, which may reduce light leakage. The wall of light fiber 430 has two internal reflectance interfaces, one between the core and cladding layers of fiber 430, and one between fiber 430 and the external air. The shape of light guide 450 may be designed to increase air surround, and to reduce contact with epoxy or other materials, in order to reduce light leakage.

Light guides 450 may permit easier and higher-throughput direction of illumination light in a 30°, 45°, 60°, or 70° offset scope, which may reduce the brightness, power consumption, and heating at the illumination LEDs.

Referring to FIGS. 4AA, 4BB, and 4CC, light guide 450 may be formed to accept fiber 430. A coupling may be arranged to capture and transfer as much light as possible from fiber 430 to light guide 450, and may be arranged to structurally retain fiber 430 to ease assembly.

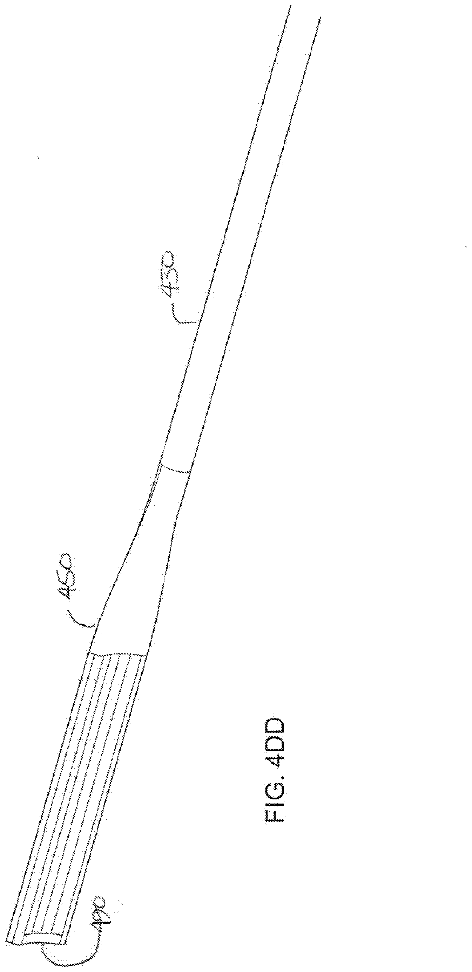

Referring to FIG. 4DD, light guide 450 may be formed by deforming fiber 430 itself. This may effect a higher-throughput coupling from fiber 430 to light guide 450 than butt-coupling of FIGS. 4AA, 4BB, and 4CC. The light fiber may be deformed by gentle heating, for example, in steam, and then applying pressure. The pressure may be applied in a highly polished mold, to provide a highly smooth surface. A shaped fiber end may provide greater tolerance to small misalignments during manufacture, compared to the high precision placement required for butt-coupling.

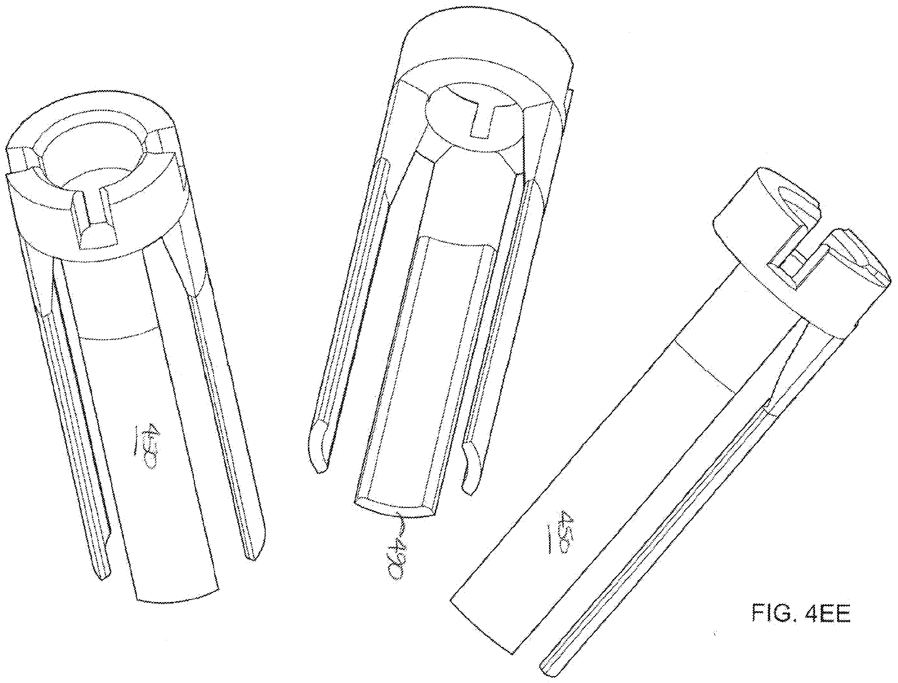

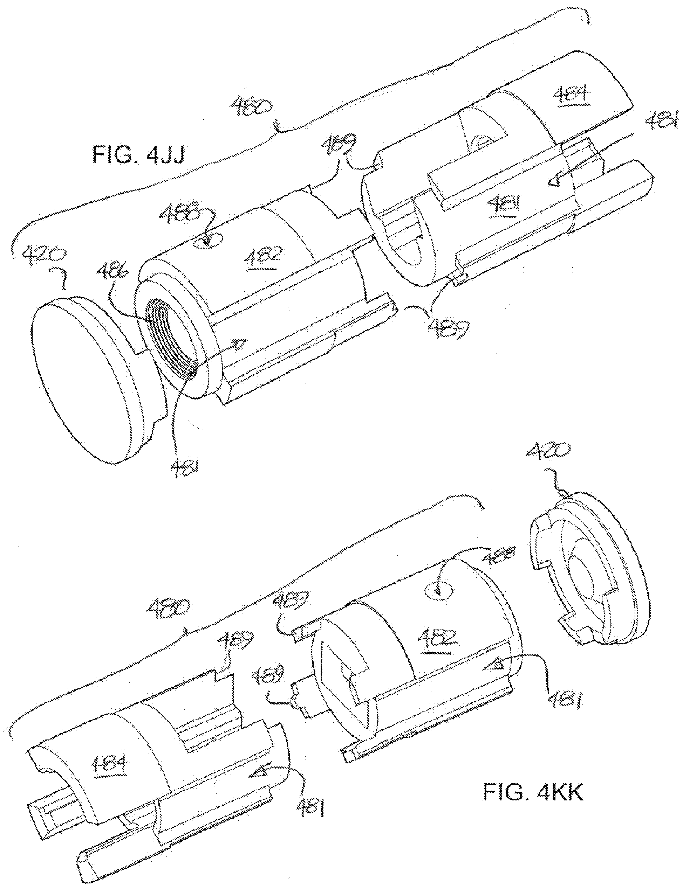

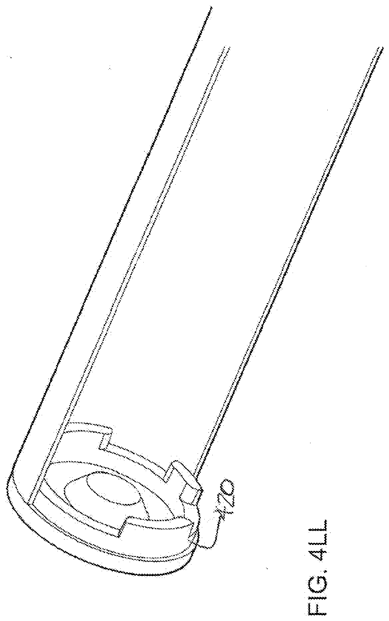

Referring to FIG. 4EE, three light guides 450 may be molded together as a single part. This may ease assembly in two respects. First, instead of assembling three small parts, this technique may permit an assembly process to manage one larger part. Second, the three prongs of light guide 450 may hold the entire assembly of front chassis 482, rear chassis 484, lens assembly 460, camera sensor 410, and flex board 416 (FIGS. 4JJ, 4KK, and 4LL) together into a unit for further assembly.

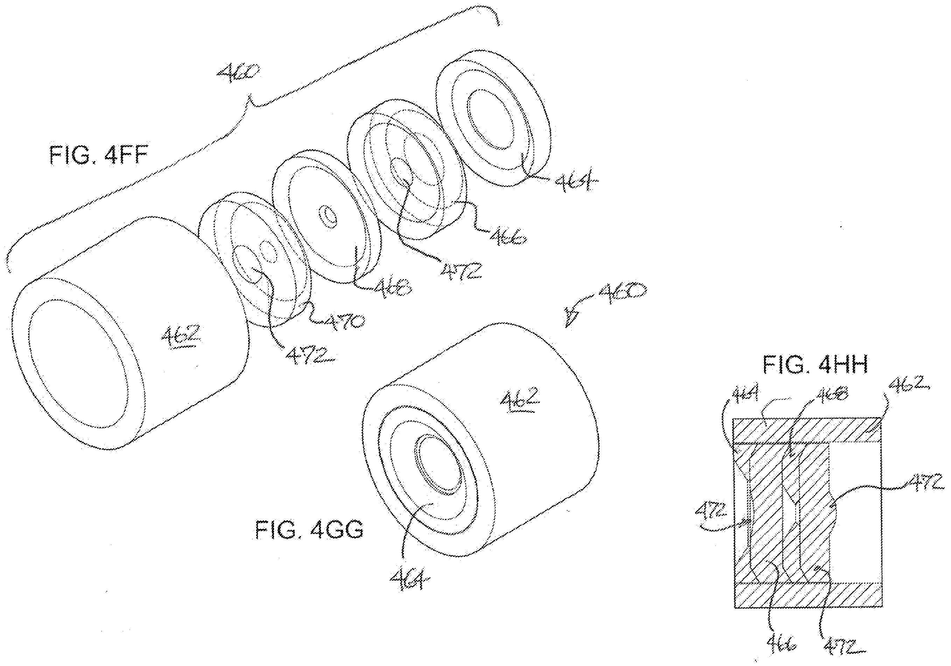

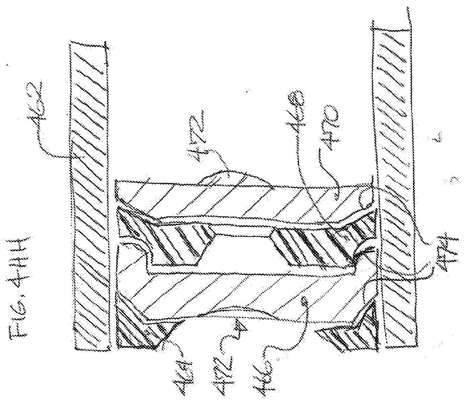

Referring to FIGS. 4FF, 4GG, and 4HH, lens assembly 460 may be formed in a tube 462 that encloses an end cap 464, a first lens 466, a spacer/iris 468, and a second lens 470. Circular parts 464, 466, 468, 470 may be about 1mm or 1.2mm in diameter. To ease reliable assembly, shapes 474 embody poka-yoke principles so that they will only stack one way. For example, the conic angle, straight-vs-curvature, etc. 474 may vary at the different joints, so that the parts cannot be assembled in incorrect orders. For the two lens parts 466, 470, the lens itself is only the center circle portion 472 (which appears similar to an eye cornea in FIG. 4FF). The optical lens is shown in FIG. 4HH as the depression 472 in the front lens and raised bubble 472 in the rear lens. Center spacer 468 may have a precise lateral depth to ensure correct spacing between the two lenses 466, 470, and a relatively small center aperture to block excess light. Outer cap 464 may function as a positioning spacer, as a flange to capture the other parts, and/or to block excess light. Excess light to be blocked may be light leaking from light guides 450, or may be light reflected indirectly from within the surgical cavity but outside the image area. Excess light may be blocked so that it that does not degrade image quality.

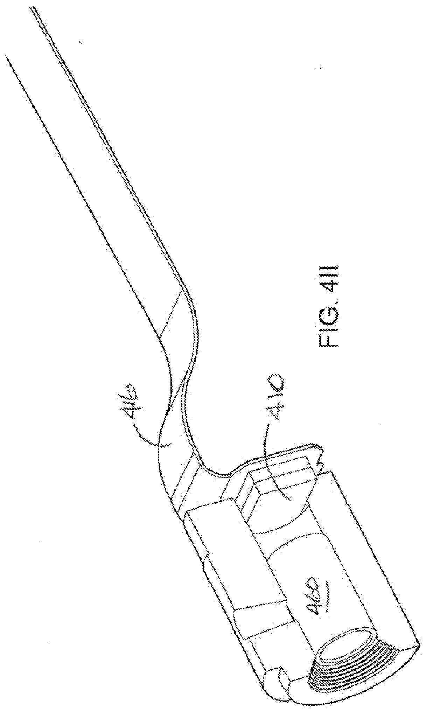

Referring to FIG. 4II, camera sensor chip 410 may be mounted on flex circuit board 416. Referring to FIGS. 4JJ, 4KK, a tip may be formed using front chassis 482 and rear chassis 484, which in turn, hold camera 410 and a cover window/lens in place. Front and rear chassis parts 482, 484 may be molded as single parts of opaque plastic. The sides of the front and rear chassis 482, 484 may have channels 481 that hold light guides 450 to the periphery of the chassis 480. The front and rear chassis 482, 484 may have features 489 at the joints that mate in only one way (for example a round protrusion on front chassis 482 that mates with a round recess in the rear chassis, and squared-off mating parts to ensure angular reproducibility). Front chassis 482 may have a stepped cone aperture 486 to reduce stray light interference reaching the camera. Rear chassis 484 may have an opening so that it does not contact light guide 450 in the narrowing region, because the internal reflection angle of the fiber optic components is higher when backed against air than when backed against plastic. Lens assembly (460 from FIGS 4GG, 4HH, 4JJ, and 4KK) may be mounted in front chassis 482. Then rear chassis 484 may be slid over the length of circuit board 416 so that circuit board 416 extends through the center hole of rear chassis part 484. Then the camera sensor 410/circuit board 416 may be mounted to front chassis 482. Then rear chassis 484 may be mated to front chassis 482 , which holds lens assembly 460, camera sensor 410, and board 416 in place relative to the two chassis parts 482, 484. This approach may reduce bending of board 416, which may reduce risk of straining the flex board 416 and its electronics, but still builds some slack and strain relief into the assembly.

Chassis 480, 482, 484 may in turn mount a clear window. Window 420 may be molded and glued in place, or may be overmolded last as the molding step that holds the other components together. Light may be communicated from light fibers to the face of the scope via light guides 450.

At this point, placement of lens 460 may be calibrated. In some cases, lens tube 462 may be made of ferromagnetic or paramagnetic material, so that magnets may be used to move lens assembly 460 within the front chassis 482 to focus the lens on image sensor 410 to improve focus, focal range, and field of view. As shown in FIGS. 4JJ and 4KK, a drop of adhesive may be applied through a microneedle through weep-hole 488 to secure the lens assembly in place. This also seals weep hole 488 against fluid intrusion. Then light guides 450 of FIGS 4X to 4EE may be added in channels 481 at the sides of chassis 480, and the window 420 may be added on the front of chassis 480. The assembly is shown in FIG. 4MM.

Referring to FIG. 4LL, the window part may have a step to engage with the edge of shaft tube 110. The step labyrinth may provide sealing against intrusion by fluids and pressurized atmosphere that is typical to surgical insufflation. In some case, the window may be secured and sealed with adhesive.

The front and rear chassis 480, 482, 484 then hold lens assembly 460, camera image sensor 410, and flex board 416 and hold them in proper spatial relation within shaft 110. This reduces part count. Chassis 480 may hold all of the components together in an assembly that can be mounted in shaft 110 in a single operation, which may ease manufacturability. The parts 474, 489, may use poka-yoke design techniques, so that the configuration of the parts allows assembly only one way, and calls attention to errors before they propagate.

### V.D. Diffusion terminal surface

In some cases, the distal surface 490 of fibers 430 or light guide 450 may be roughened or coated with a diffusive coating, analogous to the coating used to coat the inside of soft white light bulbs. By diffusing the light at emission end 490 of fiber 430 or light guide 450, the dispersion angle may be increased, which increases the cone of illumination and width of field, and may reduce undesirable shadows and other artifacts. In some cases, dispersion may be accomplished by a holographic diffuser in fiber(s) 430 or light guide(s) 450. In other cases, a diffuser may be imposed by a random process such as sandblasting, molding against a sandblasted surface, or by some similar random process. In other cases, one or more texture patterns may be photo-etched in the steel of the mold for the tip of the a fiber(s) or light guide(s) 450. One example texture may be a series of micro-domes, small circular features each having a lens profile designed to diffuse light. The microdomes may be randomly placed and of random size to avoid collimation or diffraction in specific directions, which could result in cold spots. In some cases, distal surface 490 may be roughened by a rough grinding process, analogous to the early stages of grinding a lens. Opal glass may be embedded in distal end 490 of light guide 450. The distal end 490 may be textured with other diffusion patterns such as circles, lines, or hexagons.

### VI. Antifouling and antifogging

### VI.A. Heating

Lenses may also be fogged by condensation of water vapor from the body cavity that is being operated on. Endoscope tip 400 may be the coolest point in the bodily cavity because it is nonliving tissue with no metabolism, and because operating rooms are typically kept quite cool and the stainless steel insertion shaft conducts that cold from the ambient room air to the tip. In some cases, the tip of the endoscope may be heated. In some cases, an illumination LED may provide slight heating, which may reduce condensation and fogging on the tip. The heating need only be by a few degrees, enough to ensure that the endoscope is not the coolest point in the cavity. In some cases, apertures for insufflation fluid (saline, carbon dioxide, or other, as the case may be) may be oriented to direct the fluid flow over the window surface to provide additional cleaning.

### VI.B. For endoscope's delivery packaging, vial of fluid to protective a coating on the endoscope's lens/window

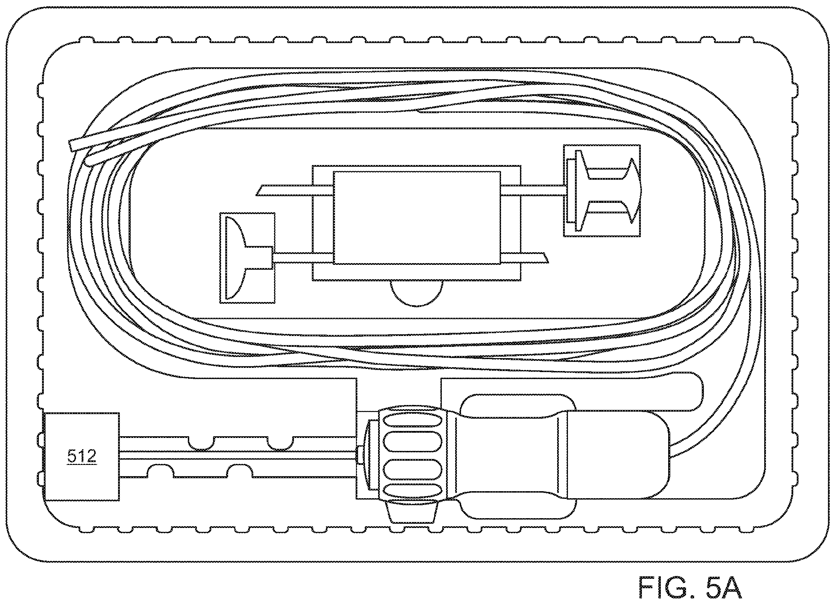

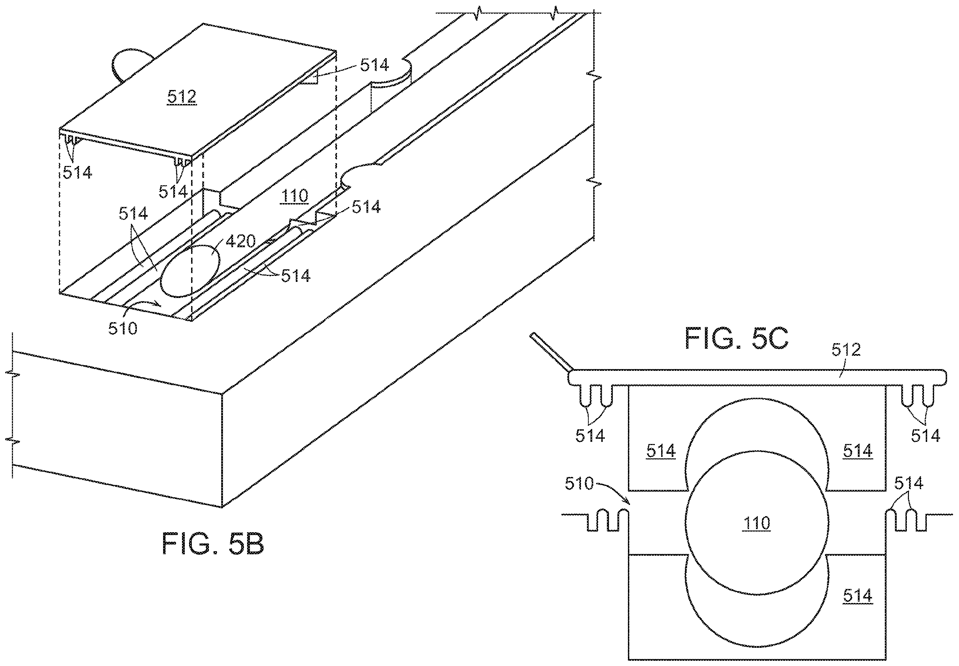

Referring to FIGS. 5A to 5C, optical lenses/windows of obturator 104 and/or endoscope may be coated with an anti-adhesive coating. Shipping or delivery packaging for endoscope 100 and/or obturator 104 may incorporate a well, vial, or other containment structure 510 to hold an anti-adhesive lubricant in contact with the coating on the objective lens or window 420 of the endoscope between the time of manufacture or refurbishing up to the moment of use, to improve the coating. Well/vial 510 may have a cap that retains the lubricant in contact with the lens/window matrix surface, to preserve the coating. The cap may have a seal against the outside environment, and a seal that seals around a shaft of the endoscope. The lubricant may be held in place over the endoscope lens/window by a rigid cap, or a flexible condom.

A lens/window 430 of endoscope 100 may have a coating to enhance optical or mechanical properties of the endoscope, and packaging for the endoscope may incorporate a vial or well or cap 510 of lubricant to maintain infusion into the retention matrix. The coating may be an anti-adhesive coating to reduce accumulation of contaminants on the surface of the lens/window, so that forward view of the endoscope remains clear. The anti-adhesive coating may be applied in two steps, first to build a porous matrix or network for retention of a lubricant on the surface of the lens/window, and then the lubricant. The lubricant may be an oil, or other liquid or gel, so that the lubricant acts as a liquid-on-liquid surface. The infused liquid may be a silicone oil (Momentive or Gelest polydimethylsiloxanes, such as 10 cSt, 350 cSt, 500 cSt), perfluorinated fluid (perfluoroperhydrophenanthrene, or Vitreon, and perfluoropolyethers or (PFPEs) of 80 cSt to 550 cSt: DuPont Krytox series), or other liquid or gel with appropriate combinations of high transparency, low surface energy, appropriate viscosity and volatility so it will be retained on the surface, chemical inertness, and prior US Food and Drug Administration (FDA)-approval. Suitable anti-adhesive coatings are described in Thin Layer Perfluorocarbon (TLP) coating developed by the Hansjörg Wyss Institute for Biologically Inspired Engineering at Harvard University, described at https://wyss.harvard.edu/technology/tlp-a-non-stick-coating-for-medical-devices (incorporated by reference), and in U.S. Pat. App. Ser. No. 16/069,220, Anti-Fouling Endoscopes and Uses Thereof, filed Oct. 24, 2018 (incorporated by reference), and commercially developed as Slippery Liquid-Infused Porous Surfaces (SLIPS) coatings and other repellent coatings and additives by Adaptive Surface Technologies, Inc. of Cambridge, MA, described at https://adaptivesurface.tech and its subsidiary pages (incorporated by reference), and as described in Steffi Sunny, et al., Transparent antifouling material for improved operative field visibility in endoscopy, Proceedings of the National Academy of Sciences U.S.A., 2016 Oct 18;113(42):11676-11681. doi: 10.1073/pnas.1605272113 (Sep. 29, 2016) (incorporated by reference).

The well or vial may be formed so that the cover includes appropriate seals 512 to retain the protective oil or gel. For example, edges around a cover may have a labyrinth seal. Two components of an end wall may each embrace slightly more than 180 degrees of the endoscope shaft so as to form a seal. Two components of an end way may have labyrinth seals against each other. The nature of the seal 512 may vary depending on the viscosity of the lubricant, and the surface energy of the lubricant vis-à-vis the material of the cap.

When endoscope 100 is placed in use, any excess of the lubricant may be wiped off. If the lubricant is bio-inert and nontoxic, it may be left in place to protect the lens during some phase of penetration.

Referring again to FIG. 5A, the shipping, delivery, or storage case for the endoscope may have compartments for one or more scope insertion shafts, for a handle, for various hoses and cords, obturator 104, in addition to the well or vial or other retention for anti-adhesive lubricant.

### VII. Lens cap with optical correction for use during insertion of offset-view endoscope

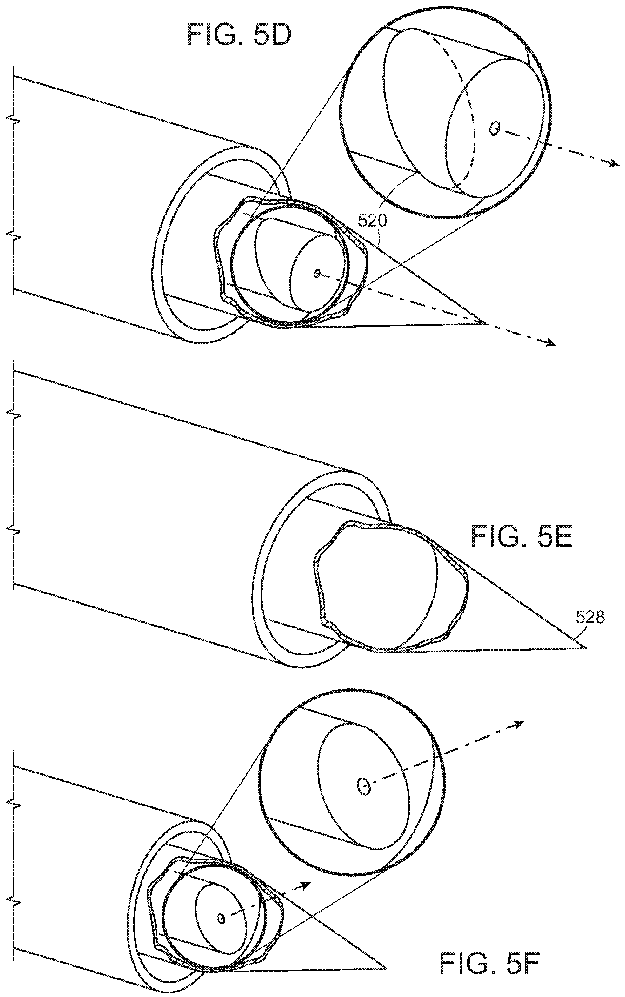

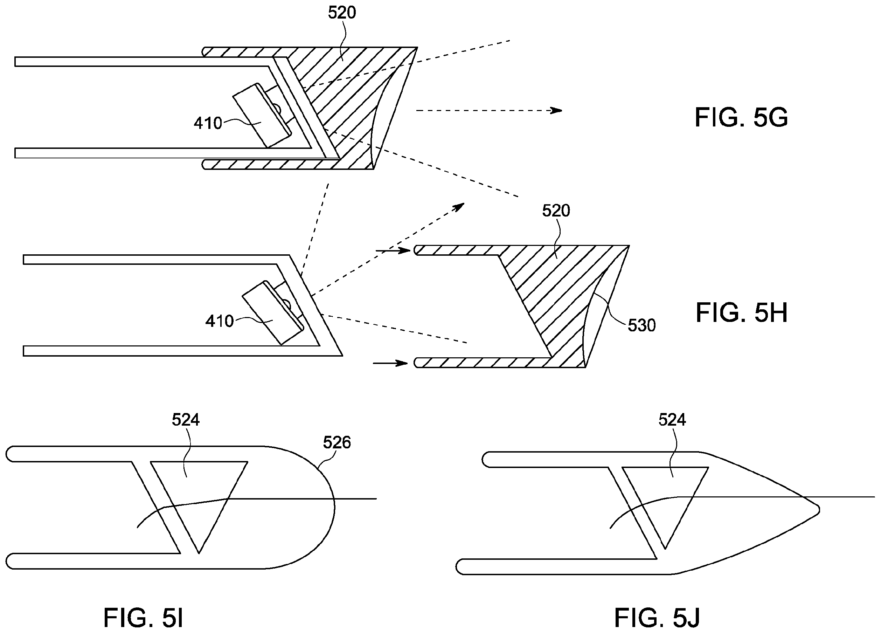

Referring to FIGS. 5D to 5J, end cap 520 may be affixed to the end of the endoscope to alter a field-of-view angle so that a single endoscope may be used at two different phases of the surgical procedure. During penetration using an endoscope with a 30° offset of view angle, the endoscope may be fitted with a refractive cap or a prism, that bends light to reduce the offset angle. Refraction by refractive cap 520 may yield a 0° on-axis view, or a near-zero angle such as 3°, 5°, or 10°. A scope with refractive cap 520 may be used during initial penetration by trocar 102 and obturator 104, to give a straight-ahead view, or a near-straight-ahead view to within 3°, 5°, or 10° . Referring to FIGS. 5E and 5H, once trocar 102 and obturator 104 have reached the site of the surgery, refractive cap 520 may be removed from endoscope 100 to yield a 30° offset scope. Removal may require withdrawal of endoscope 100, and then reinsertion. Then, referring to FIG. 5F, endoscope 100 may be returned down trocar 102 for use in a surgical procedure with a 30° offset field of view. Typically, obturator 104 is discarded once the penetration pierce is completed.

Refractive cap 520 may be molded of clear plastic, such as polycarbonate, acrylic, styrene, polyolefin, silicone, or inorganic transparent materials such as silica (silicon dioxide). In some cases, the refractive cap may be formed of multiple materials, such as glass and plastic, or two different plastic resins, to marry light refraction and various mechanical functions, such as to provide a sharp piercing point for cases where the endoscope is used without an obturator. Referring to FIGS. 5I and 5J, refractive cap 520 may be formed of different materials that provide different properties. For example, a high-index-of-refraction prism 524 may be embedded in a plastic of lower index of refraction, so that the plastic may be shaped in a desired manner. Refractive cap 520 may be shaped to provide an atraumatic rounded nose 526, to avoid tissue injury. In other cases, FIGS. 5D-5F, 5J, the low-refraction plastic may be shaped with a piercing point 528. The point may be reinforced by a steel piercing tip.

One surface or the other may be convex or concave 530 to widen or narrow the field of view, or to magnify or reduce the forward-looking view. Refractive cap 520 may permit a single endoscope to be used in two different phases of the surgery, where two different views are desired. The degree of refraction may be enough to reduce the offset angle by 5°, 10°, 15° 20°, 25°, or 30°. In some cases, obtaining partial correction less than 0° on-axis may be sufficient to improve the view during piercing. In some cases, the apex point of obturator 104 may create optical distortion, so it may be desirable to maintain some optical offset to keep that distortion away from the center of view.

Refractive cap 520 may be attached to the tip of endoscope 100 by friction fit or interference fit (the inner diameter of refractive cap 520 equal to or slightly smaller than the outer diameter of endoscope tip 400), by means of a weak or snap-frangible adhesive, via a small bump on the inner diameter of refractive cap 520 that engages with a depression or dimple in the tip of endoscope 100, a threading or channel on the inner diameter of refractive cap 520 to engage with a small raised stud on endoscope 100, or by other connector. The sleeve portion of refractive cap 520 may be formed of a heat-shrink plastic or other material that can be shrunk to secure the connection. Refractive cap 520 may be held in place by a sleeve of elastic plastic, like a condom. While it is not desirable that refractive cap 520 fall off during use, that is a low severity event, because the endoscope with refractive cap 520 is inside obturator 104, and the refractive cap will be captured and removed when obturator 104 is withdrawn.

Refractive cap 520 may have holes through the attachment sleeve to permit fluid flow and/or suction to flow through from passages in the endoscope shaft.

A surface of refractive cap 520 may be etched with a reticule or measuring scale. The reticule may be marked with a scale with which the surgeon can measure the size of objects seen through the endoscope. The surgeon may also use the reticule to align the endoscope within the surgical field.

Refractive cap 520 may have optical filters, for example, to reduce light reflected into the endoscope, to block certain wavelengths of light. The filter may include a polarizing filter, a bandpass filter, a color filter, or an interference filter. These filters can be used in conjunction with specialized light sources (e.g., ultraviolet, infrared, or polarized) and video processing for therapeutic and diagnostic purposes. Thus, refractive cap 520 may be part of a complete system to diagnose pathology using different wavelengths of light and/or colors of light and filtering the light. Further, refractive cap 520 may also be provided as part of system that delivers photonic energy to a surgical site to control and visualize photodynamic therapy.

Referring again to FIG. 5G, in some cases, objective lens/window of the endoscope may be treated with a coating, for example an anti-adhesive coating, and the endoscope may be delivered with anti-adhesive lubricant in a space between the lens/window and the proximal surface of the refractive cap's prism. In such cases, the endoscope may be used as shown in FIGS. 5D, 5E, and 5F, first with refractive cap 520 attached to provide a close-to-0° view offset. Then refractive cap 520 may be removed, leaving endoscope with its 30° offset, with the anti-adhesive-coated lens/window exposed into the surgical cavity.

### VIII. 360° view tip

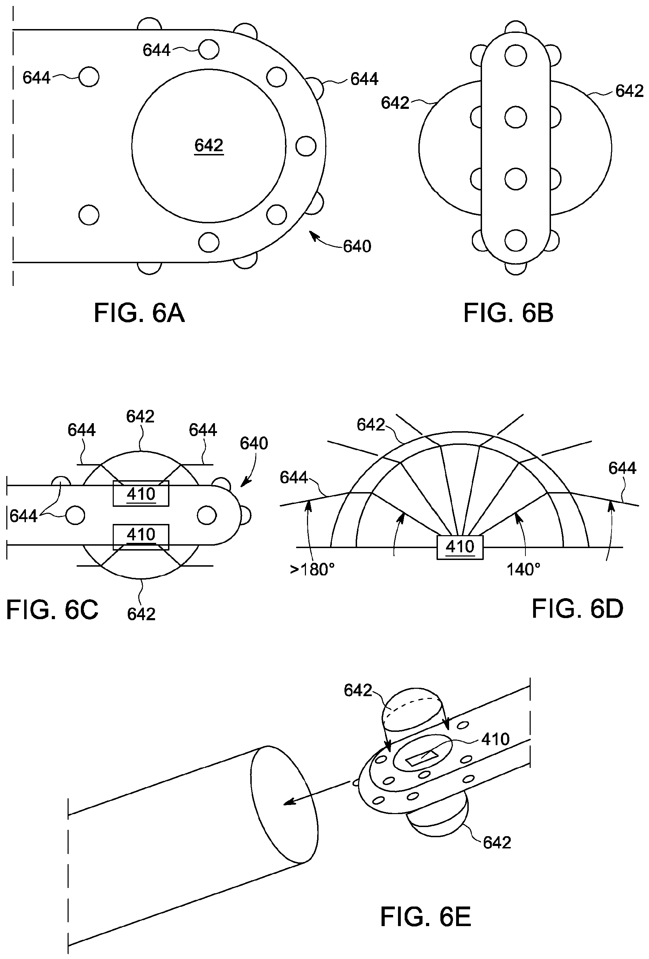

Referring to FIGS. 6A, 6B, 6C, 6D, and 6E, an endoscope tip 640 may provide a 360° view. Two cameras 410 may be mounted opposed to each other, with hemispherical lenses 642 over them. Hemispherical lenses 642 may be optically concave (as shown in FIG. 6D), to widen the field of view 644. LEDs or the emitting ends 644 of optical fibers 430 may be arrayed around the tip 640 to provide 360° illumination. Electronic image processing may be used to combine the two hemispherical views into a full 360° view that

### IX. Avoiding fasteners, springs, and other small components

Moving parts and structural components of endoscope 100 may be joined and affixed in place using techniques that avoid small components such as fasteners and springs. These manufacturing techniques may reduce costs of molding, reduce costs of assembly, reduce manufacturing steps, and reduce the likelihood that a separate component, for example, a steel spring or screw, may come loose and endanger patient safety. Likewise, assembly may be accomplished without adhesives or solvents that may be toxic.

### IX.A. A button without springs

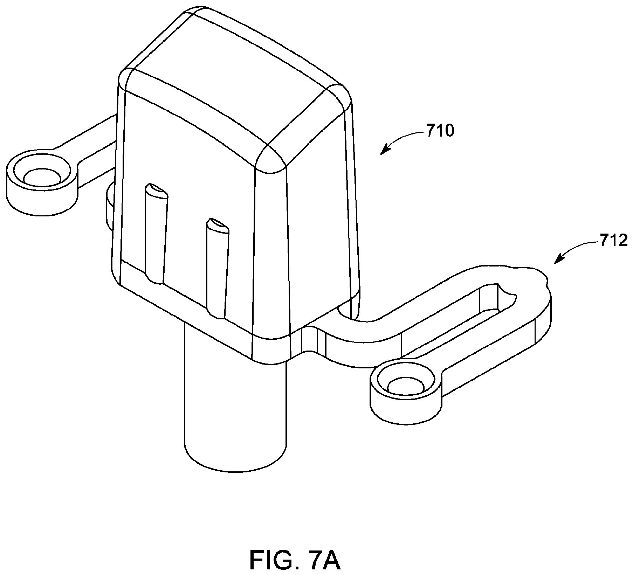

Referring to FIG 7A, a control button 710 for the endoscope may be formed as a single component with the button, its guide track, and its springs 712 molded as a single component.

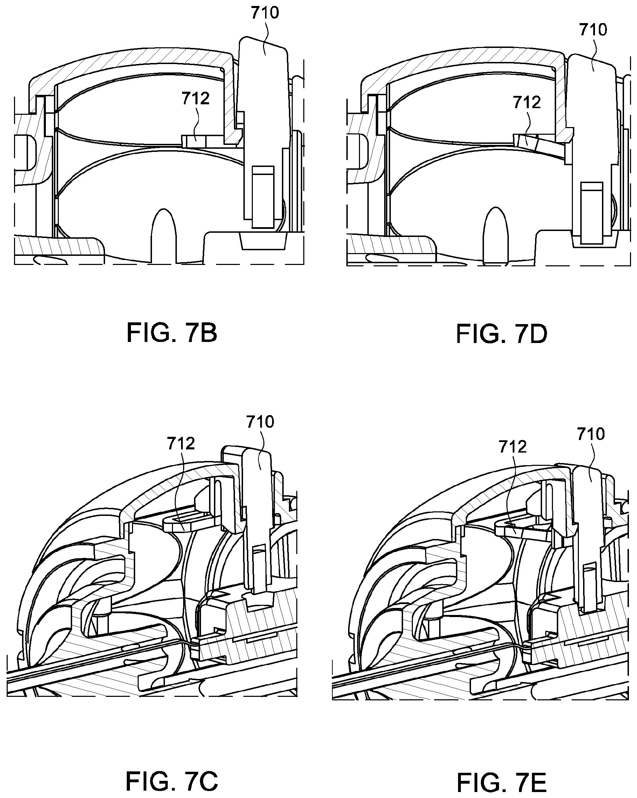

This single component may be manufactured to simultaneously provide adequate stiffness so that a button press is transmitted from the top of the button to the bottom, and adequate resilience in beam structures 712 to provide restoring force to return the button to its undepressed state. The loop beam structures 712 may be molded with flexible projections extending out from a united molded component. These extended flexible projections 712 may act in a combination of bending and torsion so that when button 710 is released, elastic memory of the materials will spring the button back into its initial position. FIGS. 7B and 7C show the button in its normally-up position, with the spring loops 712 in their originally-molded planar configuration. FIGS. 7D and 7E show the button depressed, and the spring loop 712 deformed in torsion.

The button with its springs may be molded of ABS plastic.

In some cases, the handle may have metal fasteners and other small metal parts, or assembly may use chemical adhesives and the like, but they may be encapsulated fully or partially within overmolding sufficiently to ensure no loosening , escape, or contact with fluids that flow into a patient.

### IX.B. Overmolding of case over circuit board

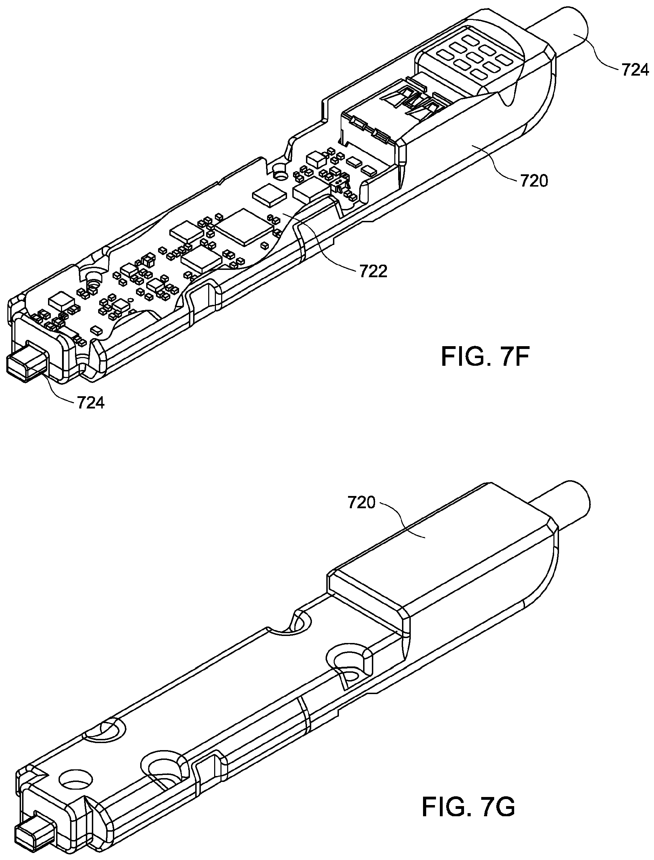

Referring to FIGS. 7F and 7G, a circuit board 722 to be mounted within the handle, with its connecting cables that will feed forward into the insertion shaft, and its connecting cables that will feed backwards to the supporting power supply and computer driver, may be overmolded 720 using low pressure overmolding. Overmolding 720 may include through-holes that provide secure mechanical mounting points for mounting within the handle. Overmolding 720 may provide a covering that protects all sides and is resilient to protect circuit board 722 from mechanical insult during manufacturing and use. Overmolded case 720 may provide strain relief for the cables 724 at the two ends of circuit board 722, to reduce damage that might be caused by pulling on the cables 724. The overmolding 720 may provide a unified, no-seams covering to protect he circuit board 722 from water intrusion. Overmolding 720 may protect against fluid ingress to a level of IP65 under international standard EN 60529. Liquid-tight protection of overmolded case 720 over circuit board 722 (and any other liquid sensitive components within the handle) may allow the sterilization of the interior of the handle via flowing a sterilizing liquid or gas through the interior of the handle, and may provide dielectric protection to prevent electrical shock to the patient. These advantages may be achieved by reducing manufacturing to a single manufacturing step.

### IX.C. Avoiding internal fasteners

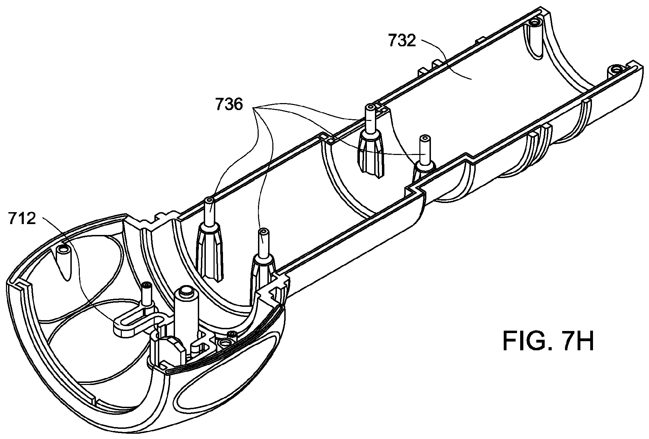

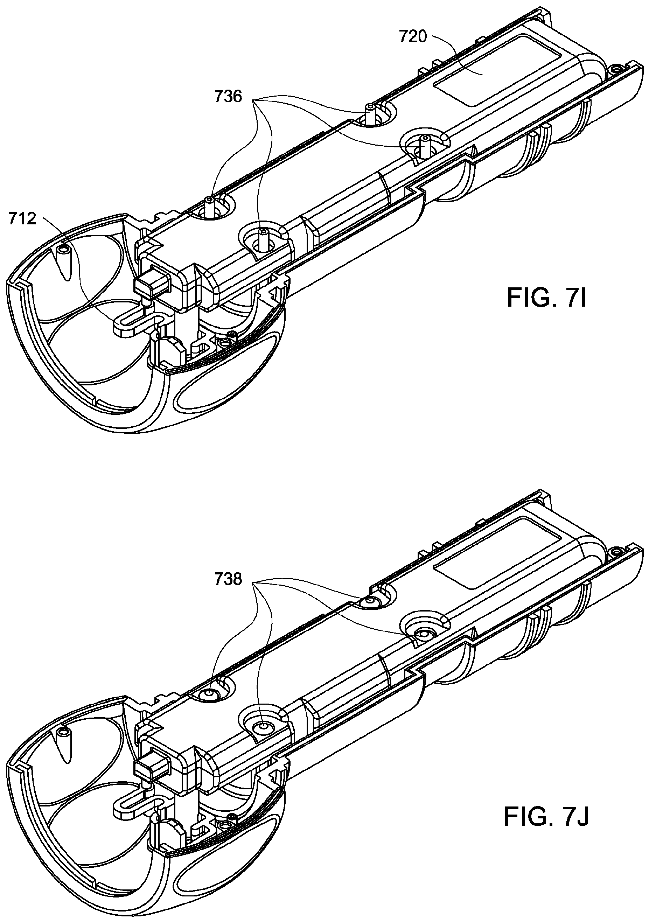

Referring to FIG. 7H, the overmolded circuit board structure may be mounted within the handle via molded clips, heat staking, and similar techniques that result in a unitary structure, without separate small-part fasteners. In FIG. 7H, the inner shell of the handle may be molded in two parts 732, 734. Top half 732, shown turned upside-down in FIG. 7I, may be molded with several molded bosses 736. In FIG. 7H, the button has been turned upside-down and set in place; the looping resilient projections are visible. In FIG. 7I, overmolded PC board 720 may be placed over bosses 736, so that bosses 736 project through the through-holes in the board casing. In FIG. 7J, heat and/or ultrasonic vibration may be used to melt the tips of bosses 736 into domes or heads 738 that hold the button and PC board in place.

By these techniques, endoscope 100 may be assembled into a structure that is strong, without small parts that require separate assembly steps or that can become dislodged, and without toxic adhesives or solvents.

### IX.D. Rotational resistance via O-rings

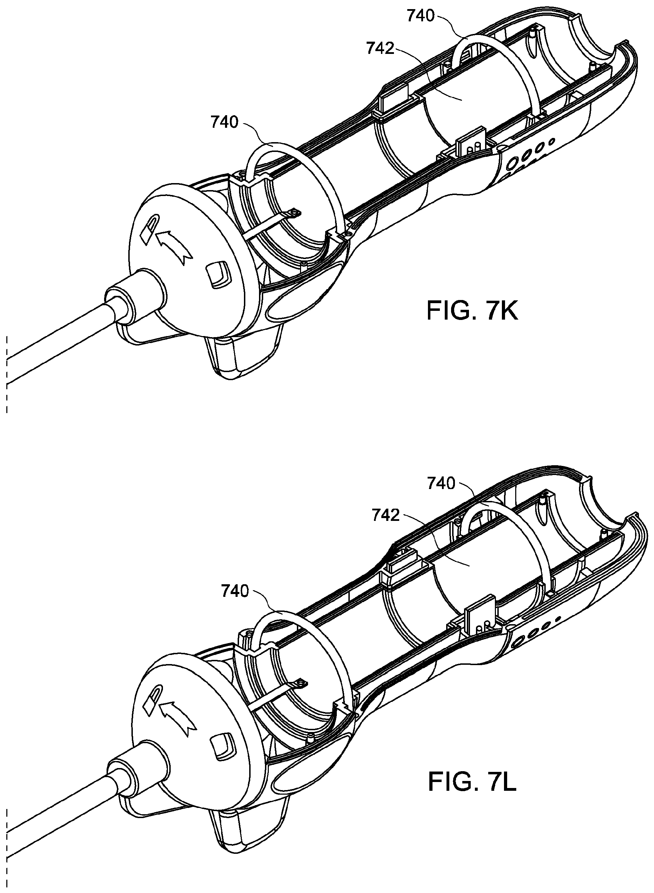

Referring to FIG. 7K and 7L, inner shell 742 and outer handle shells 732, 734 rotate relative to each other, so that the surgeon may adjust field of view, using the magnetic Hall effect sensors 324 discussed above. The inner handle 742 rotates in unison with rotation collar 112. Inner handle 742 and rotation collar rotate relative to outer handle 114. The inner handle 742 and outer handle shells 732, 734 may be coupled via two silicone O-rings 740 that provide some friction to maintain their relative position, while allowing the surgeon to rotate the two handle components to adjust the arthroscope's field of view.

For assembly, the cylindrical inner handle may be fully assembled, and the O-rings 740 may be fitted over the end of inner handle 742 into their two retaining channels. Then the two halves of the outer handle shell 732, 734 may be brought together like buns over a hotdog, surrounding the inner shell and O-rings 740. Then the two halves of the outer shell 732, 734 may be ultrasonically welded to each other.

### IX.E. Ultrasonic welding of the two halves of the outer handle shell

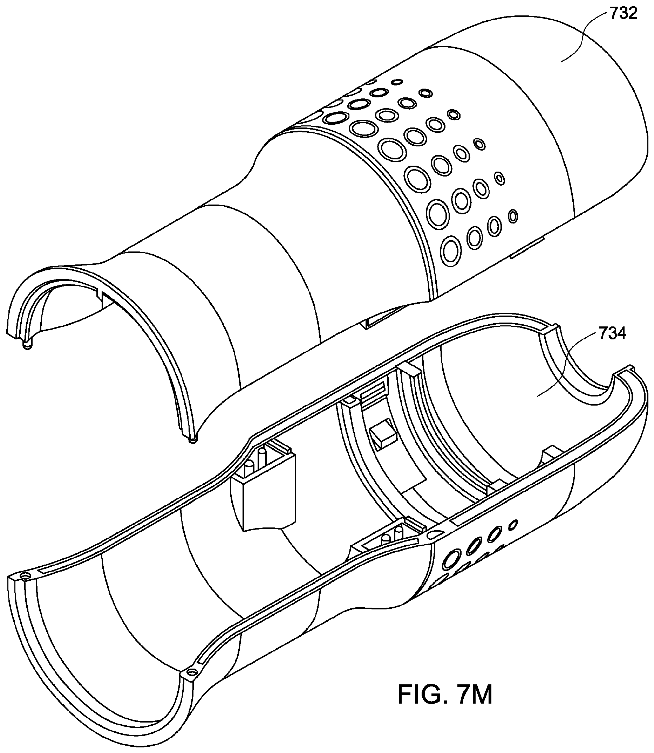

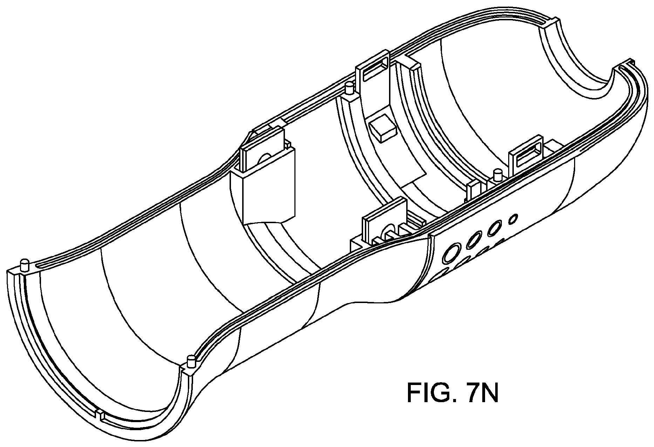

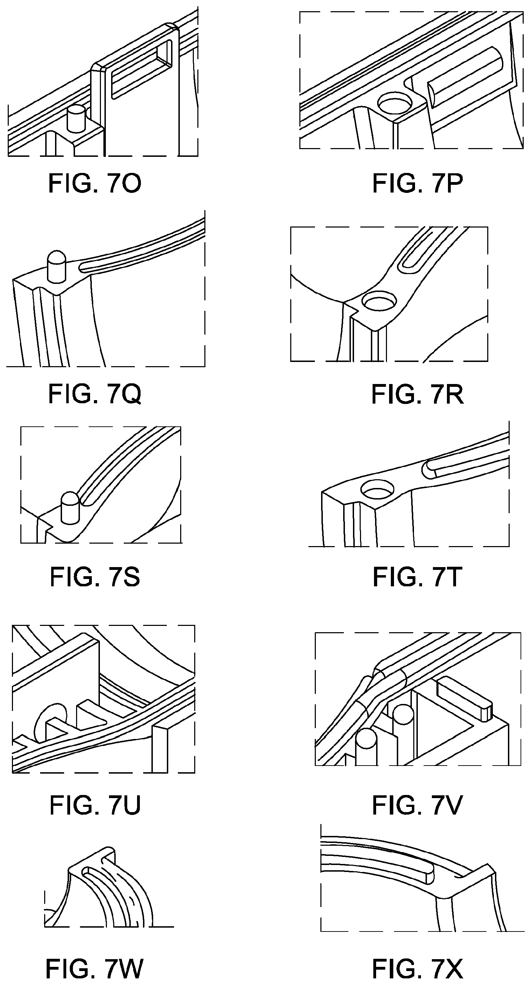

Referring to FIG. 7M and 7N, the two halves 732, 734 of the outer shell may be ultrasonically welded without adhesives or solvents. Referring to FIGS. 7O and 7P, the two outer shell halves may have locking clips or hooks that may snap together to hold them temporarily before ultrasonic welding begins.

Referring to FIGS. 7O, 7P, 7Q, 7R, 7S, 7T, 7U, 7V, 7W, and 7X, various energy directors and troughs may be designed to ensure high efficacy of ultrasonic welding. In each case, the height of the energy director is slightly higher than the depth of its mating trough, and the width of the energy director is slightly narrower than the trough. Ultrasonic vibration may cause the energy director to slightly melt and deform to fill the trough. The ultrasonically-welded seams may come very close to fully enclosing the cavity of the outer handle, to reduce the likelihood of any fluid intrusion from outside into the interior of the endoscope. Any fluid carries risk of pathogens. Sterilization of the interior of devices such as endoscopes is difficult, so exclusion of fluids is important for any components that may be reused.

### IX.F. Thermoplastic elastomer coating handle

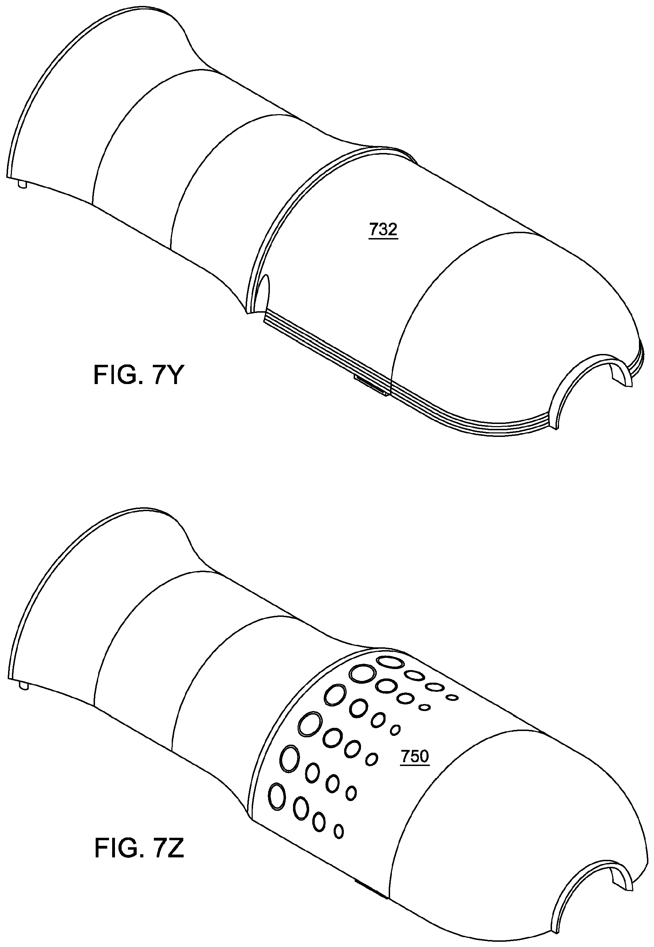

Referring to FIGS. 7Y and 7Z, a high friction surface 750 may be bonded to or overmolded onto the outer surface of the outer handle shell 732, 734, to provide a high-friction contact between a surgeon's gloved hand and the handle. During surgery, the surgeon's glove and/or the handle may become covered with bodily fluids and other liquids that may reduce friction, so that the surgeon could have trouble maintaining control of a solid endoscope. By covering the endoscope handle with a high-friction compound 750, such as thermoplastic elastomer (TPE), the handle becomes higher friction, which may improve the surgeon's control. If the cover is overmolded as a single unitary layer, it may provide additional sealing against fluid intrusion. One medical grade, biocompatible TPE is Medalist MD-34940 or MD-34950 from Teknor Apex of Pawtucket, RI.

### X. Liquid flow

### X.A. Liquid-tight seal

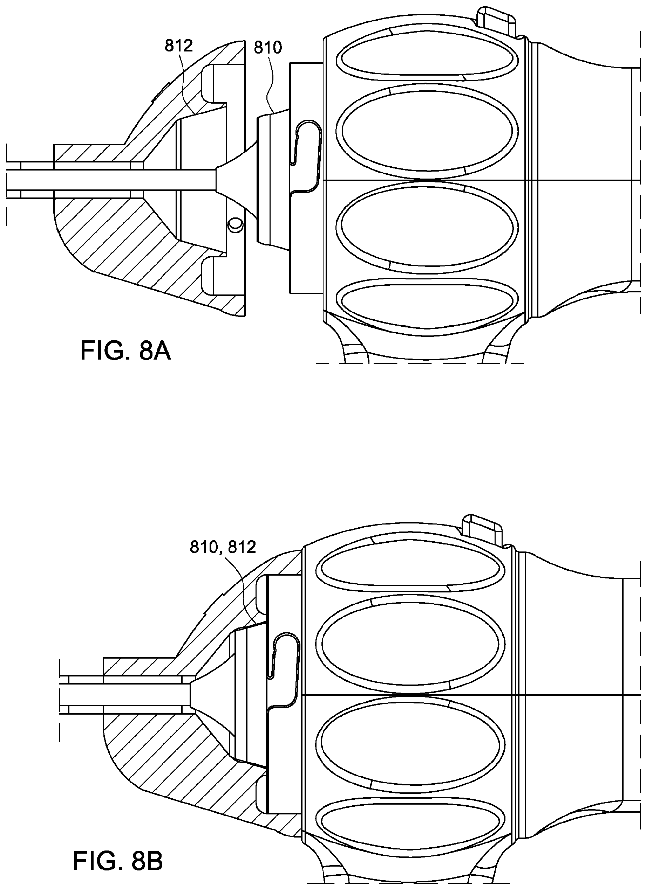

Referring to FIGS. 8A and 8B, the joint between the handle body and the outer sheath or insertion trocar 102 may be designed to provide a water tight seal between the endoscope and the outer sheath/trocar 102 without the use of an O-ring. An O-ring at this joint may be undesirable if it could fall off mid-surgery or be introduced into the patient when the endoscope is being locked into the outer sheath/trocar 102. In FIGS. 8A and 8B, the seating cup for the outer sheath/trocar 102 is shown in cutaway section, so that the male conical inner surface 810 of the seat can be seen, and the female mating cone 812 on the nose of the endoscope is shown in plan view, so that the mating between the outer surface of the male cone with the inner surface of the female cone in the cup of the outer sheath/trocar 102 can be seen.

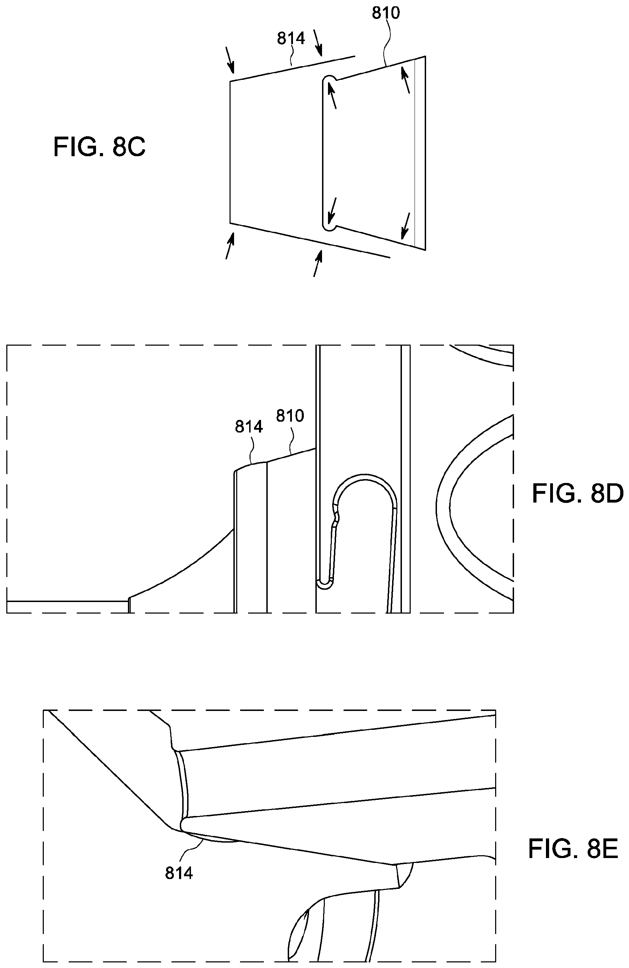

FIG. 8C shows the two cones 810, 812 with some of the dimensions exaggerated. Male cone 810 may have a slightly sharper angle of conicity than female cone 812, by about ½°. The largest diameter of female cone 812 may be slightly smaller than the largest diameter at the base of the male cone 810, so that these two surfaces 810, 812 will form a watertight interference seal. The interference of diameters may be on the order of ½ mm, when the diameters of the two cones are in the range of 19 to 25 mm. The angle may be on the order of 15°, and the two cones may differ by about ½°. Likewise, at the small end of the cones, the outer diameter of the male cone 810 may be the same as the inner diameter of the female cone 812, but male cone 810 may have small ridge 814 at its circumference, which leads to a watertight interference seal at this end as well. The polymers of the parts may be chosen to provide an appropriate amount of resilience for the interference fit to appropriately seal the water.

FIG. 8D shows ridge 814 molded onto the small end of the male cone. The ridge on male cone 810 may form an interference seal with the small end, inner diameter of female cone 814 in the mounting cup of outer sheath/trocar 102. FIG. 8E shows the two parts mated.

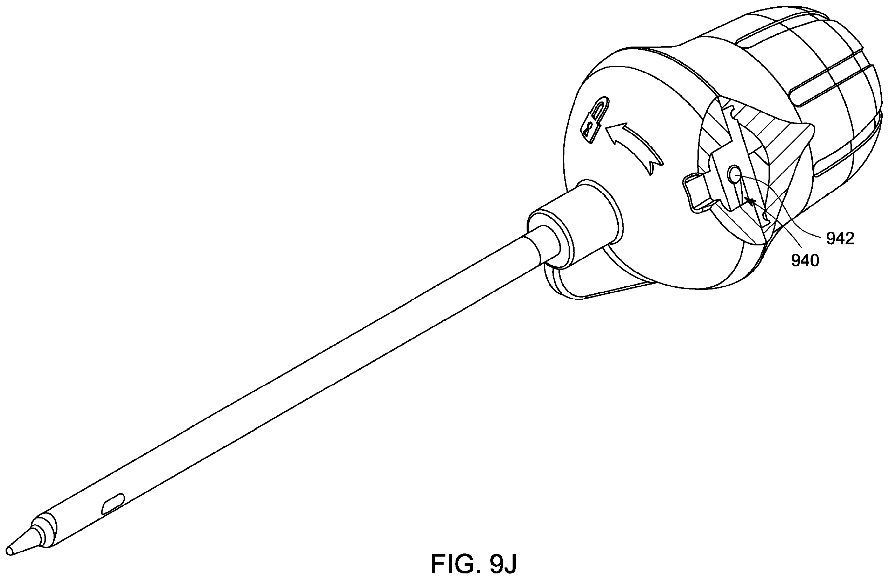

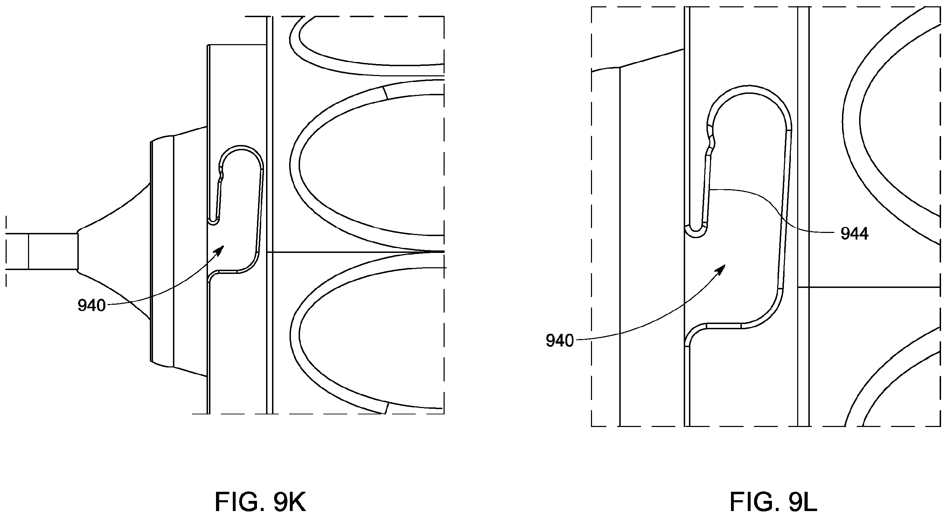

In other alternatives, the seal between the endoscope and outer sheath/trocar cap may use O-rings. O-rings may be seated in channels on the surface of the inner male cone, so that the female cone engages to compress the O-ring into its channel before the contact begins to translate into lateral forces that would displace the O-ring. O-rings may be especially desirable at the large-diameter end of the male cone abutting the face of the chassis of the endoscope, so that the female surface of the outer sheath/trocar cap cannot displace the O-ring. In some cases, the angles of the cones may be flatter than 15°, so that the compression force against the O-rings created by the twist lock (see discussion of FIGS. 9J, 9K, and 9L, in section XI.C) is increased relative to lengthwise frictional force that would tend to displace the O-rings.

### X.B. Inducing spiral flow

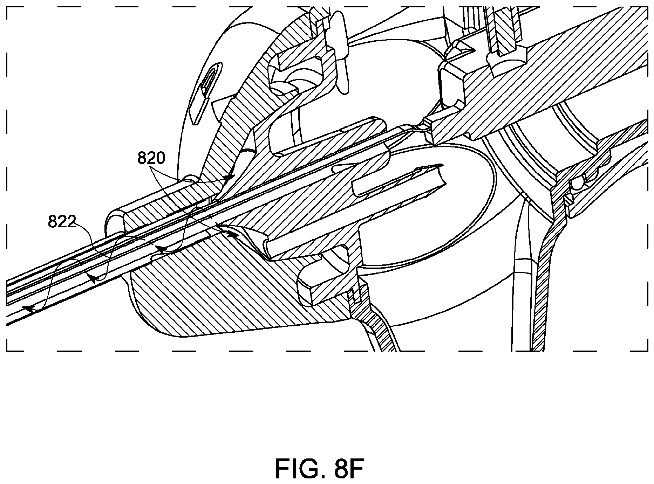

Referring to FIG. 8F, the chamber 820 between the fluid indeed tube and the fluid flow annulus of outer sheath/trocar 102 may be designed as a squeezing cone to impart spiral flow to water or similar irrigation fluid. Spiral flow 822 may be induced via the Bernoulli effect and Venturi effect as the water flows through the narrowing conical passage. Spiral vanes on the two conical surfaces may further accelerate the spiral flow.

This flow pattern may have several advantages. Spiraling flow 822 may increase pressure and water velocity as the water emerges from the flow director holes at the tip of the endoscope. Spiraling motion 822 may help clean any debris that accumulates on the front window in front of camera 410.

### XI. Molding and Joining

### XI.A. Diagonal slots to connect dissimilar materials

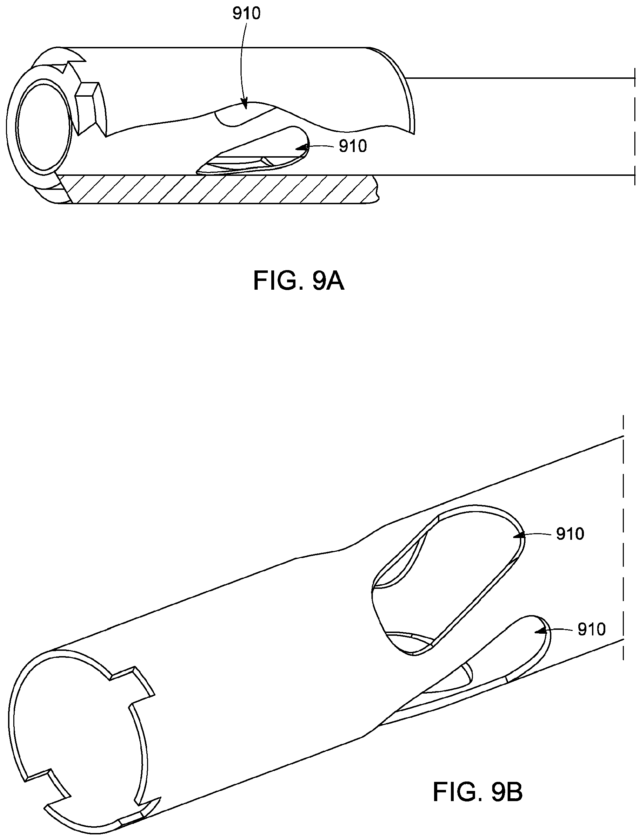

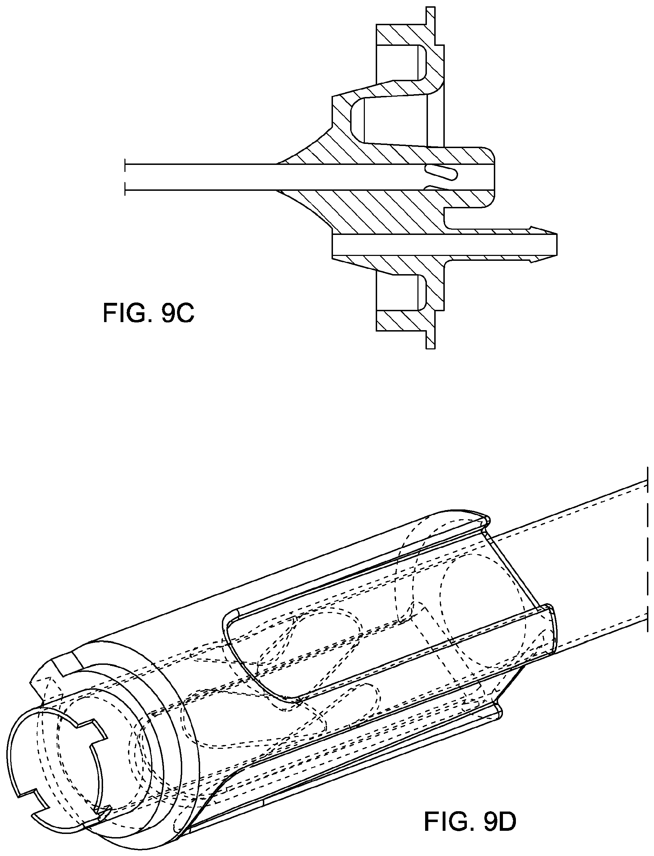

Referring to FIGS. 9A, 9B, 9C, and 9D, parts made of stainless steel may be connected to parts made of plastics such as ABS without using adhesive. One such technique is to cut holes 910 in the stainless steel and to overmold with ABS. Holes 910 may be shaped as slots, and the slots may be diagonal to the basic thrust and rotational forces. Diagonal slots 910 may provide additional securing in multiple dimensions. ABS has a shrinkage ratio, so a technique for confining play between the ABS and stainless steel may be advantageous. Joining slots 910 may be used at multiple points, for example at the base of outer sheath/trocar 102 where the base cup of outer sheath/trocar 102 mounts to it, at the base of the endoscope insertion shaft/inner sheath/cannula mounts in the nose of the endoscope, and to join the endoscope flow director onto the distal end of the insertion shaft.

### XI.B. Joining component parts of obturator

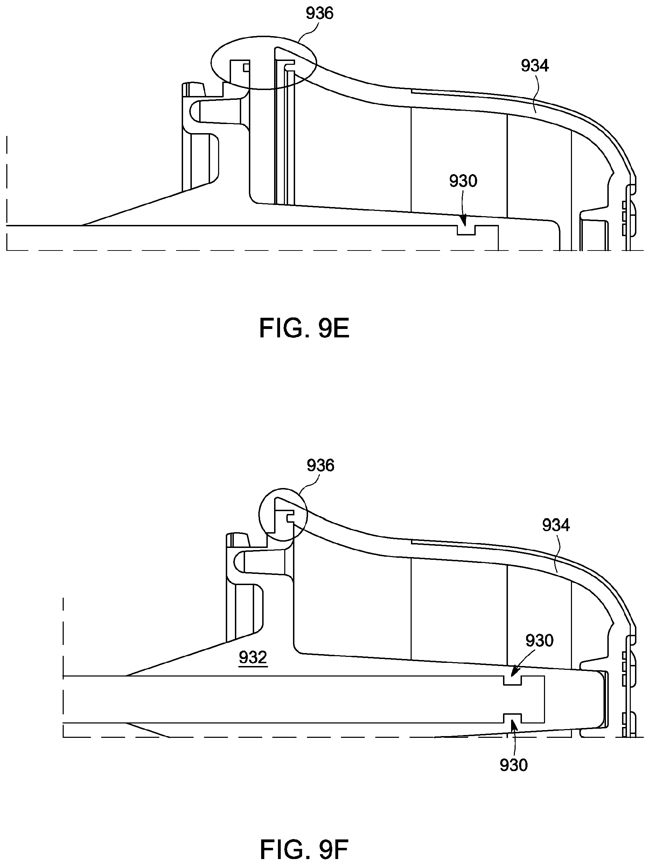

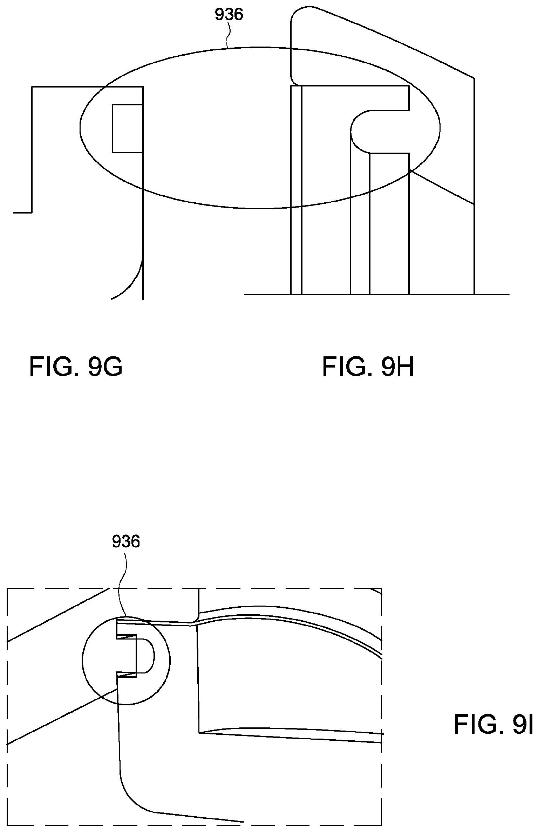

Referring to FIGS. 9F, 9G, 9H, and 9I, the handle/cap of obturator 104 may be hollow, to reduce weight and to reduce material costs. The shaft of obturator 104 may be made of solid stainless steel, because it is used to force a piercing access portal into the patient's tissues. As shown in FIG. 9E, a circumferential groove 930 may be lathed into the handle/proximal end of the obturator shaft, or two parallel cuts 930 may be milled. Base 932 of the obturator cap may then be overmolded onto this proximal end. Then the shell 934 of the handle may be ultrasonically welded 936 onto the base part. Advantages include reducing use of material, which reduces weight and cost. The ultrasonic welding may joint the outer shell to the inner base of the handle without small metal parts that may become dislodged into the patient, without adhesives that may be toxic.

FIG. 9H shows the male energy director, and FIG. 9G shows the female trough. After ultrasonic welding 936, the two parts are melted together, as shown in FIG. 9I.

### XI.C. Twist-locking the parts together

Referring to FIGS. 1C, 9J, 9K, and 9L, keyway 940 on a bayonet of the obturator handle may accept a key 942 on the inside of the mounting cap for outer sheath/trocar 102, which locks the two together during the piercing step. Slope 944 may be on the order of 3° in order to draw the parts together. Likewise, one or more keyways on a bayonet on the nose of the endoscope may engage one or more pins on the inside of the mounting cap of outer sheath/trocar 102, to lock them together during observational use of the endoscope. The key and keyways of the three components may be designed to be mutually compatible and interchangeable. This may reduce manufacturing costs, and may reduce handling during surgery.

### XII. Embodiments

Embodiments of the invention may include any one or more of the following features, singly or in any combination.

Endoscope 100 may have a handle, and an insertion shaft, the insertion shaft having at its distal end a solid state camera. The insertion shaft may have solid state illumination and imaging circuitry at or near a tip designed to provide illumination and imaging of the interior of a body cavity for a surgeon during surgery. The proximal portion of the handle may have electronics for drive of the illumination circuitry and to receive imaging signal from the imaging circuitry. The proximal handle portion may be designed to permit sterilization between uses. A joint between the proximal handle portion and the insertion shaft may designed to separably connect the insertion shaft to the proximal handle portion. When it is separated, the joint may permit removal of the insertion shaft for disposal and replacement. The joint may be designed so that, when connected, the joint can transfer mechanical force from a surgeon's hand to the insertion shaft, and provides electrical connectivity between the proximal handle circuitry and the illumination and imaging circuitry. The handle may have proximal and distal portions. The distal portion may lie between the insertion shaft and proximal handle portion. The insertion shaft may be rigidly affixed to the distal handle portion. The joint may be disposed to connect and disconnect the distal and proximal portions of the handle. The distal handle portion may be designed to indirectly transfer mechanical force between a surgeon's hand to the insertion shaft, and provide indirect electrical connectivity between the proximal handle circuitry and the illumination and imaging circuitry. The handle may have a rotation collar having surface features designed to assist the surgeon in rotating the insertion shaft in the roll dimension about the axis of the insertion shaft relative to the proximal handle portion. The electronics inside the proximal handle portion may be designed to sense roll of the insertion shaft, and provide an angular rotation signal designed to permit righting of a displayed image received from the imaging circuitry. A mounting for the image sensor may be designed to permit panning of the image sensor about a pitch or yaw axis perpendicular to the central axis of the insertion shaft. One or more ultraviolet LEDs internal to the endoscope may be designed to sterilize a region of the interior of the endoscope. Hoses for insufflation fluid or gas may be designed on lie on or near a central axis of proximal handle portion. Two or more insertion shafts each having dimensions different than the others, may each be connectable to the proximal handle portion at the joint, to permit use of the proximal handle in surgery with different requirements for insertion shaft. A sterilization cabinet may be designed to sterilize components of the endoscope. An insertion shaft of an endoscope tip has a rigid proximal portion and a distal portion. The distal portion is bendable to direct a field of view of imaging circuitry in a desired direction. An illuminator and solid state imaging circuitry are at or near a distal tip of the articulable distal portion. The illuminator is designed to illuminate, and the imaging circuitry being designed to capture imaging of, an interior of a body cavity for a surgeon during surgery. A coupling of the replaceable endoscope tip is designed to separably connect the insertion shaft at a joint to a handle portion, and to disconnect the joint. The coupling has mechanical connectors. When the joint is separated, the mechanical connectors permit removal of the insertion shaft from the handle for disposal and replacement. When the joint is connected, the joint is designed to provide mechanical force transfer between a surgeon's hand to the insertion shaft. Electrical connectors are designed to connect the insertion shaft to electronics in the handle. The handle electronics are designed for drive of the illuminator and to receive imaging signal from the imaging circuitry, the handle being designed to permit sterilization between uses. Control force transfer elements are designed to permit a surgeon to direct a direction of the imaging circuitry by transfer of mechanical force directed by a surgeon to the articulable distal portion. The distal bendable portion includes a series of articulated rigid segments. A sheath or cover over the articulated rigid segments is designed to reduce intrusion or pinching. The distal bendable portion is formed of a solid component, bendable in its lateral and elevation dimensions, and relatively incompressible in compression in its longitudinal dimension. The distal bendable portion is extendable from and retractable into a solid sheath. The distal bendable portion is bendable in one dimension. The distal bendable portion is bendable in two orthogonal dimensions. The imaging circuitry is mounted within at or near a distal tip of the articulable distal portion via a pannable mounting. The pannable mounting is designed as two sides of a parallelogram. The imaging circuitry is mounted on a structural segment hinged to the two parallelogram sides. Passages and apertures are designed to pass irrigation fluid to improve view from a lens or window over the imaging circuitry. Passages and apertures are designed to pass inflation fluid to enlarge a cavity for surgery. Mechanical connectors of the coupling include a twist-lock designed to affix the endoscope insertion shaft to the handle portion. A plurality of the endoscope tips are bundled and packaged together with a handle. The handle has electronics designed for drive of the illuminator and to receive imaging signal from the imaging circuitry. The plurality of tips and handle are packaged for integrated shipment and sale. The illuminator is an illumination LED mounted at or near the distal tip. The illuminator is an emission end of a fiber optic fiber driven by an illumination source in the handle. Camera 410 may be enclosed within a plastic casing. The plastic casing may be formed as an overmolded jacket that is designed to protect camera 410 from bodily fluids and to structurally hold components of the tip in an operating configuration. The overmolded jacket may be designed to retain a transparent window in operating configuration with camera 410. The overmolded component may be formed of transparent plastic. The overmolded component may be designed to function as a lens for camera 410. Camera 410 may be mounted on a flexible circuit board. Flexible circuit board 416 may mount an illumination LED. The LED and camera may be mounted on opposite sides of flexible circuit board 416. Camera 410 may be protected behind a transparent window. The window may be molded in two thicknesses, a thinner portion designed for mounting and to allow passage of illumination light, a thicker portion over camera 410. The handle may contain a circuit board with circuitry for control of and receipt of signals from camera 410. The handle and its components may be designed with no metal fasteners, and no adhesives, except those captured by overmolding. Control buttons of the endoscope may be molded with projections that function as return springs. The projections may be adhered into the endoscope handle via melting. The circuit board may be overmolded by plastic that encapsulate the circuit board from contact with water. The circuit board may be mounted into the handle via melting. Components of the handle may be joined to each other into a unitary structure via melting. Components of the handle may be joined by resilient clips designed to held the two components to each other before joining into unitary structure via melting. The handle may be formed of two shells concentric with each other. Rotation of the two shells relative to each other may be controlled via one or more O-rings frictionally engaged with the two respective shells. The handle may have overmolded a layer of a high-friction elastomer. The insertion shaft may be connected to the handle via a separable joint. A water joint of the separable joint may be molded for an interference seal without O-rings. A water cavity of the separable joint may be designed to impart swirl to water flowing from the handle to the insertion shaft. The insertion shaft may be formed of stainless steel and connected to the handle via a separable joint. Plastic components of the endoscope may be joined to the insertion shaft via overmolding of plastic into slots aligned at an oblique angle in the wall of the insertion shaft, without adhesives. The water joint may be formed as two cones in interference fit. The cones may interfere at a large diameter. The cones may interfere via a ridge raised on a lip of the inner male cone. Obturator 104 may be designed to pierce tissue for introduction of the endoscope. Features for twist-locking obturator 104 into trocar 102 may be compatible with features for twist-locking the endoscope into trocar.

An endoscope may have a handle and an insertion shaft. The insertion shaft has solid state illumination and imaging circuitry at or near a tip designed to provide illumination and imaging of the interior of a body cavity for a surgeon during surgery. The proximal portion of the handle has electronics for drive of the illumination circuitry and to receive imaging signal from the imaging circuitry, the proximal handle portion being designed to permit sterilization between uses. A joint between the proximal handle portion and the insertion shaft is designed to separably connect the insertion shaft to the proximal handle portion. When it is separated, the joint permits removal of the insertion shaft for disposal and replacement. The joint is designed so that, when connected, the joint can transfer mechanical force from a surgeon's hand to the insertion shaft, and provides electrical connectivity between the proximal handle circuitry and the illumination and imaging circuitry.

An endoscope may have a handle and an insertion shaft, the insertion shaft having solid state illumination and imaging circuitry at or near a tip designed to provide illumination and imaging of the interior of a body cavity for a surgeon during surgery, and the proximal portion of the handle having electronics for drive of the illumination circuitry and to receive imaging signal from the imaging circuitry, the proximal handle portion being designed to permit sterilization between uses; and a joint between the proximal handle portion and the insertion shaft designed to separably connect the insertion shaft to the proximal handle portion. The joint is separated to permit removal of the insertion shaft for disposal and replacement. The joint is reconnected with a new insertion shaft, the connection designed to provide mechanical force transfer between a surgeon's hand to the insertion shaft, and electrical connectivity between the proximal handle circuitry and the illumination and imaging circuitry.

Embodiments of the invention may include one or more of the following features. The handle may have proximal and distal portions. The distal portion may lie between the insertion shaft and proximal handle portion. The insertion shaft may be rigidly affixed to the distal handle portion. The joint may be disposed to connect and disconnect the distal and proximal portions of the handle. The distal handle portion may be designed to indirectly transfer mechanical force between a surgeon's hand to the insertion shaft, and provide indirect electrical connectivity between the proximal handle circuitry and the illumination and imaging circuitry. The handle may have a rotation collar having surface features designed to assist the surgeon in rotating the insertion shaft in the roll dimension about the axis of the insertion shaft relative to the proximal handle portion. The electronics inside the proximal handle portion may be designed to sense roll of the insertion shaft, and provide an angular rotation signal designed to permit righting of a displayed image received from the imaging circuitry. A mounting for the image sensor may be designed to permit panning of the image sensor about a pitch or yaw axis perpendicular to the central axis of the insertion shaft. One or more ultraviolet LEDs internal to the endoscope may be designed to sterilize a region of the interior of the endoscope. Hoses for insufflation fluid or gas may be designed on lie on or near a central axis of proximal handle portion. Two or more insertion shafts each having dimensions different than the others, may each be connectable to the proximal handle portion at the joint, to permit use of the proximal handle in surgery with different requirements for insertion shaft. A sterilization cabinet may be designed to sterilize components of the endoscope.

An endoscope may have a handle, and an insertion shaft. The insertion shaft may have at its distal end a solid state camera. Camera 410 may be enclosed within a plastic casing with an overmolded jacket that is designed to protect camera 410 from bodily fluids and to structurally hold components of the tip in an operating configuration. Camera 410 may be protected behind a transparent window. The window may be molded in two thicknesses. A thinner portion designed for mounting and to allow passage of illumination light, a thicker portion over camera 410. The handle may have retained within a circuit board with circuitry for control of and receipt of signals from camera 410. The handle and its components may be designed with no metal fasteners, and no adhesives, except those captured by overmolding. The handle may be formed of two shells concentric with each other. Rotation of the two shells relative to each other may be controlled via one or more O-rings frictionally engaged with the two respective shells. The handle may have an overmolded layer of a high-friction elastomer. The insertion shaft may be connected to the handle via a separable joint, a water joint of the separable joint may be molded for an interference seal without O-rings. The insertion shaft may be connected to the handle via a separable joint. A water cavity of the separable joint may be designed to impart swirl to water flowing from the handle to the insertion shaft. The insertion shaft may be formed of stainless steel and connected to the handle via a separable joint. Plastic components of the endoscope may be joined to the insertion shaft via overmolding of plastic into slots aligned at an oblique angle in the wall of the insertion shaft, without adhesives. The insertion shaft may be connected to the handle via a separable joint. Obturator 104 may be designed to pierce tissue for introduction of the endoscope. Features for twist-locking obturator 104 into trocar 102 may be compatible with features for twist-locking the endoscope into trocar 102.

The overmolded jacket may be designed to retain a transparent window in operating configuration with camera 410. The overmolded component may be formed of transparent plastic and designed to function as a lens for camera 410. Camera 410 may be mounted on a flexible circuit board: Flexible circuit board 416 may have mounted thereon an illumination LED. The LED and camera may be mounted on opposite sides of flexible circuit board 416. Control buttons of the endoscope may be molded with projections that function as return springs, the projections to be adhered into the endoscope handle via melting. The circuit board may be overmolded by plastic that encapsulates the circuit board from contact with water. The circuit board may be mounted into the handle via melting. Components of the handle may be joined to each other into a unitary structure via melting Components of the handle may be further joined by resilient clips designed to held the two components to each other before joining into unitary structure via melting. The joint may be formed as two frusta of cones in interference fit. The two frusta may interfere at their large diameters. The frusta may interfering via a ridge raised on a lip of the inner male cone.

An endoscope may have a handle and an insertion shaft. The insertion shaft has solid state illumination and imaging circuitry at or near a tip designed to provide illumination and imaging of the interior of a body cavity for a surgeon during surgery. The proximal portion of the handle has electronics for drive of the illumination circuitry and to receive imaging signal from the imaging circuitry, the proximal handle portion may be designed to permit sterilization between uses. A joint between the proximal handle portion and the insertion shaft is designed to separably connect the insertion shaft to the proximal handle portion. When it is separated, the joint permits removal of the insertion shaft for disposal and replacement. The joint is designed so that, when connected, the joint can transfer mechanical force from a surgeon's hand to the insertion shaft, and provides electrical connectivity between the proximal handle circuitry and the illumination and imaging circuitry.

An endoscope may have a handle and an insertion shaft, the insertion shaft having solid state illumination and imaging circuitry at or near a tip designed to provide illumination and imaging of the interior of a body cavity for a surgeon during surgery. The proximal portion of the handle may have electronics for drive of the illumination circuitry and to receive imaging signal from the imaging circuitry. The proximal handle portion may be designed to permit sterilization between uses. A joint between the proximal handle portion and the insertion shaft designed to separably connect the insertion shaft to the proximal handle portion. The joint may be separated to permit removal of the insertion shaft for disposal and replacement. The joint may be reconnected with a new insertion shaft, the connection designed to provide mechanical force transfer between a surgeon's hand to the insertion shaft, and electrical connectivity between the proximal handle circuitry and the illumination and imaging circuitry.

Embodiments of the invention may include one or more of the following features. The handle may have proximal and distal portions. The distal portion may lie between the insertion shaft and proximal handle portion. The insertion shaft may be rigidly affixed to the distal handle portion. The joint may be disposed to connect and disconnect the distal and proximal portions of the handle. The distal handle portion may be designed to indirectly transfer mechanical force between a surgeon's hand to the insertion shaft, and provide indirect electrical connectivity between the proximal handle circuitry and the illumination and imaging circuitry. The handle may have a rotation collar having surface features designed to assist the surgeon in rotating the insertion shaft in the roll dimension about the axis of the insertion shaft relative to the proximal handle portion. The electronics inside the proximal handle portion may be designed to sense roll of the insertion shaft, and provide an angular rotation signal designed to permit righting of a displayed image received from the imaging circuitry. A mounting for the image sensor may be designed to permit panning of the image sensor about a pitch or yaw axis perpendicular to the central axis of the insertion shaft. One or more ultraviolet LEDs internal to the endoscope may be designed to sterilize a region of the interior of the endoscope. Hoses for insufflation fluid or gas may be designed on lie on or near a central axis of proximal handle portion. Two or more insertion shafts each having dimensions different than the others, may each be connectable to the proximal handle portion at the joint, to permit use of the proximal handle in surgery with different requirements for insertion shaft. A sterilization cabinet may be designed to sterilize components of the endoscope.

A replaceable endoscope tip for an endoscope may have a rigid proximal portion and a distal portion. The distal portion may be bendable to direct a field of view of imaging circuitry in a desired direction. Illuminator and solid state imaging circuitry may be located at or near a distal tip of the articulable distal portion. The illuminator may be designed to illuminate, and the imaging circuitry may be designed to capture imaging of, an interior of a body cavity for a surgeon during surgery. A coupling is designed to separably connect the replaceable endoscope tip at a joint to a handle portion, and to disconnect the joint. The coupling has mechanical connectors designed: (a) when separated, the mechanical connectors permitting removal of the replaceable endoscope tip from the handle for disposal and replacement; and (b) when connected, the joint designed to provide mechanical force transfer between a surgeon's hand to the insertion shaft. Electrical connectors are designed to connect the replaceable endoscope tip to electronics in the handle, the handle electronics designed for drive of the illuminator and to receive imaging signal from the imaging circuitry, the handle may be designed to permit sterilization between uses. Control force transfer elements are designed to permit a surgeon to direct a direction of the imaging circuitry by transfer of mechanical force directed by a surgeon to the bendable distal portion.

An optical prism may be designed to displace a field of view offset angle of an endoscope. A connector is designed to affix the optical prism to a tip of an endoscope that has a field of view at an initial offset angle displaced off-axis of the endoscope, and to retain the optical prism against displacement forces during insertion of the endoscope into a body cavity. The optical prism and connector are designed to reduce the offset angle of the field of view of the endoscope toward on-axis relative to the initial offset when the prism and connector are affixed to an optical tip of the endoscope. The endoscope may be inserted into a body cavity. The endoscope has a field of view at an initial offset angle displaced off-axis of the endoscope. The endoscope has affixed to its distal end an optical prism designed to reduce the offset angle of the field of view of the endoscope toward on-axis relative to the initial offset. The prism is affixed to the distal end of the endoscope by a connector designed to retain the optical prism against displacement forces during insertion of the endoscope into a body cavity. The endoscope is withdrawn from the body with the prism affixed. The prism is removed from the endoscope. The endoscope is reinserted back into the body cavity with its field of view at the initial offset angle. The optical prism may be designed to reduce the offset angle of the endoscope's field of view to no more than 10°, or to no more than 5°, or to no more than 3°. The optical prism may be optically convex to magnify an image. The optical prism may be optically concave to enlarge the endoscope's field of view. The connector may be designed to affix to the endoscope by mechanical forces. An optical filter may be coupled with the prism. The endoscope may have a wetting surface designed to entrain an anti-adhesive lubricant in a layer over a lens or window of the endoscope. The wetting surface may be a porous solid. The porous solid may be formed by sintering or other heating of particles. The optical prism and connector may be affixed to the endoscope for shipment, and designed to retain an anti-adhesive lubricant in contact with a lens or window of the endoscope during shipment. The vial, well, or cavity may have a cap with a seal to seal around a shaft of the endoscope. The anti-adhesive lubricant may comprise silicone oil, or mixtures thereof. The anti-adhesive lubricant may comprise a mixture of silicone oils of different viscosities. The vial or cavity may include an optical prism designed to displace a field of view of an endoscope.

Packaging for an endoscope may have mechanical features designed to retain components of an endoscope, and to protect the endoscope for shipping and/or delivery. The packaging has a vial, well, or cavity designed to retain anti-adhesive lubricant in contact with a lens or window of the endoscope.

The distal bendable portion may include a series of articulated rigid segments. A sheath or cover may cover the articulated rigid segments designed to reduce intrusion or pinching. The distal bendable portion may be formed of a solid component, bendable in its lateral and elevation dimensions, and relatively incompressible in compression in its longitudinal dimension. The distal bendable portion may be extendable from and retractable into a solid sheath. The distal bendable portion may be bendable in one dimension. The distal bendable portion may be bendable in two orthogonal dimensions. The imaging circuitry may be mounted within at or near a distal tip of the bendable distal portion via a pannable mounting. The pannable mounting may be designed as two sides of a parallelogram, and the imaging circuitry may be mounted on a structural segment hinged to the two parallelogram sides. Passages and apertures may be designed to pass irrigation fluid to improve view from a lens or window over the imaging circuitry. Passages and apertures may be designed to pass inflation fluid to enlarge a cavity for surgery. Mechanical connectors of the coupling may include a twist-lock designed to affix the endoscope replaceable endoscope tip to the handle portion. A plurality of the endoscope replaceable endoscope tips may be packaged for integrated shipment and sale with a reusable handle, the handle having electronics designed for drive of the illuminator and to receive imaging signal from the imaging circuitry. The illuminator may be an illumination LED mounted at or near the distal tip. The illuminator may be an emission end of a fiber optic fiber driven by an illumination source in the handle.

An arthroscope may have a handle and an insertion shaft. The insertion shaft may have near its distal end a solid state camera. The shaft may enclosed therein light conductors designed to conduct illumination light to the distal end. The shaft may have an outer diameter of no more than 6mm. The shaft may have rigidity and strength for insertion of the camera into joints for arthroscopic surgery. The light conductors in the region of the camera may be designed to conduct illumination light from a light fiber to the distal end through a space between the camera and the inner surface of the insertion shaft.

A light conduction fiber may have a flattened region shaped to lie between an endoscope camera and an inner surface of an outer wall of an endoscope shaft, and shaped to conduct illumination light to a distal end of the endoscope shaft for illumination of a surgical cavity to be viewed by the camera. The shaft may be no more than 6mm in diameter. The flattened region is formed by heating a region of a plastic optical fiber, and squeezing the heated region in a polished mold.

Preferred embodiments may feature one or more of the following. One or more light guides may be designed to conduct illumination light from a light fiber to the distal end. The light guide may have a cross-section other than circular. The light guide may have a coupling to accept illumination light from a circular-cross-section optical fiber. The light guide's cross-section in the region of the camera may be narrower than the diameter if the light fiber in the light guide's dimension corresponding to a radius of the insertion shaft. At least one of an inner and outer surface of the one or more light guides may be longitudinally fluted. A distal surface of the one or more light guides or flattened region may be designed to diffuse emitted light. A distal surface of the one or more light guides may have surface microdomes designed to diffuse emitted light, or may be otherwise configured to improve uniformity of illumination into a surgical cavity accessed by the arthroscope. One or more light conductors in the region of the camera may be formed as a flattened region of an optical fiber. The flattened region may be shaped to lie between the endoscope camera and an inner surface of an outer wall of an endoscope shaft. The flattened region may be shaped to conduct illumination light to a distal end of the endoscope shaft for illumination of a surgical cavity to be viewed by the camera. The shaft may be no more than 6mm in outer diameter. The flattened region may be formed by heating a region of a plastic optical fiber. The flattened region may be formed by squeezing an optical fiber in a polished mold. Component parts for mounting near the distal end of the endoscope may be shaped using poka-yoke design principles to ensure correct assembly. Component parts of a lens assembly for mounting near the distal end may be shaped using poka-yoke design principles to ensure correct assembly. Component parts near the distal end may be formed to permit focus adjustment of a lens assembly during manufacturing. The endoscope may have a terminal window designed to seal with the shaft to prevent intrusion of bodily fluids, bodily tissues, and/or insufflation fluid. The terminal window may be designed to reduce optical artifacts. The artifacts may reduced may be reflection, light leakage within the endoscope, fouling by bodily fluids and/or bodily tissues, and fogging. The light conductors in the region of the camera may include at least nine optical fibers of essentially continuous diameter from a light source, the light fibers being no more than about 0.5mm diameter, and arrayed to subtend at least 250° of the circumference of the distal end of the endoscope. An arthroscope insertion shaft may have near its distal end a solid state camera. The shaft may have enclosed therein light conductors designed to conduct illumination light to the distal end. The shaft may have rigidity and strength for insertion of the camera into joints for arthroscopic surgery. The flattened region may be dimensioned to conduct illumination light from a light fiber to the distal end through a space between the camera and the inner surface of the insertion shaft.

Various processes described herein may be implemented by appropriately programmed general purpose computers, special purpose computers, and computing devices. Typically a processor (*e.g.,* one or more microprocessors, one or more microcontrollers, one or more digital signal processors) will receive instructions (*e.g*., from a memory or like device), and execute those instructions, thereby performing one or more processes defined by those instructions. Instructions may be embodied in one or more computer programs, one or more scripts, or in other forms. The processing may be performed on one or more microprocessors, central processing units (CPUs), computing devices, microcontrollers, digital signal processors, or like devices or any combination thereof. Programs that implement the processing, and the data operated on, may be stored and transmitted using a variety of media. In some cases, hard-wired circuitry or custom hardware may be used in place of, or in combination with, some or all of the software instructions that can implement the processes. Algorithms other than those described may be used.

Programs and data may be stored in various media appropriate to the purpose, or a combination of heterogeneous media that may be read and/or written by a computer, a processor or a like device. The media may include non-volatile media, volatile media, optical or magnetic media, dynamic random access memory (DRAM), static ram, a floppy disk, a flexible disk, hard disk, magnetic tape, any other magnetic medium, a CD-ROM, DVD, any other optical medium, punch cards, paper tape, any other physical medium with patterns of holes, a RAM, a PROM, an EPROM, a FLASH-EEPROM, any other memory chip or cartridge or other memory technologies.

Databases may be implemented using database management systems or ad hoc memory organization schemes. Alternative database structures to those described may be readily employed. Databases may be stored locally or remotely from a device which accesses data in such a database.

In some cases, the processing may be performed in a network environment including a computer that is in communication (e.g., via a communications network) with one or more devices. The computer may communicate with the devices directly or indirectly, via any wired or wireless medium (e.g. the Internet, LAN, WAN or Ethernet, Token Ring, a telephone line, a cable line, a radio channel, an optical communications line, commercial on-line service providers, bulletin board systems, a satellite communications link, a combination of any of the above). Transmission media include coaxial cables, copper wire and fiber optics, including the wires that comprise a system bus coupled to the processor. Transmission may occur over transmission media, or over electromagnetic waves, such as via infrared, WiFi, Bluetooth, and the like, at various frequencies using various protocols. Each of the devices may themselves comprise computers or other computing devices, such as those based on the Intel^{®} Pentium^{®} or Centrino^{™} processor, that are adapted to communicate with the computer. Any number and type of devices may be in communication with the computer.

A server computer or centralized authority may or may not be necessary or desirable. In various cases, the network may or may not include a central authority device. Various processing functions may be performed on a central authority server, one of several distributed servers, or other distributed devices.

Reference is made to the following: U.S. App. Ser. No. 17/824,857, filed May 25, 2022, titled Endoscope; U.S. Prov. App. Ser. No. 63/249,479, filed Sept. 28, 2021, titled Endoscope; U.S. Prov. App. Ser. No. 63/237,906, fled Aug. 27, 2021, titled Endoscope; U.S. App. Ser. No. 17/361,711, filed Jun. 29, 2021, titled Endoscope with Bendable Camera Shaft; U.S. Prov. App. Ser. No. 63/214,296, filed Jun. 24, 2021, titled Endoscope with Bendable Camera Shaft; U.S. Provisional App. Ser No. 63/193,387 titled Anti-adhesive Window or Lens for Endoscope Tip; U.S. Provisional App. Ser. No. 63/067,781, filed Aug. 19, 2020, titled Endoscope with Articulated Camera Shaft; U.S. Provisional Application Ser. No. 63/047,588, filed Jul. 2, 2020, titled Endoscope with Articulated Camera Shaft; U.S. Provisional App. Ser. No. 63/046,665, filed Jun. 30, 2020, titled Endoscope with Articulated Camera Shaft; U.S. App. Ser. No. 16/434,766, filed Jun. 7, 2019, titled Endoscope with Disposable Camera Shaft and Reusable Handle; U.S. Provisional App. Ser. No. 62/850,326, filed May 20, 2019, titled Endoscope with Disposable Camera Shaft; U.S. App. Ser. No. 16/069,220, filed Oct. 24, 2018, titled Anti-Fouling Endoscopes and Uses Thereof; U.S. Provisional App. Ser. No. 62/722,150, filed August 23, 2018, titled Endoscope with Disposable Camera Shaft; U.S. Provisional App. Ser. No. 62/682,585 filed June 8, 2018, titled Endoscope with Disposable Camera Shaft.

For clarity of explanation, the above description has focused on a representative sample of all possible embodiments, a sample that teaches the principles of the invention and conveys the best mode contemplated for carrying it out. The invention is not limited to the described embodiments. Well known features may not have been described in detail to avoid unnecessarily obscuring the principles relevant to the claimed invention. Throughout this application and its associated file history, when the term "invention" is used, it refers to the entire collection of ideas and principles described; in contrast, the formal definition of the exclusive protected property right is set forth in the claims, which exclusively control. The description has not attempted to exhaustively enumerate all possible variations. Other undescribed variations or modifications may be possible. Where multiple alternative embodiments are described, in many cases it will be possible to combine elements of different embodiments, or to combine elements of the embodiments described here with other modifications or variations that are not expressly described. A list of items does not imply that any or all of the items are mutually exclusive, nor that any or all of the items are comprehensive of any category, unless expressly specified otherwise. In many cases, one feature or group of features may be used separately from the entire apparatus described. The claims may be practiced without some or all of the specific details described in the specification. In many cases, method steps described in this specification can be performed in different orders than that presented in this specification, or in parallel rather than sequentially.

## Claims

1. An arthroscope (100), comprising:
a handle (112, 114) and an insertion shaft (110), the insertion shaft having near its distal end a solid state camera (410), the shaft having an outer diameter of no more than 6mm, the shaft having rigidity and strength for insertion of the camera into joints for arthroscopic surgery;
one or more light guides (450) designed to conduct illumination light from a light fiber to the distal end, the light guide(s) having a cross-section other than circular, the light guide having a coupling to accept illumination light from a circular-cross-section optical fiber;
at least one of an inner and outer surface of the one or more light guides are longitudinally fluted;
wherein illumination emission is via the one or more light guides (450) that conduct illumination light around the camera (410), within the diameter of the shaft (110).

2. The arthroscope of claim 1, wherein:
the light guide(s)' cross-section in the region of the camera being narrower than the diameter of the light fiber in the light guide(s)' dimension corresponding to a radius of the insertion shaft.

3. The arthroscope of any of the preceding claims, wherein:
the outer surface of the one or more light guides is longitudinally fluted.

4. The arthroscope of any of the preceding claims, wherein:
the inner surface of the one or more light guides is longitudinally fluted.

5. The arthroscope of any of the preceding claims, wherein:
a distal surface of the one or more light guides is designed to diffuse emitted light.

6. The arthroscope of claim 5, wherein:
a distal surface of the one or more light guides has surface microdomes designed to diffuse emitted light.

7. The arthroscope of any of the preceding claims:
light conductor(s) in the region of the camera being formed as a flattened region of an optical fiber, the flattened region being shaped to lie between the endoscope camera and an inner surface of an outer wall of an endoscope shaft, and shaped to conduct illumination light to a distal end of the endoscope shaft for illumination of a surgical cavity to be viewed by the camera, the shaft being no more than 6mm in diameter;
the flattened region formed by heating a region of a plastic optical fiber, and squeezing the heated region in a polished mold.

8. The arthroscope of any of the preceding claims:
light conductor(s) in the region of the camera being formed by heating a region of a plastic optical fiber, and squeezing the heated region in a polished mold.

9. The arthroscope of claim 7, wherein:
at least one of an inner and outer surface of the flattened region being longitudinally fluted.

10. The arthroscope of any of the preceding claims:
component parts for mounting near the distal end being shaped using poka-yoke design principles to ensure correct assembly.

11. The arthroscope of any of the preceding claims, wherein:
component parts of a lens assembly for mounting near the distal end being shaped using poka-yoke design principles to ensure correct assembly.

12. The arthroscope of any of the preceding claims, wherein:
component parts near the distal end being formed to permit focus adjustment of a lens assembly during manufacturing.

13. The arthroscope of any of the preceding claims, further comprising:
a terminal window designed to seal with the shaft to prevent intrusion of bodily fluids, bodily tissues, and/or insufflation fluid.

14. The arthroscope of any of the preceding claims, further comprising:
a terminal window designed to reduce optical artifacts, including one or more artifacts from the group consisting of reflection, light leakage within the endoscope, fouling by bodily fluids and/or bodily tissues, and fogging.

15. The arthroscope of any of the preceding claims, wherein:
the light conductor(s) in the region of the camera include at least nine optical fibers of essentially continuous diameter from a light source, the light fibers being no more than about 0.5mm diameter, and arrayed to subtend at least 250° of the circumference of the distal end of the endoscope.

## Patentansprüche

1. Arthroskop (100), umfassend:
einen Griff (112, 114) und einen Einführungsschaft (110), wobei der Einführungsschaft in der Nähe seines distalen Endes eine Festkörperkamera (410) aufweist, wobei der Schaft einen Außendurchmesser von nicht mehr als 6 mm aufweist, wobei der Schaft Steifigkeit und Festigkeit zum Einführen der Kamera in Gelenke für eine arthroskopische Operation aufweist;
einen oder mehrere Lichtleiter (450), der/die dazu ausgelegt ist/sind, Beleuchtungslicht von einer Lichtfaser zu dem distalen Ende zu leiten, wobei der (die) Lichtleiter einen anderen Querschnitt als kreisförmig aufweist/aufweisen, wobei der Lichtleiter eine Kopplung aufweist, um Beleuchtungslicht von einer optischen Faser mit kreisförmigem Querschnitt aufzunehmen;
mindestens eine von einer Innen- und Außenflächen des einen oder der mehreren Lichtleiter in Längsrichtung geriffelt ist;
wobei Beleuchtungsemission über den einen oder die mehreren Lichtleiter (450) erfolgt, die das Beleuchtungslicht innerhalb des Durchmessers des Schafts (110) um die Kamera (410) leiten.

2. Arthroskop nach Anspruch 1, wobei:
wobei der Querschnitt des (der) Lichtleiter(s) im Bereich der Kamera schmaler ist als der Durchmesser der Lichtfaser in der Abmessung des (der) Lichtleiter(s), die einem Radius des Einführungsschaftes entspricht.

3. Arthroskop nach einem der vorhergehenden Ansprüche, wobei:
die Außenfläche des einen oder der mehreren Lichtleiter in Längsrichtung geriffelt ist.

4. Arthroskop nach einem der vorhergehenden Ansprüche, wobei:
die Innenfläche des einen oder der mehreren Lichtleiter in Längsrichtung geriffelt ist.

5. Arthroskop nach einem der vorhergehenden Ansprüche, wobei:
eine distale Oberfläche des einen oder der mehreren Lichtleiter so gestaltet ist, dass sie das emittierte Licht streut.

6. Arthroskop nach Anspruch 5, wobei:
eine distale Oberfläche des einen oder der mehreren Lichtleiter Oberflächenmikrodome aufweist, die das emittierte Licht diffundieren.

7. Arthroskop nach einem der vorhergehenden Ansprüche:
wobei Lichtleiter im Bereich der Kamera als ein abgeflachter Bereich einer optischen Faser ausgebildet ist (sind), wobei der abgeflachte Bereich so geformt ist, dass er zwischen der Endoskopkamera und einer Innenfläche einer Außenwand eines Endoskopschafts liegt, und so geformt ist, dass er Beleuchtungslicht zu einem distalen Ende des Endoskopschafts zur Beleuchtung eines chirurgischen Hohlraums leitet, der von der Kamera betrachtet werden soll, wobei der Schaft einen Durchmesser von nicht mehr als 6 mm hat;
der abgeflachte Bereich durch Erhitzen eines Bereichs einer optischen Kunststofffaser und Zusammendrücken des erhitzten Bereichs in einer polierten Form gebildet wird.

8. Arthroskop nach einem der vorhergehenden Ansprüche:
wobei Lichtleiter in dem Bereich der Kamera durch Erhitzen eines Bereichs einer optischen Kunststofffaser und Zusammendrücken des erhitzten Bereichs in einer polierten Form gebildet wird (werden).

9. Arthroskop nach Anspruch 7, wobei:
mindestens eine von einer Innen- und Außenfläche des abgeflachten Bereichs in Längsrichtung geriffelt ist.

10. Arthroskop nach einem der vorhergehenden Ansprüche:
wobei Komponententeile für die Montage in der Nähe des distalen Endes nach Poka-Yoke-Prinzipien geformt sind, um eine korrekte Montage zu gewährleisten.

11. Arthroskop nach einem der vorhergehenden Ansprüche, wobei:
Komponententeile einer Linsenanordnung für die Montage in der Nähe des distalen Endes nach Poka-Yoke-Prinzipien geformt sind, um eine korrekte Montage zu gewährleisten.

12. Arthroskop nach einem der vorhergehenden Ansprüche, wobei:
Komponententeile in der Nähe des distalen Endes so geformt sind, dass sie die Scharfeinstellung einer Linsenanordnung während der Herstellung ermöglichen.

13. Arthroskop nach einem der vorhergehenden Ansprüche, des Weiteren umfassend:
ein Abschlussfenster, das dazu ausgelegt ist, mit dem Schaft abzuschließen, um das Eindringen von Körperflüssigkeiten, Körpergewebe und/oder Insufflationsfluid zu verhindern.

14. Arthroskop nach einem der vorhergehenden Ansprüche, des Weiteren umfassend:
ein Abschlussfenster, das dazu ausgelegt ist, optische Artefakte zu verringern, einschließlich eines oder mehrerer Artefakte aus der Gruppe bestehend aus Reflexion, Lichtleckage innerhalb des Endoskops, Verschmutzung durch Körperflüssigkeiten und/oder Körpergewebe und Beschlagen.

15. Arthroskop nach einem der vorhergehenden Ansprüche, wobei:
der (die) Lichtleiter im Bereich der Kamera mindestens neun optische Fasern mit im Wesentlichen kontinuierlichem Durchmesser von einer Lichtquelle beinhaltet (beinhalten), wobei die Lichtfasern nicht mehr als etwa 0,5 mm Durchmesser aufweisen und so angeordnet sind, dass sie mindestens 250° des Umfangs des distalen Endes des Endoskops abdecken.

## Revendications

1. - Arthroscope (100), comprenant :
une poignée (112, 114) et une tige d'insertion (110), la tige d'insertion ayant près de son extrémité distale une caméra à semi-conducteurs (410), la tige ayant un diamètre externe de pas plus de 6 mm, la tige ayant une rigidité et une résistance pour l'insertion de la caméra dans des articulations pour une chirurgie arthroscopique ;
un ou plusieurs guides de lumière (450) conçus pour conduire la lumière d'éclairage provenant d'une fibre optique à l'extrémité distale, le ou les guides de lumière ayant une section transversale autre que circulaire, le guide de lumière ayant un couplage pour accepter la lumière d'éclairage provenant d'une fibre optique à section transversale circulaire ;
au moins l'une d'une surface interne et d'une surface externe du ou des guides de lumière est à cannelures longitudinales ;
dans lequel l'émission d'éclairage se fait par l'intermédiaire du ou des guides de lumière (450) qui conduisent la lumière d'éclairage autour de la caméra (410), à l'intérieur du diamètre de la tige (110).

2. - Arthroscope selon la revendication 1, dans lequel :
la section transversale du ou des guides de lumière dans la région de la caméra est plus étroite que le diamètre de la fibre optique dans la dimension du ou des guides de lumière correspondant à un rayon de la tige d'insertion.

3. - Arthroscope selon l'une quelconque des revendications précédentes, dans lequel :
la surface externe du ou des guides de lumière est à cannelures longitudinales.

4. - Arthroscope selon l'une quelconque des revendications précédentes, dans lequel :
la surface interne du ou des guides de lumière est à cannelures longitudinales.

5. - Arthroscope selon l'une quelconque des revendications précédentes, dans lequel :
une surface distale du ou des guides de lumière est conçue pour diffuser la lumière émise.

6. - Arthroscope selon la revendication 5, dans lequel :
une surface distale du ou des guides de lumière a des microdomes de surface destinés à diffuser la lumière émise.

7. - Arthroscope selon l'une quelconque des revendications précédentes :
un ou plusieurs conducteurs de lumière dans la région de la caméra étant formés comme une région aplatie d'une fibre optique, la région aplatie étant formée pour se trouver entre la caméra d'endoscope et une surface interne d'une paroi externe d'une tige d'endoscope, et formée pour conduire la lumière d'éclairage vers une extrémité distale de la tige d'endoscope pour l'éclairage d'une cavité chirurgicale devant être visualisée par la caméra, la tige n'ayant pas plus de 6 mm de diamètre ;
la région aplatie étant formée par chauffage d'une région d'une fibre optique en plastique et par compression de la région chauffée dans un moule poli.

8. - Arthroscope selon l'une quelconque des revendications précédentes :
un ou plusieurs conducteurs de lumière dans la région de la caméra étant formés par chauffage d'une région d'une fibre optique en plastique, et par compression de la région chauffée dans un moule poli.

9. - Arthroscope selon la revendication 7, dans lequel :
au moins l'une d'une surface interne et d'une surface externe de la région aplatie est à cannelures longitudinales.

10. - Arthroscope selon l'une quelconque des revendications précédentes :
les pièces constitutives destinées à être montées à proximité de l'extrémité distale étant façonnées selon les principes de conception de Poka-Yoke afin d'assurer un assemblage correct.

11. - Arthroscope de l'une quelconque des revendications précédentes, dans lequel :
les pièces constitutives d'un ensemble de lentilles destinées à être montées près de l'extrémité distale sont façonnées selon les principes de conception de Poka-Yoke afin d'assurer un assemblage correct.

12. - Arthroscope selon l'une quelconque des revendications précédentes, dans lequel :
les pièces constitutives proches de l'extrémité distale sont formées pour permettre le réglage de la mise au point d'un ensemble de lentilles au cours de la fabrication.

13. - Arthroscope selon l'une quelconque des revendications précédentes, comprenant en outre :
une fenêtre terminale conçue pour se sceller avec la tige afin d'empêcher l'intrusion de fluides corporels, de tissus corporels et/ou de fluide d'insufflation.

14. - Arthroscope selon l'une quelconque des revendications précédentes, comprenant en outre :
une fenêtre terminale conçue pour réduire les artefacts optiques, comprenant un ou plusieurs artefacts provenant du groupe constitué par la réflexion, les fuites de lumière à l'intérieur de l'endoscope, l'encrassement par les fluides corporels et/ou les tissus corporels, et la buée.

15. - Arthroscope selon l'une quelconque des revendications précédentes, dans lequel :
le ou les conducteurs de lumière dans la région de la caméra comprennent au moins neuf fibres optiques de diamètre essentiellement continu provenant d'une source de lumière, les fibres optiques ne dépassant pas environ 0,5 mm de diamètre, et étant disposées de manière à sous-tendre au moins 250° de la circonférence de l'extrémité distale de l'endoscope.
